# EUROPEAN PATENT SPECIFICATION

(11) **EP 1 425 286 B1**
(45) Date of publication and mention of the grant of the patent: **28.02.2007**
(21) Application number: 02757132.2
(22) Date of filing: 04.09.2002
(51) Int. Cl.: C07D 519/00, A61K 31/4025, A61P 25/18

(54) **SUBSTITUTED 7-AZA-[2.2.1]BICYCLOHEPTANES FOR THE TREATMENT OF DISEASES**
SUBSTITUIERTE 7-AZA-[2.2.1]BICYCLOHEPTANE FÜR DIE BEHANDLUNG VON KRANKHEITEN
7-AZA-[2.2.1]-BICYCLOHEPTANES SUBSTITUES POUR LE TRAITEMENT DE MALADIES

(30) Priority: 12.09.2001 US 322346 P; 12.09.2001 US 322333 P; 12.09.2001 US 322100 P; 30.07.2002 US 399530 P
(43) Date of publication of application: 09.06.2004
(73) Proprietor: Pharmacia & Upjohn Company LLC, Kalamazoo, MI 49001 (US)
(72) Inventor: WISHKA, Donn, G., Kalamazoo, MI 49006-1993 (US); WALKER, Daniel, Patrick, Kalamazoo, MI 49009 (US); CORBETT, Jeffrey, W., Portage, MI 49024 (US); REITZ, Steven, Charles, Toledo, OH 43615 (US); RAUCKHORST, Mark, R., Portage, MI 49024 (US); GROPPI, Vincent, E., Jr., Kalamazoo, MI 49006 (US)
(74) Representative: Rutt, Jason Edward
(86) International application number: PCT/US2002/025959
(87) International publication number: WO 2003/022856

(56) References cited:
- WO-A-01/60821
- WO-A-96/06093
- US-A- 5 998 429
- BANNON A W ET AL: "BROAD-SPECTRUM, NON-OPIOID ANALGESIC ACTIVITY BY SELECTIVE MODULATION OF NEURONAL NICOTINIC ACETYLCHOLINE RECEPTORS" SCIENCE, AMERICAN ASSOCIATION FOR THE ADVANCEMENT OF SCIENCE,, US, vol. 279, no. 5347, 1998, pages 77-81, XP000906771 ISSN: 0036-8075

## Description

### BACKGROUND OF THE INVENTION

### FIELD OF INVENTION

Nicotinic acetylcholine receptors (nAChRs) play a large role in central nervous system (CNS) activity. Particularly, they are known to be involved in cognition, learning, mood, emotion, and neuroprotection. There are several types of nicotinic acetylcholine receptors, and each one appears to have a different role in regulating CNS function. Nicotine affects all such receptors, and has a variety of activities. Unfortunately, not all of the activities are desirable. In fact, one of the least desirable properties of nicotine is its addictive nature and the low ratio between efficacy and safety. The present invention relates to molecules that have a greater effect upon the α7 nAChRs as compared to other closely related members of this large ligand-gated receptor family. Thus, the invention provides compounds that are active drug molecules with fewer side effects.

### BRIEF DESCRIPTION OF RELATED TECHNOLOGY

Cell surface receptors are, in general, excellent and validated drug targets. nAChRs comprise a large family of ligand-gated ion channels that control neuronal activity and brain function. These receptors have a pentameric structure. In mammals, this gene family is composed of nine alpha and four beta subunits that co-assemble to form multiple subtypes of receptors that have a distinctive pharmacology. Acetylcholine is the endogenous regulator of all of the subtypes, while nicotine non-selectively activates all nAChRs.

The α7 nAChR is one receptor system that has proved to be a difficult target for testing. Native α7 nAChR is not routinely able to be stably expressed in most mammalian cell lines (Cooper and Millar, *J. Neurochem.,* 1997, 68(5):2140-51). Another feature that makes functional assays of α7 nAChR challenging is that the receptor is rapidly (100 milliseconds) inactivated. This rapid inactivation greatly limits the functional assays that can be used to measure channel activity.

Recently, Eisele et al. has indicated that a chimeric receptor formed between the N-terminal ligand binding domain of the α7 nAChR (Eisele et al., *Nature,* 366(6454), p 479-83, 1993), and the pore forming C-ternainal domain of the 5-HT₃ receptor expressed well in *Xenopus* oocytes while retaining nicotinic agonist sensitivity. Eisele et al. used the N-terminus of the avian (chick) form of the α7 nAChR receptor and the C-tenninus of the mouse form of the 5-HT₃ gene. However, under physiological conditions the α7 nAChR is a calcium channel while the 5-HT₃R is a sodium and potassium channel. Indeed, Eisele et al. teaches that the chicken α7 nAChR/ mouse 5-HT₃R behaves quite differently than the native α7 nAChR with the pore element not conducting calcium but actually being blocked by calcium ions. WO 00/73431 A2 reports on assay conditions under which the 5-HT₃R can be made to conduct calcium. This assay may be used to screen for agonist activity at this receptor.

US Patent 6,255,490 discloses7-azabicyclo[2.2.1]-heptane and-heptene derivatives as cholinergic receptor ligands.

US Patent 6,117,889 discloses discloses7-azabicyclo[2.2.1]-heptane and - heptene derivatives as analgesics and anti-inflammatory agents.

US Patent 6,060,473 discloses7-azabicyclo[2.2.1]-heptane and -heptene derivatives as cholinergic receptor ligands.

US Patent 6,054,464 discloses azabicyclic esters of carbamic acids useful in therapy, especially in the treatment or prophylaxis of psychotic disorders and intellectual impairment disorders, as well as intermediates and use of intermediates in synthesis.

US Patent 5,977,144 discloses compositions for benzylidene- and cinnamylidene-anabaseines and methods for using these compositions for treating conditions associated with defects or malfunctioning of nicotinic subtypes brain receptors. These compositions target the α7 receptor subtype with little or no activation of the α4β2 or other receptor subtypes.

US Patent 5,830,902 discloses quinuclidine derivatives having tricyclic hetero condensed ring. The compounds are disclosed as having strong squalene synthase inhibiting activity and being useful as a cholesterol lowering agent without causing side effects.

US Patent 5,817,679 discloses7-azabicyclo[2.2.1]-heptane and -heptene derivatives as cholinergic receptor ligands.

US Patent 5,723,103 discloses substituted benzamides and radioligand analogs and methods of using the compounds for the identification of 5-HT₃ receptors and the detection and treatment of abnormal conditions associated therewith.

US Patent 5,599,937 discloses heteroaromatic quinuclidines used for treating diseases related to muscarinic receptor function.

US Patent 5,576,434 discloses a novel process for preparing 2-(1-azabicyclo[2.2.2]oct-3-yl)-2,3,3a,4,5,6-hexahydro-1H-benz[de]isoquinolin-1-one, the pharmaceutically acceptable salts thereof, which are 5-HT₃ receptor antagonists, and the intermediates thereof.

US Patent 5,561,149 discloses the use of a mono or bicyclic carbocyclic, or heterocyclic carboxylic, acid ester or amide or an imidazolyl carbazol in the manufacture of a medicament suitable for the treatment of stress-related psychiatric disorders, for increasing vigilance, for the treatment of rhinitis or serotonin-induced disorders and/or coadministration with another active agent to increase the bioavailability thereof, or for nasal administration.

US Patent 5,556,851 discloses cinnoline-3-carboxylic acid derivatives having an antagonistic activity against serotonin 5-HT₃ receptor, its pharmaceutically acceptable salts, its N-oxide derivatives or solvates thereof, and pharmaceutical formulations containing the same for the prevention and/or treatment of various disorders such as nausea and/or emesis caused by anticancer drugs or X-ray treatment, central nervous disorders such as anxiety and/or neuropathy, gastroenteric diseases such as indigestion, chronic gastritis, digestive ulcer, irritable bowel syndromes and the like, hemicrania, cluster headache, trigeminal neuralgia, arrhythmia and the like.

US Patent 5,543,426 discloses the use of certain 3,7-disubstituted indole compounds for treating depression or cognitive disorders.

US Patent 5,510,478 discloses a group of 2-aroylaminothiazole derivatives which bind to and stimulate central muscarinic acetylcholine receptors and are useful agents for treating symptoms of cognitive disorders, specifically the impaired memory associated with a decrease in the neurotransmitter, acetylcholine. Some of the compounds of this invention also bind to 5HT_{1A} receptors and dopamine D₂ receptors, making them useful as antipsychotic agents.

US Patent 5,491,148 discloses isoquinolinones and dihydroisoquinolinones which are 5-HT₃ receptor antagonists.

US Patent 5,434,161 discloses imidazopyridines as serotonergic 5-HT₃ antagonists.

US Patent 5,362,740 discloses dihydrobenzofuran carboxamides useful in treating CNS disorders, but motility disorders, and/or emisis and/or pain in mammals, and/or migraine.

US Patent 5,362,734 discloses certain benzo-quinolizine compounds, derivatives thereof, and acid addition salts and identifies them as being 5-HT₃ antagonists which may be used in, for example, the treatment of neuro-psychiatric disorders.

US Patent 5,352,685 discloses thieno[3,2-b]pyridine derivatives effective for the prevention and therapeutical treatment of the symptoms caused by gastric hypanakinesis, such as heartburn, abdominal distension feeling, anorexia, unpleasant feeling on upper abdomen, abdominalgia, nausea, vomiting, etc. caused by the underlying diseases such as acute and chronic gastritis, stomach and duodenum ulcer, gastroneurosis, gastroptosis, etc.

US Patent 5,342,845 discloses indole derivatives and drugs. The compound of the invention is disclosed as being effective as a gastrointestinal motor activity regulator, antimigraine, antipsychotic or antianxiety drug and for dementia or orthostatic hypotension.

US Patent 5,322,951 discloses certain 1-(2,3-dihydro-indole)carbonyl intermediates useful for preparing 1-(2,3-dihydro)-1-carboxamide final products that possess 5-HT M-receptor antagonist activity.

US Patent 5,300,512 discloses benzimidazole compounds as being useful for treating serotonin mediated conditions with pharmaceutical compositions of the disclosed compounds which act as 5-HT₄ agonists or antagonists and/or 5-HT₃ antagonists.

US Patent 5,273,972 discloses novel 2-substituted-3-quinuclidinyl arylcarboxamides and arylthiocarboxamides and corresponding arylcarboxylates which have utility as therapeutic agents which exhibit gastric prokinetic, antiemetic, anxiolytic and 5-HT (serotonin) antagonist effects in warm blooded animals.

US Patent 5,246,942 discloses certain dibenzofurancarboxamides and their use as 5-HT₃ antagonists having unique CNS, anti-emetic and gastric prokinetic activity void of any significant D₂ receptor binding properties.

US Patent 5,223,511 discloses benzimidazoline-2-oxo-1-carboxylic acid compounds useful as 5-HT receptor antagonists.

US Patent 5,217,975 discloses azabicyclic compounds for treating dementia.

US Patent 5,185,333 discloses benzazine compounds useful as drugs for the prophylaxis or treatment of various digestive diseases vomiting and disturbances in central nervous systems and the like.

US Patent 5,175,173 discloses carboxamides useful as antiemetic or antipsychotic agents.

US Patent 5,122,528 discloses analgesic use of benzobicyclic carboxamides.

US Patent 5,114,947 discloses method for alleviating anxiety using benxobicyclic carboxamides.

US Patent 5,063,231 discloses method of treatment of visceral pain.

US Patent 5,039,680 discloses 5-HT₃ antagonists in preventing or reducing dependency on dependency-inducing agents.

US Patent 5,001,133 discloses benzoic acid derivatives.

US Patent 4,985,424 discloses a group of new substituted 1,7-annelated 1H-indazole derivatives being strong and selective antagonists of "neuronal" 5-hydroxytryptamine (5-HT) receptors.

US Patent 4,985,437 discloses the use of certain compounds which act as antagonists of 5-hydroxytryptamine (5-HT) at 5-HT₃ receptors for the treatment of cognitive disorders such as attentional and memory deficits and dementia states.

US Patent 4,983,600 discloses heterocyclic compounds useful as 5-HT₃ antagonists.

US Patent 4,973,594 discloses the use of compounds which act as antagonists of 5-hydroxytryptamine (5-HT) at the 5-HT₃ receptors for the treatment of depression.

US Patent 4,937,247 discloses 1-acyl indazoles and are disclosed as having 5-HT₃ antagonist activity.

US Patent 4,935,511 discloses benzoxazine and benzoxazepin carboxamide 5-HT₃ antagonists properties including CNS, anti-emetic and gastric prokinetic activity and which are void of any significant D₂ receptor binding affinity.

US Patent 4,933,445 discloses heteroazabenzobicyclic carboxamide 5-HT₃ antagonists properties including CNS, anti-emetic and gastric prokinetic activity.

US Patent 4,921,982 discloses 5-halo-2,3-dihydro-2,2-dimethylbenzofuran-7-carboxylic acids which are useful as intermediates for 5-HT₃ antagonists.

US Patent 4,920,227 discloses benzobicyclic carboxamide 5-HT₃ antagonists.

US Patent 4,920,219 discloses substituted saturated and unsaturated indole quinoline and benzazepine carboxamides and their valuable use as 5-HT₃ antagonists having CNS and gastric prokinetic activity void of any significant D₂ receptor binding properties.

US Patent 4,920,127 discloses substituted indoles and their use as 5-HT₃ receptor antagonists.

US Patent 4,910,193 discloses treatment of gastrointestinal disorders.

US Patent 4,892,872 discloses benzoxazine compounds exhibiting 5-HT₃ receptor antagonistic activity and being useful as antiemetics and so on.

US Patent 4,888,353 discloses carboxamides useful as antiemetic or antipsychotic agents.

US Patent 4,882,327 discloses certain heterocyclic N-substituted carboxamides having 5-HT₃ receptor antagonist activity.

US Patent 4,835,162 discloses agonists and antagonists to nicotine as smoking deterrents.

US Patent 4,822,795 discloses pharmaceutically useful esters and amides.

US Patent 4,803,199 discloses pharmaceutically useful heterocyclic acid esters and amides or alkylene bridged peperidines as serotonin M antagonists.

US Patent 4,798,829 discloses 1-azabicyclo[3.2.2]nonane derivatives having gastric motility enhancing activity and/or anti-emetic activity and/or 5-HT receptor antagonist activity.

US Patent 4,797,406 discloses amides and esters containing bridged piperidines and use as serotonin M antagonists.

US Patent 4,789,673 discloses heterocyclic carboxylic acid amides and esters.

US Patent 4,721,720 discloses a method of treating emesis, anxiety and/or irritable bowl syndrome.

US Patent 4,612,319 discloses bridged quinolizinidinylamides, compositions containing them and methods for their use.

US Patent 4,605,652 discloses a method of enhancing memory or correcting memory deficiency with arylamido (and arylthioamido)-azabicycloalkanes, and the pharmaceutically acceptable acid addition salts, hydrates and alcoholates thereof

WO 02/30405 A2 discloses the treatment of affective disorders by the combined action of a nicotinic receptor agonist and a monoaminergic substance.

WO 01/60821 discloses novel biarylcarboxamides.

WO 01/36417 A1 discloses novel N-azabicyclo-amide derivatives and use in therapy, especially in the treatment of prophylaxis of psychotic disorders and intellectual impairment disorders.

WO 01/29034 discloses quinuclidine acrylamides.

WO 00/73431 A2 discloses two binding assays to directly measure the affinity and selectivity of compounds at the α7 nAChR and the 5-HT₃R. The combined use of these functional and binding assays may be used to identify compounds that are selective agonists of the α7 nAChR.

WO 98/01443 discloses indole derivatives for the treatment of osteoporosis.

WO 97/35860 discloses novel benzimidazol derivatives having an affinity for the serotoninergic 5-HT₃/5-HT₄ receptors.

WO 97/30998 discloses azabicyclic esters of carbamic acids useful in therapy.

WO 96/33186 discloses substituted dihydrobenzofuran derivatives as 5-HT₄ agonists.

WO 95/27490 discloses serotonin antagonists (5-HT₃) for treating fibromyalgia.

WO 95/04742 discloses tropyl 7-azaindol-3-ylcarboxyamides as antitussive agents.

WO 95/01793 discloses 5-HT₃ antagonists as topical medicaments for treatment of peripheral disorders associated with pain.

WO 94/20465 discloses indole derivatives as antagonists of excitatory amino acids.

WO 92/10494 discloses novel compounds that are 5-HT₃ receptor antagonists.

WO 91/I7161 discloses isoquinoline amides and esters as 5-HT₃ receptor antagonists.

WO 91/09593 discloses 5-HT₃ antagonists for treatment of nausea, bradycardia or hypotension associated myocardial instability.

WO 90/14347 A as abstracted in chemical abstract 1991:143,158 discloses N-quinuclidinyl-indolecarboxamide derivatives as being antiemetics.

EP 512 350 A2 discloses 3-(indolyl-2-carboxamido) quinuclidines useful for treating diseases characterized by an excess or enhanced sensitivity to serotonin, e.g., psychosis, nausea, vomiting, dementia or other cognitive diseases, migraine, diabetes. The compound may be used to control anxiety, aggression, depression, and pain. The compounds are disclosed as serotonin 5-HT₃ antagonists.

EP 496 064 A1 discloses a process for the preparation of substituted benzofuran derivatives. The compounds are disclosed as being useful 5-HT₃ receptor antagonists.

EP 483 836 A1 discloses pyrazolo[1,5-a]pyridine-3-carboxylic acid derivatives, their preparation process, and serotonin receptor antagonists containing them as active ingredients.

EP 403 882 A2 discloses indole derivatives which have pharmacological activities such as 5-HT antagonism and the like.

DE 3810552 A1 discloses esters and amides ofindolyl-, benzo[b]thiophenyl-, benzo[b]furancarboxylic acids or 4-amino-2 methoxy-benzoic acids with N-heterocyclic or N-heterobicyclic alcohols or amines. The compounds disclosed have activity against pain especially migraine, as an anti-arrhythmic for gastrointestinal disturbances, stomach disturbances, gastritis ulcer, gall bladder, spastic colon, Crohn's disease, ulcerative colitis, carcinoid syndrome, diarrhea of various types. The compounds are also disclosed as speeding stomach emptying, controlling gastro duodenal and gastro esophageal reflux, disturbances of esophageal motility, hiatal hernia, cardiac insufficiency, hypotonic stomach, paralytic ileus, manic depressive psychosis and other psychoses. The compounds are also disclosed as useful for stress related diseases, senility, and enhancement of nasal absorption of other agents, e.g., in the treatment of emesis.

In *Bioorg. & Med. Chem. Lett.* 11 (2001) 319-321, the 5-HT₃ antagonist tropisetron (ICS 205-930) is discussed as a potent and selective α7 nicotinic receptor partial agonist.

In *Behavioral Brain Res.,* 113 (2000) 169-181, it is discussed that the brain α7 nicotinic receptor may be an important therapeutic target for the treatment of Alzheimer's disease using DMXBA which is known as GTS-21.

In *Eur. J.Med Chem.,* 34 (1999) 415-422, benzimidazole-2-carboxylic acid amides and esters are discussed as a new structural class of 5-HT₃ ligands.

### SUMMARY OF THE INVENTION

In general, the present invention is directed to methods and compositions useful in treating a disease, disorder, and/or condition in a mammal in need thereof wherein the α7 nAChR is implicated, where the mammal would receive symptomatic relief from the administration of an α7 nicotinic acetylcholine receptor agonist, by using a novel compound disclosed herein.

In accordance with the present invention, novel compounds which demonstrate useful biological activity, and particulary activity as an α7 nAcR agonist, are provided. More specifically, the invention provides a compound of Formula I: 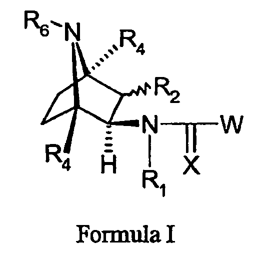
wherein the stereochemistry of the of the 7-azabicyclo[2.2.1]heptane ring is 1*S*, 4*R* and the nitrogen substituent at the C-2 carbon has the *exo* orientation and is 2R;
X is O;
W is 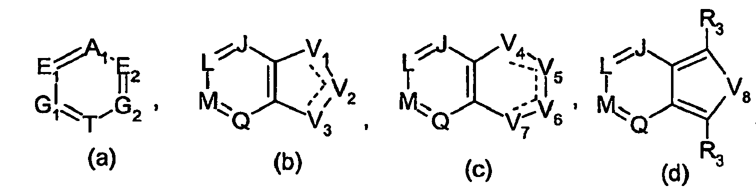 or 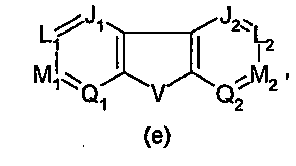
A₁ is N;
Each E₁, E₂, G₁, G₂, and T is independently selected from CR₃ and N, provided that no more than two of E₁, E₂, G₁, G₂, and T are N, and further provided that when E₁ is N, G₁ and E₂ must be CR₃, and further provided that when E₂ is N, E₁ and G₂ must be CR₃;
Each V and V₈ is independently selected from O, S, NA₅, and C(R₃)₂, provided that when V₈ is C(R₃)₂ the R₃ substitutents on the carbon atoms adjacent to V₈ are moieties other than H;
Each V₁, V₂, V₃, V₄, V₅, V₆ and V₇ is independently selected from O, S, N, C(R₃), C(R₃)₂ or NA₅, provided that V₁ and V₂, V₂ and V₃, V₄ and V₅, V₅ and V₆, and V₆ and V₇ are not simultaneously O or S, or combinations of O and S, and further provided that at least one of V₁, V₂ and V₃, at least one of V₄, V₅ and V₆, and at least one of V₅, V₆, and V₇ is C(R₃) or C(R₃)₂;
Each J, J₁, J₂, L, L₁, L₂, M, M₁, M₂, Q, Q₁, and Q₂ is independently selected from CR₃ and N, provided that no more than two of J, L, M, and Q, or no more than two of J₁, L₁, M₁, and Q₁, or no more than two of J₂, L₂, M₂, and Q₂ are N, and provided that at least one of J, L, M, and Q is N when V₁, V₂, and V₃ are independently selected from C(R₃)₂ and C(R₃), when V₄, V₅, V₆, and V₇ are independently selected from C(R₃)₂ and C(R₃), and when V₈ is C(R₃)₂, and further provided that at least one of J₁, L₁, M₁ and Q₁ or at least one of J₂, L₂, M₂, and Q₂ is N when V is C(R₃)₂;
Each A₅ is independently H, alkyl having from 1-6 carbon atoms, alkenyl having from 2-6 carbon atoms, alkynyl having from 2-6 carbon atoms, cycloalkyl having from 3-6 carbon atoms, heterocycloalkyl having 4-7 atoms with 1-2 atoms within the ring being -S-, -N(R₁₈)- or -O-, halogenated alkyl having from 1-6 carbon atoms, halogenated alkenyl having from 2-6 carbon atoms, halogenated alkynyl having from 2-6 carbon atoms, halogenated cycloalkyl having from 3-6 carbon atoms or halogenated heterocycloalkyl having 4-7 atoms with 1-2 atoms within the ring being -S-, -N(R₁₈)- or -O-;
R₁ is H, alkyl having from 1-6 carbon atoms or halogenated alkyl having from 1-6 carbon atoms;
R₂ is H, alkyl having from 1-6 carbon atoms or halogenated alkyl having from 1-6 carbon atoms;
Each R₃ is independently H, F, Cl, Br, I, -CN, -NO₂,
   alkyl having from 1-6 carbon atoms, alkenyl having from 2-6 carbon atoms, alkynyl having from 2-6 carbon atoms, cycloalkyl having from 3-6 carbon atoms, heterocycloalkyl having 4-7 atoms with 1-2 atoms within the ring being -S-, -N(R₁₈)- or - O-, halogenated alkyl having from 1-6 carbon atoms, halogenated alkenyl having from 2-6 carbon atoms, halogenated alkynyl having from 2-6 carbon atoms, halogenated cycloalkyl having from 3-6 carbon atoms, or halogenated heterocycloalkyl having 4-7 atoms with 1-2 atoms within the ring being -S-, -N(R₁₈)- or -O-, or
R₃ forms a bond to the carbon of the amide group bound to the C-2 carbon of the 7-azabicyclo[2.2.1]heptane ring;
Each R₄ is independently H or alkyl having from 1-6 carbon atoms;
R₆ is H, alkyl having from 1-6 carbon atoms, or an alkyl group having from 1-6 carbon atoms and having 1-3 substituents selected from F, Cl, Br, I, -OH, -CN, -NH₂, - NH(alkyl having from 1-6 carbon atoms), and -N(alkyl having from 1-6 carbon atoms)₂;
R₁₈ is H, alkyl having from 1-6 carbon atoms, halogenated alkyl having from 1-6 carbon atoms, cycloalkyl having from 3-6 carbon atoms or halogenated cycloalkyl having from 3-6 carbon atoms;
or pharmaceutically acceptable salt thereof.

The compounds of the present invention are used to treat or prevent diseases, disorders, and/or conditions wherein the diseases, disorders, and/or condition is any one or more or combination of the following: cognitive and attention deficit symptoms of Alzheimer's, neurodegeneration associated with diseases such as Alzheimer's disease, pre-senile dementia (mild cognitive impairment), senile dementia, schizophrenia, psychosis, attention deficit disorder, attention deficit hyperactivity disorder, depression, anxiety, general anxiety disorder, post traumatic stress disorder, mood and affective disorders, amyotrophic lateral sclerosis, borderline personality disorder, traumatic brain injury, behavioral and cognitive problems in general and associated with brain tumors, AIDS dementia complex, dementia associated with Down's syndrome, dementia associated with Lewy Bodies, Huntington's disease, Parkinson's disease, tardive dyskinesia, Pick's disease, dysregulation of food intake including bulemia and anorexia nervosa, withdrawal symptoms associated with smoking cessation and dependant drug cessation, Gilles de la Tourette's Syndrome, age-related macular degeneration, glaucoma, neurodegeneration associated with glaucoma, or symptoms associated with pain.

Embodiments of the invention may include one or more or combination of the following.

One embodiment of the present invention provides the use of a compound of Formula I, or a pharmaceutically acceptable salt thereof or pharmaceutical composition containing said compound or salt thereof, for the manufacture of a medicament.

The compound of Formula I, where W is any one or more or combination of (a), (b), (c), (d) or (e), any of which is optionally substituted with up to 4 substituents independently selected from F, Cl, Br, -CN, -NO₂, alkyl having from 1-6 carbon atoms, halogenated alkyl having from 1-6 carbon atoms, alkenyl having from 2-6 carbon atoms, halogenated alkenyl having from 2-6 carbon atoms, alkynyl having from 2-6 carbon atoms, halogenated alkynyl having from 2-6 carbon atoms, heterocycloalkyl having 4-7 atoms with 1-2 atoms within the ring being -S-, -N(R₁₈)- or -O-, and halogenated heterocycloalkyl having 4-7 atoms with 1-2 atoms within the ring being -S-, -N(R₁₈)- or -O-;
One of ordinary skill in the art can identify where substitution occurs and at which position on the W moiety the attachment to the core molecule occurs by comparing the identified moieties with W.

The compound of Formula I, where R₃ forms a bond to the carbon of the amide group bound to the C-2 carbon of the 7-azabicyclo[2.2.1]heptane ring.

The compound of Formula I, where R₁ is H or alkyl having from 1-6 carbon atoms and wherein R₂ is H, alkyl having from 1-6 carbon atoms or halogenated alkyl having from 1-6 carbon atoms.

The compound of Formula I, where (b) is any one or more or combination of the following: thieno[2,3-b]pyridin-2-yl, thieno[2,3-b]pyridin-5-yl, thieno[2,3-b]pyridin-6-yl, thieno[2,3-c]pyridin-2-yl, furo[3,2-c]pyridin-2-yl, thieno[3,2-b]pyridin-2-yl, furo[2,3-b]pyridin-2-yl, thieno[3,2-b]pyridin-5-yl, thieno[3,2-b]pyridin-6-yl, furo[2,3-c]pyridin-5-yl, thieno[3,2-c]pyridin-2-yl, 2,3-dihydrofuro[2,3-c]pyridin-5-yl, thieno[2,3-c]pyridin-5-yl, furo[2,3-c]pyridin-2-yl, thieno[3,2-c]pyridin-6-yl, 1 H-pyrrolo[2,3-c]pyridin-5-yl, furo[3,2-c]pyridin-6-yl, 1-benzofuran-5-yl, 1-benzofuran-6-yl, 1-benzofuran-2-yl, 1-benzothiophene-5-yl, 1-benzothiophen-6-yl, 1-benzothiophen-2-yl, indol-5-yl, indol-6-yl, indol-2yl, 1,3-benzothiazole-6-yl, 1,3-benzothiazole-5-yl, 1,3-benzoxazole-6-yl, 1,3-benzoxazole-5-yl, benzimidazole-6-yl, benzimidazole-5-yl, 1,3-benzodioxole-5-yl, 1H-indazole-5-yl, 1H-indazole-6-yl, 1,2-benzisothiazole-6-yl, 1,2-benzisothiazole-6-yl, 1,3-benzothiazole-5-yl, 1,3-benzothiazole-6-yl, 1,3-benzodioxole-5-yl, 1,3-benzodioxole-6-yl, 2H-isoindole-5-yl, 2H-isoindole-6-yl, 1 H-benzimidazole-5-yl, 1 H-benzimidazole-6-yl, [1,3]thiazolo[5,4-c]pyridine-6-yl, [1,3]thiazolo[4,5-c]pyridine-6-yl, [1,3]dioxolo[4,5-c]pyridine-6-yl, or [1,3]oxazolo[4,5-c]pyridine-6-yl, any of which is optionally substituted with up to 4 substituents independently selected from F, Cl, Br, -CN, -NO₂, alkyl having from 1-6 carbon atoms, halogenated alkyl having from 1-6 carbon atoms, alkenyl having from 2-6 carbon atoms, halogenated alkenyl having from 2-6 carbon atoms, alkynyl having from 2-6 carbon atoms, halogenated alkynyl having from 2-6 carbon atoms, heterocycloalkyl having 4-7 atoms with 1-2 atoms within the ring being -S-, - N(R₁₈)- or -O-, and halogenated heterocycloalkyl having 4-7 atoms with 1-2 atoms within the ring being -S-, -N(R₁₈)- or -O-.

The compound of Formula I, where R₁, R₂, and each R₄ are H.

The compound of Formula I, where the compound is any one or more or combination of the following as the free base, or pharmaceutally acceptable salt thereof:
N-[(1S, 2R, 4R)-7-azabicyclo[2.2.1]hept-2-yl]-3-methylfuro[2,3-c]pyridine-5-carboxamide;
N-[(1S, 2R, 4R)-7-azabicyclo[2.2.1]hept-2-yl]thieno[2,3-c]pyridine-5-carboxamide;
N-[(1S, 2R, 4R)-7-azabicyclo[2.2.1]hept-2-yl]thieno[3,2-c]pyridine-6-carboxamide;
N-[(1S, 2R, 4R)-7-azabicyclo[2.2.1]hept-2-yl]-3-ethylfuro[2,3-c]pyridine-5-carboxamide;
N-[(1S, 2R, 4R)-7-azabicyclo[2.2.1]hept-2-yl]-3-isopropyl-furo[2,3-c]pyridine-5-carboxamide;
N-[(1S, 2R, 4R)-7-azabicyclo[2.2.1]hept-2-yl]-1H-pyrrolo[2,3-c]pyridine-5-carboxamide;
N-[(1S, 2R, 4R)-7-azabicyclo[2.2.1]hept-2-yl]-1,3-benzodioxole-5-carboxamide;
N-[(1S, 2R, 4R)-7-azabicyclo[2.2.1]hept-2-yl]-1H-pyrrolo[2,3-c]pyridine-2-carboxamide;
N-[(1S, 2R, 4R)-7-methyl-7-azabicyclo[2.2.1]hept-2-yl]furo[2,3-c]pyridine-5-carboxamide;
N-[(1S, 2R, 4R)-7-methyl-7-azabicyclo[2.2.1]hept-2-yl]-1-benzofuran-5-carboxamide;
N-[(1S, 2R, 4R)-7-azabicyclo[2.2.1]hept-2-yl]-1-benzofuran-6-carboxamide;
N-[(1S, 2R, 4R)-7-azabicyclo[2.2.1]hept-2-yl]-1-benzofuran-5-carboxamide;
N-[(1S, 2R, 4R)-7-azabicyclo[2.2.1]hept-2-yl]-3-bromofuro[2,3-c]pyridine-5-carboxamide;
N-[(1S, 2R, 4R)-7-azabicyclo[2.2.1]hept-2-yl]-1-benzofuran-2-carboxamide;
N-[(1S, 2R, 4R)-7-azabicyclo[2.2.1]hept-2-yl]furo[3,2-c]pyridine-6-carboxamide;
N-[(1S, 2R, 4R)-7-azabicyclo[2.2.1]hept-2-yl]-3-chlorofuro[2,3-c]pyridine-5-carboxamide;
N-[(1S, 2R, 4R)-7-azabicyclo[2.2.1]hept-2-yl]benzothiophene-5-carboxamide;
N-[(1S, 2R, 4R)-7-azabicyclo[2.2.1]hept-2-yl]-2-methyl-1-benzofuran-6-carboxamide;
N-[(1S, 2R, 4R)-7-azabicyclo[2.2.1]hept-2-yl]-3-methyl-1-benzofuran-6-carboxamide;
N-[(1S, 2R, 4R)-7-azabicyclo[2.2.1]hept-2-yl]-3-isopropyl-1-benzofuran-5-carboxamide;
N-[(1S, 2R, 4R)-7-azabicyclo[2.2.1]hept-2-yl]-2-methyl-1-benzofuran-5-carboxamide;
N-[(1S, 2R, 4R)-7-azabicyclo[2.2.1]hept-2-yl]-3-bromo-1-benzofuran-5-carboxamide;
N-[(1S, 2R, 4R)-7-azabicyclo[2.2.1]hept-2-yl]-3-ethynyl-1-benzofuran-5-carboxamide;
N-[(1S, 2R, 4R)-7-azabicyclo[2.2.1]hept-2-yl]-3-prop-1-ynyl-1-benzofuran-5-carboxamide;
N-[(1S, 2R, 4R)-7-azabicyclo[2.2.1]hept-2-yl]-3-bromothieno[2,3-c]pyridine-5-carboxamide;
N-[(1S, 2R, 4R)-7-azabicyclo[2.2.1]hept-2-yl]-1H-indole-2-carboxamide;
N-[(1S,2R,4R)-7-azabicyclo[2.2.1]hept-2-yl]-1-benzothiophene-2-carboxamide; or
N-[(1S, 2R, 4R)-7-azabicyclo[2.2.1]hept-2-yl]-3-methyl-1-benzofuran-5-carboxamide.

The compound of Formula I, where the compound is any one or more or combination of the following as the free base, or pharmaceutally acceptable salt thereof:
7-methyl-N-[(1S, 2R, 4R)-7-azabicyclo[2.2.1]hept-2-yl]benzofuran-5-carboxamide;
7-chloro-N-[(1S, 2R, 4R)-7-azabicyclo[2.2.1]hept-2-yl]benzofuran-5-carboxamide;
N-[(1S, 2R, 4R)-7-azabicyclo[2.2.1]hept-2-yl]-2-vinylbenzofuran-5-carboxamide;
N-[(1S, 2R, 4R)-7-azabicyclo[2.2.1]hept-2-yl]-2-ethynylbenzofuran-5-carboxamide;
N-[(1S, 2R, 4R)-7-azabicyclo[2.2.1]hept-2-yl]-2-prop-1-ynylbenzofuran-5-carboxamide;
N-[(1S, 2R, 4R)-7-azabicyclo[2.2.1]hept-2-yl]-2-cyanobenzofuran-5-carboxamide;
N-[(1S, 2R, 4R)-7-azabicyclo[2.2.1]hept-2-yl]-2-chlorobenzofiuan-5-carboxamide;
N-[(1S, 2R, 4R)-7-azabicyclo[2.2.1]hept-2-yl]-2-fluorobenzofuran-5-carboxaanide;
N-[(1S, 2R, 4R)-7-azabicyclo[2.2.1]hept-2-yl]-2-iodobenzofuran-5-carboxamide;
N-[(1S, 2R, 4R)-7-azabicyclo[2.2.1]hept-2-yl]-2-trifluoromethylbenzofuran-5-carboxamide;
N-[(1S,2R,4R)-7-azabicyclo[2.2.1]hept-2-yl]-2-(pyrrolidin-1-yl)benzofuran-5-carboxamide;
N-[(1S, 2R, 4R)-7-azabicyclo[2.2.1]hept-2-yl]-2-(piperidin-1-yl)benzofuran-5-carboxamide;
N-[(1S,2R,4R)-7-azabicyclo[2.2.1]hept-2-yl]-2-(morpholin-4-yl)benzofuran-5-carboxamide;
N-[(1S,2R,4R)-7-azabicyclo[2.2.1]hept-2-yl]-2-(thiomorpholin-4yl)benzoiuran-5-carboxamide;
N-[(1S, 2R, 4R)-7-azabicyclo[2.2.1]hept-2-yl]-2-(piperazin-1-yl)benzofurun-5-carboxamide;
N-[(1S, 2R, 4R)-7-azabicyclo[2.2.1]hept-2-yl]-2-vinylbenzofuran-6-carboxamide;
4-methyl-N [(1S, 2R, 4R)-7-azabicyclo[2.2.1]hept-2-yl]lbenzofuran-6-carboxamide;
4-chloro-N-[(1S, 2R, 4R)-7-azabicyclo[2.2.1]hept-2-yl]benzofuran-6-carboxamide;
N-[(1S, 2R, 4R)-7-azabicyclo[2.2.1]hept-2-yl]-2-ethynylbenzofuran-6-carboxamide;
N-[(1S, 2R, 4R)-7-azabicyclo[2.2.1]hept-2-yl]-2-prop-1-ynylbenzofuran-6-carboxamide;
N-[(1S, 2R, 4R)-7-azabicyclo[2.2.1]hept-2-yl]-2-cyanobenzofuran-6-carboxamide;
N-[(1S, 2R, 4R)-7-azabicyclo[2.2.1]hept-2-yl]-2-chlorobenzofuran-6-carboxamide;
N-[(1S, 2R, 4R)-7-azabicyclo[2.2.1]hept-2-yl]-2-fluorobenzofuran-6-carboxamide;
N-[(1S, 2R, 4R)-7-azabicyclo[2.2.1]hept-2-yl]-2-iodobenzofuran-6-carboxamide;
N-[(1S, 2R, 4R)-7-azabicyclo[2.2.1]hept-2-yl]-2-trifluoromethylbenzofuran-6-carboxamide;
N-[(1S, 2R, 4R)-7-azabicyclo[2.2.1]hept-2-yl]-2-(pyzrolidin-1-yl)benzofuran-6-carboxamide;
N-[(1S, 2R, 4R)-7-azabicyclo[2.2.1]hept-2-yl]-2-(piperidin-1-yl)benzofuran-6-carboxamide;
N-[(1S, 2R, 4R)-7-azabicyclo[2.2.1]hept-2-yl]-2-(morpholin-4-yl)benzofuran-6-carboxamide;
N-[(1S, 2R, 4R)-7-azabicyclo[2.2.1]hept-2-yl]-2-(thiomorpholin-4yl)benzofuran-6-carboxamide;
N-[(1S, 2R, 4R)-7-azabicyclo[2.2.1]hept-2-yl]-2-(piperazin-1-yl)benzofuran-6-carboxamide;
N-[(1S, 2R, 4R)-7-azabicyclo[2.2.1]hept-2-yl]-3-vinylbenzofuran-5-carboxamide;
N-[(1S, 2R, 4R)-7-azabicyclo[2.2.1]hept-2-yl]-3-ethynylbenzofuran-5-carboxamide;
N-[(1S, 2R, 4R)-7-azabicyclo[2.2.1]hept-2-yl]-3-prop-1-ynylbenzofuran-5-carboxamide;
N-[(1S, 2R, 4R)-7-azabicyclo[2.2.1]hept-2-yl]-3-cyanobenzofuran-5-carboxamide;
N-[(1S, 2R, 4R)-7-azabicyclo[2.2.1]hept-2-yl]-3-chlorobenzofuran-5-carboxamide;
N-[(1S, 2R, 4R)-7-azabicyclo[2.2.1]hept-2-yl]-3-fluorobenzofuran-5-carboxamide;
N-[(1S, 2R, 4R)-7-azabicyclo[2.2.1]hept-2-yl]-3-iodobenzofuran-5-carboxamide;
N-[(1S, 2R, 4R)-7-azabicyclo[2.2.1]hept-2-yl]-3-trifluoromethylbenzofuran-5-carboxamide;
N-[(1S, 2R, 4R)-7-azabicyclo[2.2.1]hept-2-yl]-3-(pyrrolidin-1-yl)benzofuran-5-carboxamide;
N-[(1S, 2R, 4R)-7-azabicyclo[2.2.1]hept-2-yl]-3-(piperidin-1-yl)benzofuran-5-carboxamide;
N-[(1S, 2R, 4R)-7-azabicyclo[2.2.1]hept-2-yl]-3-(morpholin-4-yl)benzofuran-5-carboxamide;
N-[(1S, 2R, 4R)-7-azabicyclo[2.2.1]hept-2-yl]-3-(thiomorpholin-4yl)benzofuran-5-carboxamide;
N-[(1S, 2R, 4R)-7-azabicyclo[2.2.1]hept-2-yl]-3-(piperazin-1-yl)benzofuran-5-carboxamide;

The compound of Formula I, where the compound is any one or more or combination of the following as the free base, or pharmaceutally acceptable salt thereof:
N-[(1S, 2R, 4R)-7-azabicyclo[2.2.1]hept-2-yl]-2-vinylbenzothiophene-5-carboxamide;
N-[(1S, 2R, 4R)-7-azabicyclo[2.2.1]hept-2-yl]-2-ethynylbenzothiophene-5-carboxamide;
N-[(1S, 2R, 4R)-7-azabicyclo[2.2.1]hept-2-yl]-2-prop-1-ynylbenzothiaphene-5-carboxamide;
N-[(1S, 2R, 4R)-7-azabicyclo[2.2.1]hept-2-yl]-2-cyanobenzothiophene-5-carboxamide;
N-[(1S, 2R, 4R)-7-azabicyclo[2.2.1]hept-2-yl]-2-chlorobenzothiophene-5-carboxamide;
N-[(1S, 2R, 4R)-7-azabicyclo[2.2.1]hept-2-yl]-2-fluorobenzothiophene-5-carboxamide;
N-[(1S, 2R, 4R)-7-azabicyclo[2.2.1]hept-2-yl]-2-iodobenzothiophene-5-carboxamide;
N-[(1S, 2R, 4R)-7-azabicyclo[2.2.1]hept-2-yl]-2-trifluommethylbenzothiophene-5-carboxamide;
N-[(1S, 2R, 4R)-7-azabicyclo[2.2.1]hept-2-yl]-2-(pyrrolidin-1-yl)benzothiophene-5-carboxamide;
N-[(1S, 2R, 4R)-7-azabicyclo[2.2.1]hept-2-yl]-2-(piperidin-1-yl)benzothiophene-5-carboxamide;
N-[(1S, 2R, 4R)-7-azabicyclo[2.2.1]hept-2-yl]-2-(morpholin-4-yl)benzothiophene-5-carboxamide;
N-[(1S, 2R, 4R)-7-azabicyclo[2.2.1]hept-2-yl]-2-(thiomorpholin-4yl)benzothiophene-5-carboxamide;
N-[(1S, 2R, 4R)-7-azabicyclo[2.2.1]hept-2-yl]-2-(piperazin-1-yl)benzothiophene-5-carboxamide;
N-[(1S, 2R, 4R)-7-azabicyclo[2.2.1]hept-2-yl]-2-vinylbenzothiophene-6-carboxamide;
4-methyl-N-[(1S, 2R, 4R)-7-azabicyclo[2.2.1]hept-2-yl]lbenzothiophene-6-carboxamide;
4-chloro-N-[(1S, 2R, 4R)-7-azabicyclo[2.2.1]hept-2-yl]benzothiophene-6-carboxamide;
N-[(1S, 2R, 4R)-7-azabicyclo[2.2.1]hept-2-yl]-2-ethynylbenzothiophene-6-carboxamide;
N-[(1S, 2R, 4R)-7-azabicyclo[2.2.1]hept-2-yl]-2-prop-1-ynylbenzothiophene-6-carboxamide;
N-[(1S, 2R, 4R)-7-azabicyclo[2.2.1]hept-2-yl]-2-cyanobenzothiophene-6-carboxamide;
N-[(1S, 2R, 4R)-7-azabicyclo[2.2.1]hept-2-yl]-2-chlorobenzothiophene-6-carboxamide;
N-[(1S, 2R, 4R)-7-azabicyclo[2.2.1]hept-2-yl]-2-fluorobenzothiophene-6-carboxamide;
N-[(1S, 2R, 4R)-7-azabicyclo[2.2.1]hept-2-yl]-2-iodobenzothiophene-6-carboxamide;
N-[(1S, 2R, 4R)-7-azabicyclo[2.2.1]hept-2-yl]-2-trifluoromethylbenzothiophene-6-carboxamide;
N-[(1S, 2R, 4R)-7-azabicyclo[2.2.1]hept-2-yl]-2-(pyrrolidin-1-yl)benzothiophene-6-carboxamide;
N-[(1S, 2R, 4R)-7-azabicyclo[2.2.1]hept-2-yl]-2-(piperidin-1-yl)benzothiophene-6-carboxamide;
N-[(1S, 2R, 4R)-7-azabicyclo[2.2.1]hept-2-yl]-2-(morpholin-4-yl)benzothiophene-6-carboxamide;
N-[(1S, 2R, 4R)-7-azabicyclo[2.2.1]hept-2-yl]-2-(thiomorpholin-4yl)benzothiophene-6-carboxamide;
N-[(1S, 2R, 4R)-7-azabicyclo[2.2.1]hept-2-yl]-2-(piperazin-1-yl)benzothiophene-6-carboxamide;
7-methyl-N-[(1S, 2R, 4R)-7-azabicyclo[2.2.1]hept-2-yl]benzothiophene-5-carboxamide;
7-chloro-N-[(1S, 2R, 4R)-7-azabicyclo[2.2.1]hept-2-yl]benzothiophene-5-carboxamide;
N-[(1S, 2R, 4R)-7-azabicyclo[2.2.1]hept-2-yl]-3-vinylbenzothiophene-5-carboxamide;
N-[(1S, 2R, 4R)-7-azabicyclo[2.2.1]hept-2-yl]-3-ethynylbenzothiophene-5-carboxamide;
N-[(1S, 2R, 4R)-7-azabicyclo[2.2.1]hept-2-yl]-3-prop-1-ynylbenzothiophene-5-carboxamide;
N-[(1S, 2R, 4R)-7-azabicyclo[2.2.1]hept-2-yl]-3-cyanobenzothiophene-5-carboxamide;
N-[(1S, 2R, 4R)-7-azabicyclo[2.2.1]hept-2-yl]-3-chlorobenzothiophene-5-carboxamide;
N-[(1S, 2R, 4R)-7-azabicyclo[2.2.1]hept-2-yl]-3-fluorobenzothiophene-5-carboxamide;
N-[(1S, 2R, 4R)-7-azabicyclo[2.2.1]hept-2-yl]-3-iodobenzothiophene-5-carboxamide;
N-[(1S, 2R, 4R)-7-azabicyclo[2.2.1]hept-2-yl]-3-trifluoromethylbenzothiophene-5-carboxamide;
N-[(1S, 2R, 4R)-7-azabicyclo[2.2.1]hept-2-yl]-3-(pyrrolidin-1-yl)benzothiophene-5-carboxamide;
N-[(1S, 2R, 4R)-7-azabicyclo[2.2.1]hept-2-yl]-3-(piperidin-1-yl)benzothiophene-5-carboxamide;
N-[(1S, 2R, 4R)-7-azabicyclo[2.2.1]hept-2-yl]-3-(morpholin-4-yl)benzothiophene-5-carboxamide;
N-[(1S, 2R, 4R)-7-azabicyclo[2.2.1]hept-2-yl]-3-(thiomorpholin-4yl)benzothiophene-5-carboxamide;
N-[(1S, 2R, 4R)-7-azabicyclo[2.2.1]hept-2-yl]-3-(piperazin-1-yl)benzothiophene-5-carboxamide;

The compound of Formula I, where the compound is any one or more or combination of the following as the free base, or phatmaceutally acceptable salt thereof:
N-[(1S, 2R, 4R)-7-azabicyclo[2.2.1]hept-2-yl]-3-vinylfuro[2,3-c]pyridine-5-carboxamide;
7-methyl-N-[(1S, 2R, 4R)-7-azabicyclo[2.2.1]hept-2-yl]futo[2,3-c]pyridine-5-carboxamide;
7-chloro-N-[(1S, 2R, 4R)-7-azabicyclo[2.2.1]hept-2-yl]furo[2,3-c]pyridine-5-carboxamide;
N-[(1S, 2R, 4R)-7-azabicyclo[2.2.1]hept-2-yl]-3-ethynylfuro[2,3-c]pyridine-5-carboxamide;
N-[(1S, 2R, 4R)-7-azabicyclo[2.2.1]hept-2-yl]-3-prop-1-ynylfuro[2,3-c]pyridine-5-carboxamide;
N-[(1S, 2R, 4R)-7-azabicyclo[2.2.1]hept-2-yl]-3-cyanofuro[2,3-c]pyridine-5-carboxamide;
N-[(1S, 2R, 4R)-7-azabicyclo[2.2.1]hept-2-yl]-3-fluorofuro[2,3-c]pyridine-5-carboxamide;
N-[(1S, 2R, 4R)-7-azabicyclo[2.2.1]hept-2-yl]-3-iodofuro[2,3-c]pyridine-5-carboxamide;
N-[(1S, 2R, 4R)-7-azabicyclo[2.2.1]hept-2-yl]-3-trifluoromethylfuro[2,3-c]pyridine-5-carboxamide;
N-[(1S, 2R, 4R)-7-azabicyclo[2.2.1]hept-2-yl]-3-(pyrrolidin-1-yl)furo[2,3-c]pyridine-5-carboxamide;
N-[(1S, 2R, 4R)-7-azabicyclo[2.2.1]hept-2-yl]-3-(piperidin-1-yl)furo[2,3-c]pyridine-5-carboxamide;
N-[(1S, 2R, 4R)-7-azabicyclo[2.2.1]hept-2-yl]-3-(morpholin-4-yl)furo[2,3-c]pyridine-5-carboxamide;
N-[(1S, 2R, 4R)-7-azabicyclo[2.2.1]hept-2-yl]-3-(thiomorpholin-4yl)furo[2,3-c]pyridine-5-carboxamide;
N-[(1S, 2R, 4R)-7-azabicyclo[2.2.1]hept-2-yl]-3-(piperazin-1-yl)furo[2,3-c]pyridine-5-carboxamide;
N-[(1S, 2R, 4R)-7-azabicyclo[2.2.1]hept-2-yl]-2-vinylfuro[3,2-c]pyridine-6-carboxamide;
4-methyl-N-[(1S, 2R, 4R)-7-azabicyclo[2.2.1]hept-2-yl]furo[3,2-c]pyridine-6-carboxamide;
4-chloro-N-[(1S, 2R, 4R)-7-azabicyclo[2.2.1]hept-2-yl]furo[3,2-c]pyridine-6-carboxamide;
N-[(1S, 2R, 4R)-7-azabicyclo[2.2.1]hept-2-yl]-2-ethynylfuro[3,2-c]pyridine-6-carboxamide;
N-[(1S, 2R, 4R)-7-azabicyclo[2.2.1]hept-2-yl]-2-prop-1-ynylfuro[3,2-c]pyridine-6-carboxamide;
N-[(1S, 2R, 4R)-7-azabicyclo[2.2.1]hept-2-yl]-2-cyanofuro[3,2-c]pyridine-6-carboxamide;
N-[(1S, 2R, 4R)-7-azabicyclo[2.2.1]hept-2-yl]-2-chlorofuro[3,2-c]pyridine-6-carboxamide;
N-[(1S, 2R, 4R)-7-azabicyclo[2.2.1]hept-2-yl]-2-fluorofuro[3,2-c]pyridine-6-carboxamide;
N-[(1S, 2R, 4R)-7-azabicyclo[2.2.1]hept-2-yl]-2-iodofuro[3,2-c]pyridine-6-carboxamide;
N-[(1S, 2R, 4R)-7-azabicyclo[2.2.1]hept-2-yl]-2-trifluoromethylfuro[3,2-c]pyridine-6-carboxamide;
N-[(1S, 2R, 4R)-7-azabicyclo[2.2.1]hept-2-yl]-2-(pyrrolidin-1-yl)furo[3,2-c]pyridine-6-carboxamide;
N-[(1S, 2R, 4R)-7-azabicyclo[2.2.1]hept-2-yl]-2-(piperidin-1-yl)furo[3,2-c]pyridine-6-carboxamide;
N-[(1S, 2R, 4R)-7-azabicyclo[2.2.1]hept-2-yl]-2-(morpholin-4-yl)furo[3,2-c]pyridine-6-carboxamide;
N-[(1S, 2R, 4R)-7-azabicyclo[2.2.1]hept-2-yl]-2-(thiomorpholin-4yl)furo[3,2-c]pyridine-6-carboxamide;
N-[(1S, 2R, 4R)-7-azabicyclo[2.2.1]hept-2-yl]-2-(piperazin-1-yl)furo[3,2-c]pyridine-6-carboxamide;
N-[(1S, 2R, 4R)-7-azabicyclo[2.2.1]hept-2-yl]-2-vinylfuro[2,3-c]pyridine-5-carboxamide;
N-[(1S, 2R, 4R)-7-azabicyclo[2.2.1]hept-2-yl]-2-ethynylfuro[2,3-c]pyridine-5-carboxamide;
N-[(1S, 2R, 4R)-7-azabicyclo[2.2.1]hept-2-yl]-2-prop-1-ynylfuro[2,3-c]pyridine-5-carboxamide;
N-[(1S, 2R, 4R)-7-azabicyclo[2.2.1]hept-2-yl]-2-cyanofuro[2,3-c]pyridine-5-carboxamide;
N-[(1S, 2R, 4R)-7-azabicyclo[2.2.1]hept-2-yl]-2-chlorofuro[2,3-c]pyridine-5-carboxamide;
N-[(1S, 2R, 4R)-7-azabicyclo[2.2.1]hept-2-yl]-2-fluorofuro[2,3-c]pyridine-5-carboxamide;
N-[(1S, 2R, 4R)-7-azabicyclo[2.2.1]hept-2-yl]-2-iodofuro[2,3-c]pyridine-5-carboxamide;
N-[(1S, 2R, 4R)-7-azabicyclo[2.2.1]hept-2-yl]-2-trifluoromethylfuro[2,3-c]pyridine-5-carboxamide;
N-[(1S, 2R, 4R)-7-azabicyclo[2.2.1]hept-2-yl]-2-(pyrrolidin-1-yl)furo[2,3-c]pyridine-5-carboxamide;
N-[(1S, 2R, 4R)-7-azabicyclo[2.2.1]hept-2-yl]-2-(piperidin-1-yl)furo[2,3-c]pyridine-5-carboxamide;
N-[(1S, 2R, 4R)-7-azabicyclo[2.2.1]hept-2-yl]-2-(morpholin-4-yl)furo[2,3-c]pyridine-5-carboxamide;
N-[(1S, 2R, 4R)-7-azabicyclo[2.2.1]hept-2-yl]-2-(thiomorpholin-4yl)furo[2,3-c]pyridine-5-carboxamide;
N-[(1S, 2R, 4R)-7-azabicyclo[2.2.1]hept-2-yl]-2-(piperazin-1-yl)furo[2,3-c]pyridine-5-carboxamide;

The compound of Formula I, where the compound is any one or more or combination of the following as the free base, or pharmaceutally acceptable salt thereof:
7-methyl-N-[(1S, 2R, 4R)-7-azabicyclo[2.2.1]hept-2-yl]thieno[2,3-c]pyridine-5-carboxamide;
7-chloro-N-[(1S, 2R, 4R)-7-azabicyclo[2.2.1]hept-2-yl]thieno[2,3-c]pyridine-5-carboxamide;
N-[(1S, 2R, 4R)-7-azabicyclo[2.2.1]hept-2-yl]-3-vinylthieno[2,3-c]pyridine-5-carboxamide;
N-[(1S, 2R, 4R)-7-azabicyclo[2.2.1]hept-2-yl]-3-ethynylthieno[2,3-c]pyridine-5-carboxamide;
N-[(1S, 2R, 4R)-7-azabicyclo[2.2.1]hept-2-yl]-3-prop-1-ynylthieno[2,3-c]pyridine-5-carboxamide;
N-[(1S, 2R, 4R)-7-azabicyclo[2.2.1]hept-2-yl]-3-cyanothieno[2,3-c]pyridine-5-carboxamide;
N-[(1S, 2R, 4R)-7-azabicyclo[2.2.1]hept-2-yl]-3-chlorothieno[2,3-c]pyridine-5-carboxamide;
N-[(1S, 2R, 4R)-7-azabicyclo[2.2.1]hept-2-yl]-3-fluorothieno[2,3-c]pyridine-5-carboxamide;
N-[(1S, 2R, 4R)-7-azabicyclo[2.2.1]hept-2-yl]-3-iodothieno[2,3-c]pyridine-5-carboxamide;
N-[(1S, 2R, 4R)-7-azabicyclo[2.2.1]hept-2-yl]-3-trifluoromethylthieno[2,3-c]pyridine-5-carboxamide;
N-[(1S, 2R, 4R)-7-azabicyclo[2.2.1]hept-2-yl]-3-(pyrrolidin-1-yl)thieno[2,3-c]pyridine-5-carboxamide;
N-[(1S, 2R, 4R)-7-azabicyclo[2.2.1]hept-2-yl]-3-(piperidin-1-yl)thieno[2,3-c]pyridine-5-carboxamide;
N-[(1S, 2R, 4R)-7-azabicyclo[2.2.1]hept-2-yl]-3-(morpholin-4-yl)thieno[2,3-c]pyridine-5-carboxamide;
N-[(1S, 2R, 4R)-7-azabicyclo[2.2.1]hept-2-yl]-3-(thiomorpholin-4yl)thieno[2,3-c]pyridine-5-carboxamide;
N-[(1S, 2R, 4R)-7-azabicyclo[2.2.1]hept-2-yl]-3-(piperazin-1-yl)thieno[2,3-c]pyridine-5-carboxamide;
N-[(1S, 2R, 4R)-7-azabicyclo[2.2.1]hept-2-yl]-3-(4-methylpiperazin-1-yl)thieno[2,3-c]pyridine-5-carboxamide;
N-[(1S, 2R, 4R)-7-azabicyclo[2.2.1]hept-2-yl]-2-vinylthieno[2,3-c]pyridine-5-carboxamide;
N-[(1S, 2R, 4R)-7-azabicyclo[2.2.1]hept-2-yl]-2-ethynylthieno[2,3-c]pyridine-5-carboxamide;
N-[(1S, 2R, 4R)-7-azabicyclo[2.2.1]hept-2-yl]-2-prop-1-ynylthieno[2,3-c]pyridine-5-carboxamide;
N-[(1S, 2R, 4R)-7-azabicyclo[2.2.1]hept-2-yl]-2-cyanotl]ieno[2,3-c]pyndme-5-carboxamide;
N-[(1S, 2R, 4R)-7-azabicyclo[2.2.1]hept-2-yl]-2-chlorothieno[2,3-c]pyridine-S-carboxamide;
N-[(1S, 2R, 4R)-7-azabicyclo[2.2.1]hept-2-yl]-2-fluorothieno[2,3-c]pyridine-5-carboxamide;
N-[(1S, 2R, 4R)-7-azabicyclo[2.2.1]hept-2-yl]-2-iodothieno[2,3-c]pyridine-5-carboxamide;
N-[(1S, 2R, 4R)-7-azabicyclo[2.2.1]hept-2-yl]-2-trifluoromethylthieno[2,3-c]pyridine-5-carboxamide;
N-[(1S, 2R, 4R)-7-azabicyclo[2.2.1]hept-2-yl]-2-(pyrrolidin-1-yl)thieno[2,3-c]pyridine-5-carboxamide;
N-[(1S, 2R, 4R)-7-azabicyclo[2.2.1]hept-2-yl]-2-(piperidin-1-yl)thieno[2,3-c]pyridine-5-carboxamide;
N-[(1S, 2R, 4R)-7-azabicyclo[2.2.1]hept-2-yl]-2-(morpholin-4-yl)thieno[2,3-c]pyridine-5-carboxamide;
N-[(1S, 2R, 4R)-7-azabicyclo[2.2.1]hept-2-yl]-2-(thiomorpholin-4yl)thieno[2,3-c]pyridine-5-carboxamide;
N-[(1S, 2R, 4R)-7-azabicyclo[2.2.1]hept-2-yl]-2-(piperazin-1-yl)thieno[2,3-c]pyridine-5-carboxamide;
N-[(1S, 2R, 4R)-7-azabicyclo[2.2.1]hept-2-yl]-2-vinylthieno[3,2-c]pyridine-6-carboxamide;
4-methyl-N-[(1S, 2R, 4R)-7-azabicyclo[2.2.1]hept-2-yl]thieno[3,2-c]pyridine-6-carboxamide;
4-chloro-N-[(1S, 2R, 4R)-7-azabicyclo[2.2.1]hept-2-yl]thieno[3,2.-c]pyridine-6-carboxamide;
N-[(1S, 2R, 4R)-7-azabicyclo[2.2.1]hept-2-yl]-2-ethynylthieno[3,2-c]pyridine-6-carboxamide;
N-[(1S, 2R, 4R)-7-azabicyclo[2.2.1]hept-2-yl]-2-prop-1-ynylthieno[3,2-c]pyridine-6-carboxamide;
N-[(1S, 2R, 4R)-7-azabicyclo[2.2.1]hept-2-yl]-2-cyanothieno[3,2-c]pyridine-6-carboxamide;
N-[(1S, 2R, 4R)-7-azabicyclo[2.2.1]hept-2-yl]-2-chlorothieno[3,2-c]pyridine-6-carboxamide;
N-[(1S, 2R, 4R)-7-azabicyclo[2.2.1]hept-2-yl]-2-fluorothieno[3,2-c]pyridine-6-carboxamide;
N-[(1S, 2R, 4R)-7-azabicyclo[2.2.1]hept-2-yl]-2-iodothieno[3,2-c]pyridine-6-carboxamide;
N-[(1S, 2R, 4R)-7-azabicyclo[2.2.1]hept-2-yl]-2-trifluoromethylthieno[3,2-c]pyridine-6-carboxamide;
N-[(1S, 2R, 4R)-7-azabicyclo[2.2.1]hept-2-yl]-2-(pyrrolidin-1-yl)thieno[3,2-c]pyridine-6-carboxamide;
N-[(1S, 2R, 4R)-7-azabicyclo[2.2.1]hept-2-yl]-2-(piperidin-1-yl)thieno[3,2-c]pyridine-6-carboxamide;
N-[(1S, 2R, 4R)-7-azabicyclo[2.2.1]hept-2-yl]-2-(morpholin-4-yl)thieno[3,2-c]pyridine-6-carboxamide;
N-[(1S, 2R, 4R)-7-azabicyclo[2.2.1]hept-2-yl]-2-(thiomorpholin-4yl)thieno[3,2-c]pyridine-6-carboxamide;
N=[(1S, 2R, 4R)-7-azabicyclo[2.2.1]hept-2-yl]-2-(piperazin-1-yl)thieno[3,2-c]pyridine-6-carboxamide;

The compound of Formula I, where the compound is any one or more or combination of the following as the free base, or pharmaceutally acceptable salt thereof:
N-[(1S, 2R, 4R)-7-azabicyclo[2.2.1]hept-2-yl]-1H-indazole-5-carboxamide;
N-[(1S, 2R, 4R)-7-azabicyclo[2.2.1]hept-2-yl]-1H-indazole-6-carboxamide;
N-[(1S, 2R, 4R)-7-azabicyclo[2.2.1]hept-2-yl]-3-methyl-1H-indazole-6-carboxamide;
N-[(1S, 2R, 4R)-7-azabicyclo[2.2.1Jhept-2-yl]-3-methyl-1H-indazole-5-carboxamide;
N-[(1S, 2R, 4R)-7-azabicyclo[2.2.1]hept-2-yl]-3-ethyl-1H-indazole-6-carboxamide;
N-[(1S, 2R, 4R)-7-azabicyclo[2.2.1]hept-2-yl]-3-ethyl-1H-indazole-5-carboxamide;
N-[(1S, 2R, 4R)-7-azabicyclo[2.2.1]hept-2-yl]-1,2-benzisothiazole-6-carboxamide;
N-[(1S, 2R, 4R)-7-azabicyclo[2.2.1]hept-2-yl]-1,2-benzisothiazole-5-carboxamide;
N-[(1S, 2R, 4R)-7-azabicyclo[2.2.1]hept-2-yl]-3-ethyl-1,2-benzisothiazole-6-carboxamide;
N-[(1S, 2R, 4R)-7-azabicyclo[2.2.1]hept-2-yl]-3-ethyl-1,2-benzisothiazole-5-carboxamide;
N-[(1S, 2R, 4R)-7-azabicyclo[2.2.1]hept-2-yl]-3-methyl-1,2-benzisothiazole-6-carboxamide;
N-[(1S, 2R, 4R)-7-azabicyclo[2.2.1]hept-2-yl]-3-methyl-1,2-benzisothiazole-5-carboxamide;
N-[(1S, 2R, 4R)-7-azabicyclo[2.2.1]hept-2-yl]-2-methyl-1,3-benzothiazole-6-carboxamide;
N-[(1S, 2R, 4R)-7-azabicyclo[2.2.1]hept-2-yl]-2-methyl-1,3-benzothiazole-5-carboxamide;
N-[(1S, 2R, 4R)-7-azabicyclo[2.2.1]hept-2-yl]-2-ethyl-1,3-benzothiazole-6-carboxamide;
N-[(1S, 2R, 4R)-7-azabicyclo[2.2.1]hept-2-yl]-2-ethyl-1,3-benzothiazole-5-carboxamide;
N-[(1S, 2R, 4R)-7-azabicyclo[2.2.1]hept-2-yl]-2-methyl-1,3-benzodioxole-5-carboxamide;
N-[(1S, 2R, 4R)-7-azabicyclo[2.2.1]hept-2-yl]-2-ethyl-1,3-benzodioxole-5-carboxamide;
N-[(1S, 2R, 4R)-7-azabicyclo[2.2.1]hept-2-yl]-2,2-dimethyl-1,3-benzodioxole-5-carboxamide;
N-[(1S, 2R, 4R)-7-azabicyclo[2.2.1]hept-2-yl]-2H-isoindole-5-carboxamide;
N-[(1S, 2R, 4R)-7-azabicyclo[2.2.1]hept-2-yl]-2H-isoindole-6-carboxamide;
N-[(1S, 2R, 4R)-7-azabicyclo[2.2.1]hept-2-yl]-1H-benzimidazole-5-carboxamide;
N-[(1S, 2R, 4R)-7-azabicyclo[2.2.1]hept-2-yl]-1H-benzimidazole-6-carboxamide;
N-[(3R)-1-azabicyclo[2.2.2]oct-3-yl][1,3]thiazolo[5,4-c]pyridine-6-carboxamide;
N-[(3R)-1-azabicyclo[2.2.2]oct-3-yl][1,3]thiazolo[4,5-c]pyridine-6-carboxamide;
N-[(3R)-1-azabicyclo[2.2.2]oct-3-yl][1,3]dioxolo[4,5-c]pyndme-6-carboxamide;
N-[(3R)-1-azabicyclo[2.2.2]oct-3-yl][1,3]oxazolo[4,5-c]pyridine-6-carboxamide;
N-[(1S, 2R, 4R)-7-azabicyclo[2.2.1]hept-2-yl]-1H-imidazo[4,5-c]pyridine-6-carboxamide;
N-[(1S, 2R, 4R)-7-azabicyclo[2.2.1]hept-2-yl]-1,3-benzothiazole-5-carboxamide;
N-[(1S, 2R, 4R)-7-azabicyclo[2.2.1]hept-2-yl]-2-bromo-1,3-benzothiazole-5-carboxamide;
N-[(1S, 2R, 4R)-7-azabicyclo[2.2.1]hept-2-yl]-2-chloro-1,3-benzothiazole-5-carboxamide;
N-[(1S, 2R, 4R)-7-azabicyclo[2.2.1]hept-2-yl]-2-morpholin-4-yl-1,3-benzothiazole-5-carboxamide;
N-[(1S, 2R, 4R)-7-azabicyclo[2.2.1]hept-2-yl]-2-piperazin-1-yl-1,3-benzothiazole-5-carboxamide;
N-[(1S, 2R, 4R)-7-azabicyclo [2.2.1]hept-2-yl]-1,3-benzothiazole-6-carboxamide;
N-[(1S, 2R, 4R)-7-azabicyclo[2.2.1]hept-2-yl]-2-bromo-1,3-benzothiazole-6-carboxamide;
N-[(1S, 2R, 4R)-7-azabicyclo[2.2.1]hept-2-yl]-2-chloro-1,3-benzothiazole-6-carboxamide;
N-[(1S, 2R, 4R)-7-azabicyclo[2.2.1]hept-2-yl]-2-morpholin-4-yl-1,3-benzothiazole-6-carboxamide; or
N-[(1S, 2R, 4R)-7-azabicyclo[2.2.1]hept-2-yl]-2-piperazin-1-yl-1,3-benzothiazole-6-carboxamide.

The compound of Formula I, where (c) is any one or more or combination of the following: isoquinolin-3-yl, quinoline-3-yl, 2,3-dihydro[1,4]dioxino[2,3-c]pyridine-7-yl, 2,3-dihydro-1,4-benzodioxine-6-yl, chromane-6-yl, 2H-chromene-6-yl, 2H-pyrano[2,3-c]pyridine-6-yl, 2H-pyrano[2,3-c]pyridine-7-yl, 3,4-dihydro-2H-pyrano[2,3-c]pyridine-6-yl, or 3,4-dihydro-2H-pyrano[2,3-c]pyridine-7-yl, any of which is optionally substituted with up to 4 substituents independently selected from F, Cl, Br, -CN, -NO₂, alkyl, having from 1-6 carbon atoms, halogenated alkyl having from 1-6 carbon atoms, alkenyl having from 2-6 carbon atoms, halogenated alkenyl having from 2-6 carbon atoms, alkynyl having from 2-6 carbon atoms, halogenated alkynyl having from 2-6 carbon atoms, heterocycloalkyl having 4-7 atoms with 1-2 atoms within the ring being -S- , -N[R₁₈], or -O- or, halogenated heterocycloalkyl having 4-7 atoms with 1-2 atoms within the ring being -S-, -N[R₁₈], or -O-.

The coupond of Formula I, where the compound is N-[(1S, 2R, 4R)-7-azabicyclo[2.2.1]hept-2-yl]isoquinoline-3-carboxamide; or a pharmaceutically acceptable salt thereof.

The compound of Formula I, where the compound is any one or more or combination of the following as the free base, or pharmaceutally acceptable salt thereof:
N-[(1S, 2R, 4R)-7-azabicyclo[2.2.1]hept-2-yl]-6-methylisoquinoline-3-carboxamide;
N-[(1S, 2R, 4R)-7-azabicyclo[2.2.1]hept-2-yl]-6-methylisoquinoline-3-carboxamide;
N-[(1S, 2R, 4R)-7-azabicyclo[2.2.1]hept-2-yl]-6-trifluoromethylisoquinoline-3-carboxamide;
N-[(1S, 2R, 4R)-7-azabicyclo[2.2.1]hept-2-yl]-6-chloroisoquinoline-3-carboxamide;
N-[(1S, 2R, 4R)-7-azabicyclo[2.2.1]hept-2-yl]-6-bromoisoquinoline-3-carboxamide;
N-[(1S, 2R, 4R)-7-azabicyclo[2.2.1]hept-2-yl]-6-ffuoroisoquinoline-3-carboxamide;
N-[(1S, 2R, 4R)-7-azabicyclo[2.2.1]hept-2-yl]-6-iodoisoquinoline-3-carboxamide;
N-[(1S, 2R, 4R)-7-azabicyclo[2.2.1]hept-2-yl]-6-ethynyhsoquinoline-3-carboxamide;
N-[(1S, 2R, 4R)-7-azabicyclo[2.2.1]hept-2-yl]-6-cyanoisoquinoline-3-carboxamide;
N-[(1S, 2R, 4R)-7-azabicyclo[2.2.1]hept-2-yl]-6-ethenylisoquinoline-3-carboxamide;
N-[(1S, 2R, 4R)-7-azabicyclo[2.2.1]hept-2-yl]-6-nitroisoquinoline-3-carboxamide;
N-[(1S, 2R, 4R)-7-azabicyclo[2.2.1]hept-2-yl]2,3-dihydro-1,4-benzodioxane-6-carboxamide;
N-[(1S, 2R, 4R)-7-azabicyclo[2.2.1]hept-2-yl]chromane-6-carboxamide; or a pharmaceutically acceptable salt thereof.

The compound of Formula I, where (d) is thieno[3,4-c]pyridin-6-yl, furo[3,4-c]pyridin-6-yl, 2-benzothiophen-5-yl, 2-benzothiophen-6-yl, 2-benzofuran-5-yl, or 2-benzofuran-6-yl, any of which is optionally substituted with up to 4 substituents independently selected from F, Cl, Br, -CN, -NO₂, alkyl having from 1-6 carbon atoms, halogenated alkyl having from 1-6 carbon atoms, alkenyl having from 2-6 carbon atoms, halogenated alkenyl having from 2-6 carbon atoms, alkynyl having from 2-6 carbon atoms, halogenated alkynyl having from 2-6 carbon atoms, heterocycloalkyl having 4-7 atoms with 1-2 atoms within the ring being -S- , -N(R₁₈), or -O-, or, halogenated heterocycloalkyl having 4-7 atoms with 1-2 atoms within the ring being -S- , -N(R₁₈), or -O-.

The compound of Formula I, where the compound is N-[(1S, 2R, 4R)-7-azabicyclo[2.2.1]hept-2-yl]thieno[3,4-c]pyridine-6-carboxamide; or a pharmaceutically acceptable salt thereof.

The compound of Formula I, where the compound is any one or more or combination of the following as the free base, or pharmaceutally acceptable salt thereof: N-[(1S, 2R, 4R)-7-azabicyclo[2.2.1]hept-2-yl]furo[3,4-c]pyridine-6-carboxamide, N-[(1S, 2R, 4R)-7-azabicyclo[2.2.1]hept-2-yl]-2-benzofuran-5-carboxamide; or N-[(1S, 2R, 4R)-7-azabicyclo[2.2.1]hept-2-yl]-2-benzothiophen-5-carboxamide.

The compound of Formula I, where (e) is any one or more or combination of the following: [1]benzothieno[2,3-c]pyridin-3-yl, [1]benzothieno[3,2-c]pyridin-3-yl, [1]benzofuro[3,2-c]pyridin-3-yl, [1]benzofuro[2,3-c]pyridin-3-yl, dibenzo[b,d]thiophen-2-yl, or dibenzo[b,d]furan-2-yl, any of which is optionally substituted with up to 4 substituents independently selected from F, Cl, Br, -CN, -NO₂, alkyl having from 1-6 carbon atoms, halogenated alkyl having from 1-6 carbon atoms, alkenyl having from 2-6 carbon atoms, halogenated alkenyl having from 2-6 carbon atoms, alkynyl having from 2-6 carbon atoms, halogenated alkynyl having from 2-6 carbon atoms, heterocycloalkyl having 4-7 atoms with 1-2 atoms within the ring being -S- , -N(R₁₈)-, or -O-, or, halogenated heterocycloalkyl having 4-7 atoms with 1-2 atoms within the ring being -S- , -N(R₁₈)-, or -O-.

The compound of Formula I, where the compound is any one or more or combination of the following as the free base, or pharmaceutally acceptable salt thereof: N-[(1S, 2R, 4R)-7-azabicyclo[2.2.1]hept-2-yl]dibenzo[b,d]thiophene-2-carboxamide; N-[(1S, 2R, 4R)-7-azabicyclo[2.2.1]hept-2-yl]benzothieno[2,3-c]pyridine-3-carboxamide; N-[(1S, 2R, 4R)-7-azabicyclo[2.2.1]hept-2-yl]benzothieno[3,2-c]pyridine-3-carboxamide; N-[(1S, 2R, 4R)-7-azabicyclo[2.2.1]hept-2-yl]dibenzo[b,d]furan-2-carboxamide; N-[(1S, 2R, 4R)-7-azabicyclo[2.2.1]hept-2-yl]benzofuro[3,2-c]pyridine-3-carboxamide; N-[(1S, 2R, 4R)-7-azabicyclo[2.2.1]hept-2-yl]benzofuro[2,3-c]pyridine-3-carboxamide; or a pharmaceutically acceptable salt thereof.

The present invention also includes a pharmaceutical composition comprising a compound of Formula I or a pharmaceutically acceptable salt thereof and a pharmaceutically acceptable excipient. The pharmaceutical composition is administered rectally, topically, orally, sublingually, or parenterally for a therapeutically effective interval. The pharmaceutical composition is administered to deliver a compound of Formula I in an amount of from about 0.001 to about 100mg/kg of body weight of said mammal per day. The pharmaceutical composition is also administered to deliver a compound of Formula I in an amount of from about 0.1 to about 50mg/kg of body weight of said mammal per day.

A pharmaceutical composition comprising a compound of Formula I or a pharmaceutically acceptable salt thereof, an anti-psychotic agent, and a pharmaceutically acceptable excipient. The pharmaceutical composition is administered to independently administer said compound and said agent rectally, topically, orally, sublingually, or parenterally for a therapeutically effective interval. The pharmaceutical composition is administered to deliver a compound of Formula I in an amount of from about 0.001 to about 100mg/kg of body weight of said mammal per day. The pharmaceutical composition is also administered to deliver a compound of Formula I in an amount of from about 0.1 to about 50mg/kg of body weight of said mammal per day.

The present invention also includes a use of a compound according to Formula I or pharmaceutically acceptable salt thereof for the preparation of a medicament for treating a disease or condition, wherein the mammal would receive symptomatic relief from the administration of a therapeutically effective amount of α7 nicotinic acetylcholine receptor agonist.

The present invention also includes a use of a compound according to Formula I or pharmaceutically acceptable salt thereof for the preparation of a medicament for treating a disease or condition, wherein the mammal would receive symptomatic relief from the administration of a therapeutically effective amount of α7 nicotinic acetylcholine receptor agonist, wherein the disease, or condition is any one or more or combination of the following: cognitive and attention deficit symptoms of Alzheimer's, neurodegeneration associated with diseases such as Alzheimer's disease, pre-senile dementia (mild cognitive impairment), senile dementia, schizophrenia, psychosis, attention deficit disorder, attention deficit hyperactivity disorder, depression, anxiety, general anxiety disorder, post traumatic stress disorder, mood and affective disorders, amyotrophic lateral sclerosis, borderline personality disorder, traumatic brain injury, behavioral and cognitive problems in general and associated with brain tumors, AIDS dementia complex, dementia associated with Down's syndrome, dementia associated with Lewy Bodies, Huntington's disease, Parkinson's disease, tardive dyskinesia, Pick's disease, dysregulation of food intake including bulemia and anorexia nervosa, withdrawal symptoms associated with smoking cessation and dependant drug cessation, Gilles de la Tourette's Syndrome, age-related macular degeneration, glaucoma, neurodegeneration associated with glaucoma, or symptoms associated with pain.

Further aspects and embodiments of the invention may become apparent to those skilled in the art from a review of the following detailed description, taken in conjunction with the examples and the appended claims. While the invention is susceptible of embodiments in various forms, described hereafter are specific embodiments of the invention with the understanding that the present disclosure is intended as illustrative, and is not intended to limit the invention to the specific embodiments described herein.

### DETAILED DESCRIPTION OF THE INVENTION

Alkyl is both straight- and branched-chain moieties having from 1-6 carbon atoms;
Halogenated alkyl is an alkyl moiety having from 1-6 carbon atoms and having 1 to (2n+1) substituent(s) independently selected from F, Cl, Br, or I where n is the maximum number of carbon atoms in the moiety;
Cycloalkyl is a cyclic alkyl moiety having from 3-6 carbon atoms;
Alkenyl is straight- and branched-chain moieties having from 2-6 carbon atoms and having at least one carbon-carbon double bond;
Halogenated alkenyl is an unsaturated alkenyl moiety having from 2-6 carbon atoms and having 1 to (2n-1) substituent(s) independently selected from F, Cl, Br, or I where n is the maximum number of carbon atoms in the moiety;
Alkynyl is straight- and branched-chained moieties having from 2-6 carbon atoms and having at least one carbon-carbon triple bond;
Halogenated alkynyl is an unsaturated alkynyl moiety having from 3-6 carbon atoms and having 1 to (2n-3) substituent(s) independently selected from F, Cl, Br, or I where n is the maximum number of carbon atoms in the moiety;
Halogenated cycloalkyl is a cyclic moiety having from 3-6 carbon atoms and having 1-4 substituents independently selected from F, Cl, Br, or I;
Heterocycloalkyl is a cyclic moiety having 4-7 atoms with 1-2 atoms within the ring being -S-, -N(R₁₈)-, or -O-;
Halogenated heterocycloalkyl is a cyclic moiety having from 4-7 atoms with 1-2 atoms within the ring being -S-, -N(R₁₈)-, or -O-, and having 1-4 substituents independently selected from F, Cl, Br, or I;

Surprisingly, we have found that compounds or formula (I), as defined above and their pharmaceutically acceptable salts are useful to treat any one or more or combination of the following: cognitive and attention deficit symptoms of Alzheimer's, neurodegeneration associated with diseases such as Alzheimer's disease, pre-senile dementia (mild cognitive impairment), senile dementia, schizophrenia, psychosis, attention deficit disorder, attention deficit hyperactivity disorder, depression, anxiety, general anxiety disorder, post traumatic stress disorder, mood and affective disorders, amyotrophic lateral sclerosis, borderline personality disorder, traumatic brain injury, behavioral and cognitive problems in general and associated with brain tumors, AIDS dementia complex, dementia associated with Down's syndrome, dementia associated with Lewy Bodies, Huntington's disease, Parkinson's disease, tardive dyskinesia, Pick's disease, dysregulation of food intake including bulemia and anorexia nervosa, withdrawal symptoms associated with smoking cessation and dependant drug cessation, Grilles de la Tourette's Syndrome, age-related macular degeneration, glaucoma, neurodegeneration associated with glaucoma, or symptoms associated with pain.

In another aspect, the invention includes the use for the manufacture of a medicament for treating a mammal suffering from schizophrenia or psychosis of a compound or Formula I or pharmaceutically acceptable salt thereof in conjunction with antipsychotic drugs. The compounds of Formula I and the antipsychotic drugs can be administered simultaneously or at separate intervals. When administered simultaneously the compounds of Formula I and the antipsychotic drugs can be incorporated into a single pharmaceutical composition. Alternatively, two separate compositions, i.e., one containing compounds of Formula I and the other containing antipsychotic drugs, can be administered simultaneously.

The compounds of Formula I have optically active centers on the 7-azabicyclo[2.2.1]heptane ring which can exhibit a number of stereochemical configurations. The terms *exo* and *endo* are stereochemical prefixes that describe the relative configuration of a substituent on a bridge (not a bridgehead) of a bicyclic system. If a substituent is oriented toward the larger of the other bridges, it is *endo.* If a substituent is oriented toward the smaller bridge it is *exo.* Depending on the substitution on the carbon atoms, the *endo* and *exo* orientations can give rise to different stereoisomers. For instance, when carbons 1 and 4 are substituted with hydrogen and carbon 2 is bonded to a nitrogen containing species, the *endo* orientation gives rise to the possibility of a pair of enantiomers: either the1*S*, 2*S*, 4*R* isomer or its enantiomer, the 1*R*, 2*R*, 4*S* isomer. Likewise, the *exo* orientation gives rise to the possibility of another pair of stereoisomers which are diastereomeric and C-2 epimers with respect to the *endo* isomers: either the 1*R*, 2*S*, 4*S* isomer or its enantiomer, the 1*S*, 2*R*, 4*R* isomer. The compounds of this invention exist in the *exo* orientation. For example, when R₂ = R₄ = H, the absolute stereochemistry is (1*S*, 2*R*, 4*R*) for the compounds in Formula I.

Stereoselective syntheses and/or subjecting the reaction product to appropriate purification steps produces substantially optically pure materials. Suitable stereoselective synthetic procedures for producing optically pure materials are well known in the art, as are procedures for purifying racemic mixtures into optically pure fractions.

The compounds of the present invention have the *exo* orientation at the C-2 carbon and *S* configuration at the C-1 carbon and the *R* configuration at the C-2 and the C-4 carbons of the 7-azabicyclo[2.2.1]heptane ring. Unexpectedly, the inventive compounds exhibit much higher activity relative to compounds lacking the 1*S*, 2*R*, and 4*R* stereochemistry within the 7-azabicyclo[2.2.1]heptane ring system. For example, the ratio of activities for compounds having the 1*S*, 2*R*, and 4*R* configuration to other stereochemical configurations of the 7-azabicyclo[2.2.1]heptane ring system maybe greater than about 100. Surprisingly, the compounds with the 1*S*, 2*R*, and 4*R* stereochemistry exhibit preferred toxicology. Although it is desirable that the stereochemical purity be as high as possible, absolute purity is not required. For example, pharmaceutical compositions can include one or more compounds, each having an 1*S*, 2*R*, and 4*R* configuration, or mixtures of compounds having 1*S*, 2*R*, and 4R and other configurations. In mixtures of compounds, those species possessing stereochemical configurations other than 1*S*, 2*R*, and 4*R* act as diluents and tend to lower the activity of the pharmaceutical composition. Typically, pharmaceutical compositions including mixtures of compounds possess a larger percentage of species having the 1*S*, 2*R*, and 4*R* configuration relative to other configurations.

Abbreviations which are well known to one of ordinary skill in the art may be used (e.g., "Ph" for phenyl, "Me" for methyl, "Et" for ethyl, "h" for hour or hours, min for minute or minutes, and "rt" or "RT" for room temperature).

All temperatures are in degrees Centigrade.

Room temperature is within the range of 15-25 degrees Celsius.

AChR refers to acetylcholine receptor.

nAChR refers to nicotinic acetylcholine receptor.

Pre-senile dementia is also known as mild cognitive impairment.

5HT₃R refers to the serotonin-type 3 receptor.

α-btx refers to α-bungarotoxin.

FLIPR refers to a device marketed by Molecular Devices, Inc. designed to precisely measure cellular fluorescence in a high throughput whole-cell assay. (Schroeder et. al., *J. Biomolecular Screening,* 1(2), p 75-80, 1996).

TLC refers to thin-layer chromatography.

HPLC refers to high pressure liquid chromatography.

MeOH refers to methanol.

IPA refers to isopropyl alcohol.

THF refers to tetrahydrofuran.

DMSO refers to dimethylsulfoxide.

DMF refers to dimethylformamide.

EtOAc refers to ethyl acetate.

TMS refers to tetramethylsilane.

TEA refers to triethylamine.

DIEA refers to diisopropylethylamine.

MLA refers to methyllycaconitine.

Ether refers to diethyl ether.

HATU refers to O-(7-azabenzotriazol-1-yl)-N,N,N', N-tetramethyluronium hexafluorophosphate.

DBU refers to 1,8-diazabicyclo[5.4.0]undec-7-ene.

When potassium carbonate (K₂CO₃), magnesium sulfate (MgSO₄), and sodium sulfate (Na₂SO₄) are used as a drying agent, the agent is anhydrous.

The carbon atom content of various hydrocarbon-containing moieties is indicated by a prefix designating the minimum and maximum number of carbon atoms in the moiety, i.e., the prefix Cᵢ₋ⱼ indicates a moiety of the integer 'i" to the integer "j" carbon atoms, inclusive: Thus, for example, C₁₋₆ alkyl refers to alkyl of one to six carbon atoms.

Lower alkyl is both straight- and branched-chain moieties having 1-4 carbon atoms.

Halogenated lower alkyl is lower alkyl having 1 to (2n+1) substituent(s) independently selected from F, Cl, Br, or I where n is the maximum number of carbon atoms in the moiety.

Non-inclusive examples of heterocycloalkyl include, but are not limited to, tetrahydrofurano, tetrahydropyrano, morpholino, pyrrolidino, piperidino, piperazine, azetidino, azetidinono, oxindolo, dihydroimidazolo, pyrrolidino, or isoxazolinyl.

The core molecule refers to the amide or thio amide group bound to the C-2 carbon of the 7-azabicyclo[2.2.1]heptane ring:

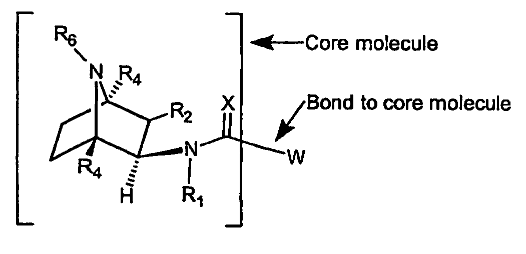

A bond to the core molecule refers to the bond from W to the carbon of the amide group of the core molecule.

Halogen or halo is F, Cl, Br, or I.

Mammal denotes human and other mammals.

Brine refers to an aqueous saturated sodium chloride solution.

IR refers to infrared spectroscopy.

Lv refers to leaving groups within a molecule, including Br, Cl, -OH, PhSO₂, -Oalkyl, -Oaryl, or mixed anhydride.

NMR refers to nuclear (proton) magnetic resonance spectroscopy, chemical shifts are reported in ppm (δ) downfield from TMS.

MS refers to mass spectrometry expressed as m/e or mass/charge unit. HRMS refers to high resolution mass spectrometry expressed as m/e or mass/charge unit. M+H⁺ refers to the positive ion of a parent plus a hydrogen atom. M-H⁻ refers to the negative ion of a parent minus a hydrogen atom. M+Na⁺ refers to the positive ion of a parent plus a sodium atom. M+K⁺ refers to the positive ion of a parent plus a potassium atom. EI refers to electron impact. ESI refers to electrospray ionization. CI refers to chemical ionization. FAB refers to fast atom bombardment.

Compounds of the present invention may be in the form of pharmaceutically acceptable salts. The term "pharmaceutically acceptable salts" refers to salts prepared from pharmaceutically acceptable non-toxic bases including inorganic bases and organic bases, and salts prepared from inorganic acids, and organic acids. Salts derived from inorganic bases include aluminum, ammonium, calcium, ferric, ferrous, lithium, magnesium, potassium, sodium, zinc, and the like. Salts derived from pharmaceutically acceptable organic non-toxic bases include salts of primary, secondary, and tertiary amines, substituted amines including naturally occurring substituted amines, cyclic amines, such as arginine, betaine, caffeine, choline, N, N-dibenzylethylenediamine, diethylamine, 2-diethylaminoethanol, 2-dimethylaminoethanol, ethanolamine, ethylenediamine, N-ethylmorpholine, N-ethylpiperidine, glucamine, glucosamine, histidine, hydrabamine, isopropylamine, lysine, methylglucamine, morpholine, piperazine, piperidine, polyamine resins, procaine, purines, theobromine, triethylamine, trimethylamine, tripropylamine, and the like. Salts derived from inorganic acids include salts of hydrochloric acid, hydrobromic acid, hydroiodic acid, sulfuric acid, phosphoric acid, phosphorous acid and the like. Salts derived from pharmaceutically acceptable organic non-toxic acids include salts of C₁₋₆ alkyl carboxylic acids, di-carboxylic acids, and tri-carboxylic acids such as acetic acid, propionic acid, fumaric acid, succinic acid, tartaric acid, maleic acid, adipic acid, and citric acid, and aryl and alkyl sulfonic acids such as toluene sulfonic acids and the like.

By the term "effective amount" of a compound as provided herein is meant a nontoxic but sufficient amount of the compound(s) to provide the desired effect. As pointed out below, the exact amount required will vary from subject to subject, depending on the species, age, and general condition of the subject, the severity of the disease that is being treated, the particular compound(s) used, the mode of administration, and the like. Thus, it is not possible to specify an exact "effective amount." However, an appropriate effective amount may be determined by one of ordinary skill in the art using only routine experimentation.

The amount of therapeutically effective compound(s) that is administered and the dosage regimen for treating a disease condition with the compounds and/or compositions of this invention depends on a variety of factors, including the age, weight, sex and medical condition of the subject, the severity of the disease, the route and frequency of administration, and the particular compound(s) employed, and thus may vary widely. The compositions contain well known carriers and excipients in addition to a therapeutically effective amount of compounds of Formula I. The pharmaceutical compositions may contain active ingredient in the range of about 0.001 to 100 mg/kg/day for an adult, preferably in the range of about 0.1 to 50 mg/kg/day for an adult. A total daily dose of about 1 to 1000 mg of active ingredient may be appropriate for an adult. The daily dose can be administered in one to four doses per day.

In addition to the compound(s) of Formula I, the composition for therapeutic use may also comprise one or more non-toxic, pharmaceutically acceptable carrier materials or excipients. The term "carrier" material or "excipient" herein means any substance, not itself a therapeutic agent, used as a carrier and/or diluent and/or adjuvant, or vehicle for delivery of a therapeutic agent to a subject or added to a pharmaceutical composition to improve its handling or storage properties or to permit or facilitate formation of a dose unit of the composition into a discrete article such as a capsule or tablet suitable for oral administration. Excipients can include, by way of illustration and not limitation, diluents, disintegrants, binding agents, adhesives, wetting agents, polymers, lubricants, glidants, substances added to mask or counteract a disagreeable taste or odor, flavors, dyes, fragrances, and substances added to improve appearance of the composition. Acceptable excipients include lactose, sucrose, starch powder, cellulose esters of alkanoic acids, cellulose alkyl esters, talc, stearic acid, magnesium stearate, magnesium oxide, sodium and calcium salts of phosphoric and sulfuric acids, gelatin, acacia gum, sodium alginate, polyvinylpyrrolidone, and/or polyvinyl alcohol, and then tableted or encapsulated for convenient administration. Such capsules or tablets may contain a controlled-release formulation as may be provided in a dispersion of active compound in hydroxypropylmethyl cellulose, or other methods known to those skilled in the art. For oral administration, the pharmaceutical composition may be in the form of, for example, a tablet, capsule, suspension or liquid. If desired, other active ingredients may be included in the composition.

In addition to the oral dosing, noted above, the compositions of the present invention may be administered by any suitable route, in the form of a pharmaceutical composition adapted to such a route, and in a dose effective for the treatment intended. The compositions may, for example, be administered parenterally, e.g., intravascularly, intraperitoneally, subcutaneously, or intramuscularly. For parenteral administration, saline solution, dextrose solution, or water may be used as a suitable carrier. Formulations for parenteral administration may be in the form of aqueous or non-aqueous isotonic sterile injection solutions or suspensions. These solutions and suspensions may be prepared from sterile powders or granules having one or more of the carriers or diluents mentioned for use in the formulations for oral administration. The compounds may be dissolved in water, polyethylene glycol, propylene glycol, ethanol, corn oil, cottonseed oil, peanut oil, sesame oil, benzyl alcohol, sodium chloride, and/or various buffers. Other adjuvants and modes of administration are well and widely known in the pharmaceutical art.

The serotonin type 3 receptor (5HT₃R) is a member of a superfamily of ligand-gated ion channels, which includes the muscle and neuronal nAChR, the glycine receptor, and the γ-aminobutyric acid type A receptor. Like the other members of this receptor superfamily, the 5HT₃R exhibits a large degree of sequence homology with α7 nAChR but functionally the two ligand-gated ion channels are very different. For example, α7 nAChR is rapidly inactivated, is highly permeable to calcium and is activated by acetylcholine and nicotine. On the other hand, 5HT₃R is inactivated slowly, is relatively impermeable to calcium and is activated by serotonin. These experiments suggest that the α7 nAChR and 5HT₃R proteins have some degree of homology, but function very differently. Indeed the pharmacology of the channels is very different. For example, Ondansetron, a highly selective 5HT₃R antagonist, has little activity at the α7 nAChR. The converse is also true. For example, GTS-21, a highly selective α7 nAChR agonist, has little activity at the 5HT₃R.

α7 nAChR is a ligand-gated Ca⁺⁺ channel formed by a homopentamer of α7 subunits. Previous studies have established that α-bungarotoxin (α-btx) binds selectively to this homopetameric, α7 nAChR subtype, and that α7 nAChR has a high affinity binding site for both α-btx and methyllycaconitine (MLA). α7 nAChR is expressed at high levels in the hippocampus, ventral tegmental area and ascending cholinergic projections from nucleus basalis to thalamocortical areas. α7 nAChR agonists increase neurotransmitter release, and increase cognition, arousal, attention, learning and memory.

Data from human and animal pharmacological studies establish that nicotinic cholinergic neuronal pathways control many important aspects of cognitive function including attention, learning and memory (Levin, E.D., *Psychopharmacology,* 108:417-31, 1992; Levin, E.D. and Simon B.B., *Psychopharmacology,* 138:217-30, 1998). For example, it is well known that nicotine increases cognition and attention in humans. ABT-418, a compound that activates α4β2 and α7 nAChR, improves cognition and attention in clinical trials of Alzheimer's disease and attention-deficit disorders (Potter, A. et. al., *Psychopharmacology (Berl).,* 142(4):334-42, Mar. 1999; Wilens, T. E. et. al., *Am. J. Psychiatry,* 156(12):1931-7, Dec. 1999). It is also clear that nicotine and selective but weak α7 nAChR agonists increase cognition and attention in rodents and non-human primates.

Schizophrenia is a complex multifactorial illness caused by genetic and non-genetic risk factors that produce a constellation of positive and negative symptoms. The positive symptoms include delusions and hallucinations and the negative symptoms include deficits in affect, attention, cognition and information processing. No single biological element has emerged as a dominant pathogenic factor in this disease. Indeed, it is likely that schizophrenia is a syndrome that is produced by the combination of many low penetrance risk factors. Pharmacological studies established that dopamine receptor antagonists are efficacious in treating the overt psychotic features (positive symptoms) of schizophrenia such as hallucinations and delusions. Clozapine, an "atypical" antipsychotic drug, is novel because it is effective in treating both the positive and some of the negative symptoms of this disease. Clozapine's utility as a drug is greatly limited because continued use leads to an increased risk of agranulocytosis and seizure. No other antipsychotic drug is effective in treating the negative symptoms of schizophrenia. This is significant because the restoration of cognitive functioning is the best predictor of a successful clinical and functional outcome of schizophrenic patients (Green, M.F., *Am J Psychiatry,* 153:321-30, 1996). By extension, it is clear that better drugs are needed to treat the cognitive disorders of schizophrenia in order to restore a better state of mental health to patients with this disorder.

One aspect of the cognitive deficit of schizophrenia can be measured by using the auditory event-related potential (P50) test of sensory gating. In this test, electroencepholographic (EEG) recordings of neuronal activity of the hippocampus are used to measure the subject's response to a series of auditory "clicks" (Adler, L.E. et. al., *Biol. Psychiatry,* 46:8-18, 1999). Normal individuals respond to the first click with greater degree than to the second click. In general, schizophrenics and schizotypal patients respond to both clicks nearly the same (Cullum, C.M. et. al., *Schizophr. Res.,* 10:131-41, 1993). These data reflect a schizophrenic's inability to "filter" or ignore unimportant information. The sensory gating deficit appears to be one of the key pathological features of this disease (Cadenhead, K.S. et. al., *Am. J. Psychiatry,* 157:55-9, 2000). Multiple studies show that nicotine normalizes the sensory deficit of schizophrenia (Adler, L.E. et. al., *Am. J. Psychiatry,* 150:1856-61, 1993). Pharmacological studies indicate that nicotine's effect on sensory gating is via the α7 nAChR (Adler, L.E. et. al., *Schizophr. Bull.,* 24:189-202, 1998). Indeed, the biochemical data indicate that schizophrenics have 50% fewer of α7 nAChR receptors in the hippocampus, thus giving a rationale to partial loss of α7 nAChR functionality (Freedman, R et. al., *Biol. Psychiatry,* 38:22-33, 1995). Interestingly, genetic data indicate that a polymorphism in the promoter region of the α7 nAChR gene is strongly associated with the sensory gating deficit in schizophrenia (Freedman, R et. al., *Proc. Nat'l Acad. Sci. USA,* 94(2):587-92, 1997; Myles-Worsley, M. et. al., *Am. J. Med. Genet,* 88(5):544-50, 1999). To date, no mutation in the coding region of the α7 nAChR has been identified. Thus, schizophrenics express the same α7 nAChR as non-schizophrenics.

Selective α7 nAChR agonists may be found using a functional assay on FLIPR (see WO 00/73431 A2). FLIPR is designed to read the fluorescent signal from each well of a 96 or 384 well plate as fast as twice a second for up to 30 minutes. This assay may be used to accurately measure the functional pharmacology of α7 nAChR and 5HT₃R. To conduct such an assay, one uses cell lines that expressed functional forms of the α7 nAChR using the α7/5-HT₃ channel as the drug target and cell lines that expressed functional 5HT₃R. In both cases, the ligand-gated ion channel was expressed in SH-EP1 cells. Both ion channels can produce robust signal in the FLIPR assay.

The compounds of the present invention are α7 nAChR agonists and may be used to treat a wide variety of diseases. For example, they may be used in treating schizophrenia, or psychosis.

Schizophrenia is a disease having multiple aspects. Currently available drugs are generally aimed at controlling the positive aspects of schizophrenia, such as delusions. One drug, Clozapine, is aimed at a broader spectrum of symptoms associated with schizophrenia. This drug has many side effects and is thus not suitable for many patients. Thus, there is a need for a drug to treat the cognitive and attention deficits associated with schizophrenia. Similarly, there is a need for a drug to treat the cognitive and attention deficits associated with schizoaffective disorders, or similar symptoms found in the relatives of schizophrenic patients.

Psychosis is a mental disorder characterized by gross impairment in the patient's perception of reality. The patient may suffer from delusions, and hallucinations, and may be incoherent in speech. His behavior may be agitated and is often incomprehensible to those around him. In the past, the term psychosis has been applied to many conditions that do not meet the stricter definition given above. For example, mood disorders were named as psychoses.

There are a variety of antipsychotic drugs. The conventional antipsychotic drugs include Chlorpromazine, Fluphenazine, Haloperidol, Loxapine, Mesoridazine, Molindone, Perphenazine, Pimozide, Thioridazine, Thiothixene, and Trifluoperazine. These drugs all have an affinity for the dopamine 2 receptor.

These conventional antipsychotic drugs have several side effects, including sedation, weight gain, tremors, elevated prolactin levels, akathisia (motor restlessness), dystonia and muscle stiffness. These drugs may also cause tardive dyskinesia. Unfortunately, only about 70% of patients with schizophrenia respond to conventional antipsychotic drugs. For these patients, atypical antipsychotic drugs are available.

Atypical antipsychotic drugs generally are able to alleviate positive symptoms of psychosis while also improving negative symptoms of the psychosis to a greater degree than conventional antipsychotics. These drugs may improve neurocognitive deficits. Extrapyramidal (motor) side effects are not as likely to occur with the atypical antipsychotic drugs, and thus, these atypical antipsychotic drugs have a lower risk of producing tardive dyskinesia. Finally these atypical antipsychotic drugs cause little or no elevation of prolactin. Unfortunately, these drugs are not free of side effects. Although these drugs each produce different side effects, as a group the side effects include: agranulocytosis; increased risk of seizures, weight gain, somnolence, dizziness, tachycardia, decreased ejaculatory volume, and mild prolongation of QTc interval.

In a combination therapy to treat multiple symptoms of diseases such as schizophrenia, the compounds of Formula I and the anti-psychotic drugs can be administered simultaneously or at separate intervals. When administered simultaneously the compounds of Formula I and the anti-psychotic drugs can be incorporated into a single pharmaceutical composition, e.g., a pharmaceutical combination therapy composition. Alternatively, two separate compositions, i.e., one containing compounds of Formula I and the other containing anti-psychotic drugs, can be administered simultaneously. Examples of anti-psychotic drugs, in addition to those listed above, include, but are not limited to, Thorazine, Mellaril, Trilafon, Navane, Stelazine, Permitil, Prolixin, Risperdal, Zyprexa, Seroquel, ZELDOX, Acetophenazine, Carphenazine, Chlorprothixene, Droperidol, Loxapine, Mesoridazine, Molindone, Ondansetron, Pimozide, Prochlorperazine, and Promazine.

A pharmaceutical combination therapy composition can include therapeutically effective amounts of the compounds of Formula I, noted above, and a therapeutically effective amount of anti-psychotic drugs (also called agents). These compositions may be formulated with common excipients, diluents or carriers, and compressed into tablets, or formulated elixirs or solutions for convenient oral administration or administered by intramuscular intravenous routes. The compounds can be administered rectally, topically, orally, sublingually, or parenterally and maybe formulated as sustained relief dosage forms and the like.

When separately administered, therapeutically effective amounts of compositions containing compounds of Formula I and anti-psychotic drugs are administered on a different schedule. One may be administered before the other as long as the time between the two administrations falls within a therapeutically effective interval. A therapeutically effective interval is a period of time beginning when one of either (a) the compounds of Formula I, or (b) the anti-psychotic drugs is administered to a human and ending at the limit of the beneficial effect in the treatment of schizophrenia or psychosis of the combination of (a) and (b). The methods of administration of the compounds of Formula I and the anti-psychotic drugs may vary. Thus, either agent or both agents may be administered rectally, topically, orally, sublingually, or parenterally.

As discussed, the compounds of the present invention are α7 nAChR agonists. Therefore, as another aspect of the present invention, the compounds of the present invention may be used to treat a variety of diseases including cognitive and attention deficit symptoms of Alzheimer's, neurodegeneration associated with diseases such as Alzheimer's disease, pre-senile dementia (also known as mild cognitive impairment), and senile dementia.

Alzheimer's disease has many aspects, including cognitive and attention deficits. Currently, these deficits are treated with cholinesterase inhibitors. These inhibitors slow the break down of acetylcholine, and thereby provide a general nonspecific increase in the activity of the cholinergic nervous system. Since the drugs are nonspecific, they have a wide variety of side effects. Thus, there is a need for a drug that stimulates a portion of the cholinergic pathways and thereby provides improvement in the cognitive and attention deficits associated with Alzheimer's disease without the side effects created by nonspecific stimulation of the cholinergic pathways.

Neurodegeneration is a common problem associated with diseases such as Alzheimer's disease. While the current drugs treat some of the symptoms of this disease, they do not control the underlying pathology of the disease. Accordingly, it would be desirable to provide a drug that can slow the progress of Alzheimer's disease.

Pre-senile dementia (mild cognitive impairment) concerns memory impairment rather than attention deficit problems and otherwise unimpaired cognitive functioning. Mild cognitive impairment is distinguished from senile dementia in that mild cognitive impairment involves a more persistent and troublesome problem of memory loss for the age of the patient. There currently is no medication specifically identified for treatment of mild cognitive impairment, due somewhat to the newness of identifying the disease. Therefore, there is a need for a drug to treat the memory problems associated with mild cognitive impairment.

Senile dementia is not a single disease state. However, the conditions classified under this name frequently include cognitive and attention deficits. Generally, these deficits are not treated. Accordingly, there is a need for a drug that provides improvement in the cognitive and attention deficits associated with senile dementia.

As discussed, the compounds of the present invention are α7 nAChR agonists. Therefore, yet other diseases to be treated with compounds of the present invention include treating the cognitive and attention deficits as well as the neurodegeneration associated with any one or more or combination of the following: attention deficit disorder, attention deficit hyperactivity disorder, depression, anxiety, general anxiety disorder, post traumatic stress disorder, mood and affective disorders, amyotrophic lateral sclerosis, borderline personality disorder, traumatic brain injury, behavioral and cognitive problems associated with brain tumors, AIDS dementia complex, dementia associated with Down's syndrome, dementia associated with Lewy Bodies, Huntington's disease, Parkinson's disease, tardive dysldnesia, Pick's disease, dysregulation of food intake including bulemia and anorexia nervosa, withdrawal symptoms associated with smoking cessation and dependant drug cessation, Gilles de la Tourette's Syndrome, age-related macular degeneration, glaucoma, neurodegeneration associated with glaucoma, or symptoms associated with pain.

Attention deficit disorder is generally treated with methylphenidate, an amphetamine-like molecule that has some potential for abuse. Accordingly, it would be desirable to provide a drug that treats attention deficit disorder while having fewer side effects than the currently used drug.

Attention deficit hyperactivity disorder, otherwise known as ADHD, is a neurobehavioral disorder affecting 3-5% of all American children. ADHD concerns cognitive alone or both cognitive and behavioral actions by interfering with a person's ability to stay on a task and to exercise age-appropriate inhibition. Several types of ADHD exist: a predominantly inattentive subtype, a predominantly hyperactive-impulsive subtype, and a combined subtype. Treatment may include medications such as methylphenidate, dextroamphetamine, or pemoline, which act to decrease impulsivity and hyperactivity and to increase attention. No "cure" for ADHD currently exists. Children with the disorder seldom outgrow it; therefore, there is a need for appropriate medicaments.

Depression is a mood disorder of varying lengths of normally several months to more than two years and of varying degrees of feelings involving sadness, despair, and discouragement. The heterocyclic antidepressants (HCA's) are currently the largest class of antidepressants, but monoamine oxidase inhibitors (MAOI's) are used in particular types of depression. Common side effects from HCA's are sedation and weight gain. In elderly patients with organic brain disease, the side effects from HCA's can also include seizures and behavioral symptoms. The main side effects from using MAOI's occur from dietary and drug interactions. Therefore, agents with fewer side effects would be useful.

Anxiety disorders (disorders with prominent anxiety or phobic avoidance), represent an area of umet medical needs in the treatment of psychiatric illness. See Diagnostic & Statistical Manual of Mental Disorders, IV (1994), pp 393-394, for various disease forms of anxiety.

General anxiety disorder (GAD) occurs when a person worries about things such as family, health, or work when there is no reason to worry and is unable not to worry. About 3 to 4% of the U.S. population has GAD during the course of a year. GAD most often strikes people in childhood or adolescence, but can begin in adulthood, too. It affects women more often than men. Currently, treatment involves cognitive-behavioral therapy, relaxation techniques, and biofeedback to control muscle tension and medications such as benzodiazepines, imipramine, and buspirone. These drugs are effective but all have side-effect liabilities. Therefore, there is a need of a pharmaceutical agent to address the symptoms with fewer side effects.

Anxiety also includes post-traumatic stress disorder (PTSD), which is a form of anxiety triggered by memories of a traumatic event that directly affected the patient or that the patient may have witnessed. The disorder commonly affects survivors of traumatic events including sexual assault, physical assault, war, torture, natural disasters, an automobile accident, an airplane crash, a hostage situation, or a death camp. The affliction also can affect rescue workers at an airplane crash or a mass shooting, someone who witnessed a tragic accident or someone who has unexpectedly lost a loved one. Treatment for PTSD includes cognitive-behavioral therapy, group psychotherapy, and medications such as Clonazepam, Lorazepam and selective serotonin-reuptake inhibitors such as Fluoxetine, Sertraline, Paroxetine, Citalopram and Fluvoxamine. These medications help control anxiety as well as depression. Various forms of exposure therapy (such as systemic desensitization and imaginal flooding) have all been used with PTSD patients. Exposure treatment for PTSD. involves repeated reliving of the trauma, under controlled conditions, with the aim of facilitating the processing of the trauma. Therefore, there is a need for better pharmaceutical agents to treat post traumatic stress disorder.

Mood and affective disorders fall within a large group of diseases, including monopolar depression and bi-polar mood disorder. These diseases are treated with three major classes of compounds. The first group is the heterocyclic antidepressant (HCA's). This group includes the well-known tricyclic antidepressants. The second group of compounds used to treat mood disorders is the monoamine oxidase inhibitors (MAOI's) that are used in particular types of diseases. The third drug is lithium. Common side effects from HCA's are sedation and weight gain. In elderly patients with organic brain disease, the side effects of HCA's can also include seizures and behavioral symptoms. The main side effects from using MAOI's occur from dietary and drug interactions. Benign side effects from the use of lithium include, but are not limited to, weight gain, nausea, diarrhea, polyuria, polydipsia, and tremor. Toxic side effects from lithium can include persistent headache, mental confusion, and may reach seizures and cardiac arrhythmias. Therefore, agents with less side effects or interactions with food or other medications would be useful.

Borderline personality disorder, although not as well known as bipolar disorder, is more common. People having borderline personality disorder suffer from a disorder of emotion regulation. Pharmaceutical agents are used to treat specific symptoms, such as depression or thinking distortions.

Acquired immune deficiency syndrome (AIDS) results from an infection with the human immunodeficiency virus (HIV). This virus attacks selected cells and impairs the proper function of the immune, nervous, and other systems. HIV infection can cause other problems such as, but not limited to, difficulties in thinking, otherwise known as AIDS dementia complex. Therefore, there is a need to drugs to relieve the confusion and mental decline of persons with AIDS.

Amyotrophic lateral sclerosis, also known as Lou Gehrig's disease, belongs to a class of disorders known as motor neuron diseases wherein specific nerve cells in the brain and spinal cord gradually degenerate to negatively affect the control of voluntary movement. Currently, there is no cure for amyotrophic lateral sclerosis although patients may receive treatment from some of their symptoms and although Riluzole has been shown to prolong the survival of patients. Therefore, there is a need for a pharmaceutical agent to treat this disease.

Traumatic brain injury occurs when the brain is damaged from a sudden physical assault on the head. Symptoms of the traumatic brain injury include confusion and other cognitive problems. Therefore, there is a need to address the symptoms of confusion and other cognitive problems.

Brain tumors are abnormal growths of tissue found inside of the skull. Symptoms of brain tumors include behavioral and cognitive problems. Surgery, radiation, and chemotherapy are used to treat the tumor, but other agents are necessary to address associated symptoms. Therefore, there is a need to address the symptoms of behavioral and cognitive problems.

Persons with Down's syndrome have in all or at least some of their cells an extra, critical portion of the number 21 chromosome. Adults who have Down's syndrome are known to be at risk for Alzheimer-type dementia. Currently, there is no proven treatment for Down's syndrome. Therefore, there is a need to address the dementia associated with Down's syndrome.

Genetically programmed degeneration of neurons in certain areas of the brain cause Huntington's disease. Early symptoms of Huntington's disease include mood swings, or trouble learning new things or remembering a fact. Most drugs used to treat the symptoms of Huntington's disease have side effects such as fatigue, restlessness, or hyperexcitability. Currently, there is no treatment to stop or reverse the progression of Huntington's disease. Therefore, there is a need of a pharmaceutical agent to address the symptoms with fewer side effects.

Dementia with Lewy Bodies is a neurodegenerative disorder involving abnormal structures known as Lewy bodies found in certain areas of the brain. Symptoms of dementia with Lewy bodies include, but are not limited to, fluctuating cognitive impairment with episodic delirium. Currently, treatment concerns addressing the parkinsonian and psychiatric symptoms. However, medicine to control tremors or loss of muscle movement may actually accentuate the underlying disease of dementia with Lewy bodies. Therefore, there is a need of a pharmaceutical agent to treat dementia with Lewy bodies.

Parkinson's disease is a neurological disorder characterized by tremor, hypokinesia, and muscular rigidity. Currently, there is no treatment to stop the progression of the disease. Therefore, there is a need of a pharmaceutical agent to address Parkinson's.

Tardive dyskinesia is associated with the use of conventional antipsychotic drugs. This disease is characterized by involuntary movements most often manifested by puckering of the lips and tongue and/or writhing of the arms or legs. The incidence of tardive dyskinesia is about 5% per year of drug exposure among patients taking conventional antipsychotic drugs. In about 2% of persons with the disease, tardive dyskinesia is severely disfiguring. Currently, there is no generalized treatment for tardive dyskinesia. Furthermore, the removal of the effect-causing drugs is not always an option due to underlying problems. Therefore, there is a need for a pharmaceutical agent to address the symptoms of tardive dyskinesia.

Pick's disease results from a slowly progressive deterioration of social skills and changes in personality with the resulting symptoms being impairment of intellect, memory, and language. Common symptoms include memory loss, lack of spontaneity, difficulty in thinking or concentrating, and speech disturbances. Currently, there is no specific treatment or cure for Pick's disease but some symptoms can be treated with cholinergic and serotonin-boosting antidepressants. In addition, antipsychotic medications may alleviate symptoms in FTD patients who are experiencing delusions or hallucinations. Therefore, there is a need for a pharmaceutical agent to treat the progressive deterioration of social skills and changes in personality and to address the symptoms with fewer side effects.

Dysregulation of food intake associated with eating disease, including bulemia nervosa and anorexia nervosa, involve neurophysiological pathways. Anorexia nervosa is hard to treat due to patients not entering or remaining in after entering programs. Currently, there is no effective treatment for persons suffering from severe anorexia nervosa. Cognitive behavioral therapy has helped patients suffering from bulemia nervosa; however, the response rate is only about 50% and current treatment does not adequately address emotional regulation. Therefore, there is a need for pharmaceutical agents to address neurophysiological problems underlying diseases of dysregulation of food intake.

Cigarette smoking has been recognized as a major public health problem for a long time. However, in spite of the public awareness of health hazard, the smoking habit remains extraordinarily persistent and difficult to break. There are many treatment methods available, and yet people continue to smoke. Administration of nicotine transdermally, or in a chewing gum base is common treatments. However, nicotine has a large number of actions in the body, and thus can have many side effects. It is clear that there is both a need and a demand of long standing for a convenient and relatively easy method for aiding smokers in reducing or eliminating cigarette consumption. A drug that could selectively stimulate only certain of the nicotinic receptors would be useful in smoke cessation programs.

Smoke cessation programs may involve oral dosing of the drug of choice. The drug may be in the form of tablets. However, it is preferred to administer the daily dose over the waking hours, by administration of a series of incremental doses during the day. The preferred method of such administration is a slowly dissolving lozenge, troche, or chewing gum, in which the drug is dispersed. Another drug in treating nicotine addiction is Zyban. This is not a nicotine replacement, as are the gum and patch. Rather, this works on other areas of the brain, and its effectiveness is to help control nicotine craving or thoughts about cigarette use in people trying to quit. Zyban is not very effective and effective drugs are needed to assist smokers in their desire to stop smoking. These drugs may be administered transdermally through the use of skin patches. In certain cases, the drugs may be administered by subcutaneous injection, especially if sustained release formulations are used.

Drug use and dependence is a complex phenomenon, which cannot be encapsulated within a single definition. Different drugs have different effects, and therefore different types of dependence. Drug dependence has two basic causes, that is, tolerance and physical dependence. Tolerance exists when the user must take progressively larger doses to produce the effect originally achieved with smaller doses. Physical dependence exists when the user has developed a state of physiologic adaptation to a drug, and there is a withdrawal (abstinence) syndrome when the drug is no longer taken. A withdrawal syndrome can occur either when the drug is discontinued or when an antagonist displaces the drug from its binding site on cell receptors, thereby counteracting its effect. Drug dependence does not always require physical dependence.

In addition drug dependence often involves psychological dependence, that is, a feeling of pleasure or satisfaction when taking the drug. These feelings lead the user to repeat the drug experience or to avoid the displeasure of being deprived of the drug. Drugs that produce strong physical dependence, such as nicotine, heroin and alcohol are often abused, and the pattern of dependence is difficult to break. Drugs that produce dependence act on the CNS and generally reduce anxiety and tension; produce elation, euphoria, or other pleasurable mood changes; provide the user feelings of increased mental and physical ability; or alter sensory perception in some pleasurable manner. Among the drugs that are commonly abused are ethyl alcohol, opioids, anxiolytics, hypnotics, cannabis (marijuana), cocaine, amphetamines, and hallucinogens. The current treatment for drug-addicted people often involves a combination of behavioral therapies and medications. Medications, such as methadone or LAAM (levo-alpha-acetyl-methadol), are effective in suppressing the withdrawal symptoms and drug craving associated with narcotic addiction, thus reducing illicit drug use and improving the chances of the individual remaining in treatment. The primary medically assisted withdrawal method for narcotic addiction is to switch the patient to a comparable drug that produces milder withdrawal symptoms, and then gradually taper off the substitute medication. The medication used most often is methadone, taken orally once a day. Patients are started on the lowest dose that prevents the more severe signs of withdrawal and then the dose is gradually reduced. Substitutes can be used also for withdrawal from sedatives. Patients can be switched to long-acting sedatives, such as diazepam or phenobarbital, which are then gradually reduced.

Gilles de la Tourette's Syndrome is an inherited neurological disorder. The disorder is characterized by uncontrollable vocal sounds called tics and involuntary movements. The symptoms generally manifest in an individual before the person is 18 years of age. The movement disorder may begin with simple tics that progress to multiple complex tics, including respiratory and vocal ones. Vocal tics may begin as grunting or barking noises and evolve into compulsive utterances. Coprolalia (involuntary scatologic utterances) occurs in 50% of patients. Severe tics and coprolalia may be physically and socially disabling. Tics tend to be more complex than myoclonus, but less flowing than choreic movements, from which they must be differentiated. The patient may voluntarily suppress them for seconds or minutes.

Currently simple tics are often treated with benzodiazepines. For simple and complex tics, Clonidine may be used. Long-term use of Clonidine does not cause tardive dyskinesia; its limiting adverse effect is hypotension. In more severe cases, antipsychotics, such as Haloperidol may be required, but side effects of dysphoria, parkinsonism, akathisia, and tardive dyskinesia may limit use of such antipsychotics. There is a need for safe and effective methods for treating this syndrome.

Age-related macular degeneration (AMD) is a common eye disease of the macula which is a tiny area in the retina that helps produce sharp, central vision required for "straight ahead" activities that include reading and driving. Persons with AMD lose their clear, central vision. AMD takes two forms: wet and dry. In dry AMD, there is a slow breakdown of light-sensing cells in the macula. There currently is no cure for dry AMD. In wet AMD, new, fragile blood vessels growing beneath the macula as dry AMD worsens and these vessels often leak blood and fluid to cause rapid damage to the macula quickly leading to the loss of central vision. Laser surgery can treat some cases of wet AMD. Therefore, there is a need of a pharmaceutical agent to address AMD.

Glaucoma is within a group of diseases occurs from an increase in intraocular pressure causing pathological changes in the optical disk and negatively affects the field of vision. Medicaments to treat glaucoma either decrease the amount of fluid entering the eye or increase drainage of fluids from the eye in order to decrease intraocular pressure. However, current drugs have drawbacks such as not working over time or causing side effects so the eye-care professional has to either prescribe other drugs or modify the prescription of the drug being used. There is a need for safe and effective methods for treating problems manifesting into glaucoma.

Ischemic periods in glaucoma cause release of excitotoxic amino acids and stimulate inducible form of nitric oxide synthase (iNOS) leading to neurodegeneration. Alpha 7 nicotinic agonists may stimulate the release of inhibitory amino acids such as GABA which will dampen hyperexcitablity. Alpha 7 nicotinic agonists are also directly neuroprotective on neuronal cell bodies. Thus alpha 7 nicotinic agonists have the potential to be neuroprotective in glaucoma.

Persons afflicted with pain often have what is referred to as the "terrible triad" of suffering from the pain, resulting in sleeplessness and sadness, all of which are hard on the afflicted individual and that individual's family. Pain can manifest itself in various forms, including, but not limited to, headaches of all severity, back pain, neurogenic, and pain from other ailments such as arthritis and cancer from its existence or from therapy to irradicate it. Pain can be either chronic (persistent pain for months or years) or acute (short-lived, immediate pain to inform the person of possible injury and need of treatment). Persons suffering from pain respond differently to individual therapies with varying degrees of success. There is a need for safe and effective methods for treating pain.

Finally, the compounds of the present invention may be used in combination therapy with typical and atypical anti-psychotic drugs. All compounds within the present invention are useful for and may also be used in combination with each other to prepare pharmaceutical compositions. Such combination therapy lowers the effective dose of the anti-psychotic drug and thereby reduces the side effects of the anti-psychotic drugs. Some typical anti-psychotic drugs that may be used in the practice of the invention include Haldol. Some atypical anti-psychotic drugs include Ziprasidone, Olanzapine, Resperidone, and Quetiapine.

Compounds of Formula I can be prepared as shown in Scheme 1. Starting materials can be prepared by procedures described below or by procedures that would be well known to one of ordinary skill in organic chemistry. The variables used in Scheme 1 are defined below or as in the claims. The key step in the preparation of this class of compounds is the coupling of *tert*-butyl (1*S,* 2*R,* 4*R*)-(+)-2-amino-7-azabicyclo[2.2.1]heptane-7-carboxylate ((2R)-7-aza-[2.2.1]-Amine) with the requisite acid chloride (Lv = Cl), mixed anhydride (e.g., Lv = diphenyl phosphoryl, bis(2-oxo-3-oxazolidinyl)phosphinyl, or acyloxy of the general formula of O-C(O)-R_{Lv}, where R_{Lv} includes phenyl or t-butyl), ester (e.g., Lv = alkyl, aryl, or electron deficient aryl), or carboxylic acid (Lv =OH) in the presence of an activating agent. Suitable activating reagents are well known in the art, for examples see Kiso, Y., Yajima, H. "Peptides" pp. 39-91, San Diego, CA, Academic Press, (1995), and include, but are not limited to, agents such as carbodiimides, phosphonium and uronium salts (such as uronium salt HATU).

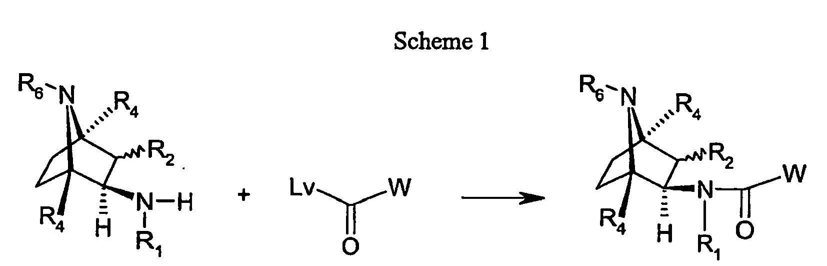

Preferably, (2R)-7-aza-[2.2.1]-Amine can be coupled to the acid in the presence of an appropriate base, such as DIEA, and a uronium salt, such as HATU, in an aprotic medium, such as DMF, to give the desired amides. Alternatively, the acid is converted into a mixed anhydride by treatment with bis (2-oxo-3-oxazolidinyl) phosphinic chloride in the presence of TEA with CH₂Cl₂ or CHCl₃ as the solvent. The resulting anhydride solution is directly reacted with (2R)-7-aza-[2.2.1]-Amine added neat or using CH₂Cl₂ or CHCl₃ as solvent. Furthermore, condensation of the amine with an ester (W-C(O)-O-alkyl or W-C(O)-O-(electron-deficient aryl)) in an alcoholic solvent such as ethanol at an elevated temperature will yield desired amides.

Treatment of the carboxamide with a sulfurating agent such as Lawesson's Reagent (2,4-bis(4-methoxyphenyl)-1,3-dithia-2,4-diphosphetane-2,4-disulfide) in, for instance, dioxane at an appropriate temperature provides the corresponding thioamide, e.g., X in formula I is S. See Lawesson et. al. in *Bull. Soc. Chim. Belg.,* 229 (1978)), or P₄S₁₀ (see *Chem. Rev.,* 45 (1961). Alternatively, one can react a dithiocarboxylic ester with the corresponding azbicyclo moiety to form the same thioamide.

There are various methods for the construction of the optionally substituted 7-azabicyclo[2.2.1]heptane ring system. For example, the independent work of Trudell (R₄ = H, Zhang, C., Trudell, M.L., *J. Org. Chem., 61,* 7189-7191, **1996**), and Schultz (R₄ = Me, Schultz, A.G., Shen, M.S., *Tetrahedron Lett.,* 22, 3347-3350,**1981**) describes the utility of a Diels-Alder approach toward preparing this ring system with functionality suitable for further elaboration to the desired 2-amino-7-azabicyclo[2.2.1]heptane (Scheme 2). For instance, Trudell reports (Zhang, C., Trudell, M.L., *Tetrahedron,* 54, 8349-8354, **1998**) that Diels-Alder adduct **1a** (where R₆ = methylcarbamate, R₄ = H, and Lv = Br) could readily be functionalized at C-3 via reaction with organocopper species to introduce the substituent R₂ in **2a,b.** Likewise, hydrogenolysis of adduct **1a,b** or **2a,b** followed by isomerization of the *endo* products as described by Singh (Singh, S., Basmadjian, G.P., *Tetrahedron Lett., 38,* 6829-6830, **1997)** could provide access to the required *exo* acid **3a-d.** Treatment of **3** with diphenylphosphoryl azide in the presence of a tertiary amine base (e.g., Et₃N) in a suitable solvent such as toluene, followed by warming of the intermediate acylazide in the presence of a suitable alcohol (e.g., benzyl alcohol) would effect the well-known Curtius rearrangement to provide a differentially protected *bis* carbamate which could be cleaved under typical hydrogenolysis conditions (e.g., 10% Pd/C, EtOH, H₂, ambient to 50 psi) to give the desired amine **4.** Alternatively, the differentially protected *bis* carbamate might provide an attractive point of intervention for the chromatographic resolution of the individual 2-*exo* isomers prior to cleavage to amine **4.**

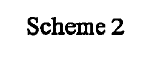

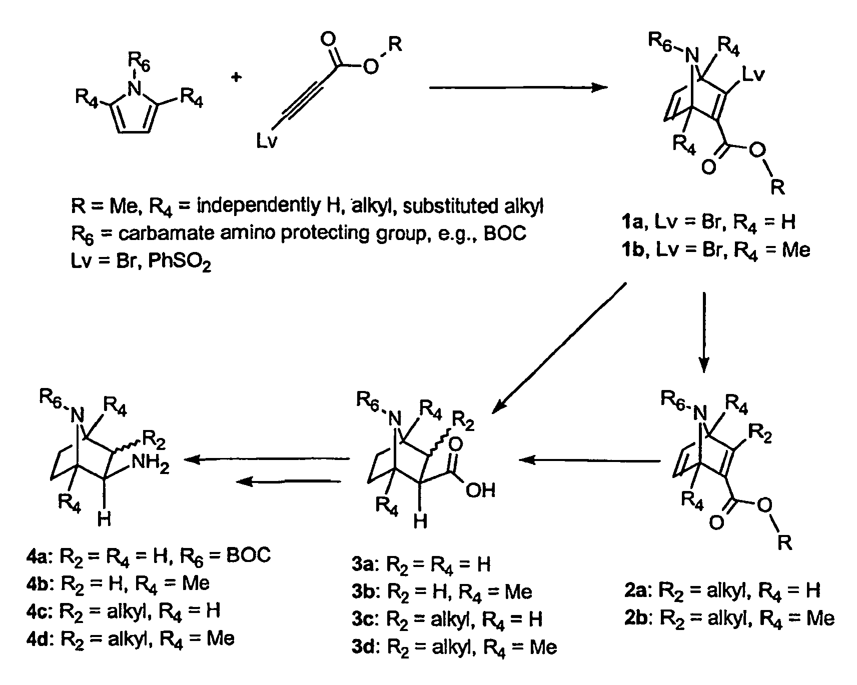

In the case where R₆ = *tert*-butyloxycarbonyl, deprotection of the 7-aza group can be conveniently accomplished under acidic conditions in a suitable solvent such as methanol. After deprotection, the secondary amine may be functionalized with alkyl and substituted alkyl via reductive amination or alkylative procedures.

It will be apparent to those skilled in the art that the requisite carboxylic acids can be obtained through synthesis via literature procedures or through the slight modification thereof.

### Preparation of tert-butyl (1S, 2R, 4R)-2-amino-7-azabicyclo[2.2.1]heptane-7-carboxylate:

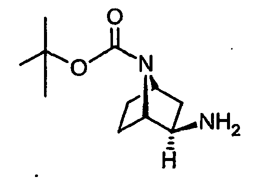

Methyl propiolate (52 ml, 0.583 mol) is combined with recrystallized N-bromo-succinimide (120 g, 0.674 mol) in 1,700 ml acetone under nitrogen. The solution is treated with silver nitrate (9.9 g, 0.0583 mol) neat in a single lot and the reaction is stirred 6 h at RT. The acetone is removed under reduced pressure (25°C, bath temperature) to provide a gray slurry. The slurry is washed with 2 x 200 ml hexane, the gray solid is removed by filtration, and the filtrate is concentrated *in vacuo* to provide 95 g of a pale yellow oily residue. The crude material is distilled via short path under reduced pressure (65°C, about 25 mm Hg) into a dry ice/acetone cooled receiver to give 83.7 g (88%) of methyl-3-bromo-propiolate as a pale yellow oil. Anal. calc'd for C₄H₃BrO₂: C, 29.48; H, 1.86. Found: C, 29.09; H, 1.97.

Methyl-3-bromo-propiolate (83.7 g, 0.513 mol) is added to *N-t*-butyloxy-pyrrole (430 ml, 2.57 mol) under nitrogen. The dark mixture is warmed in a 90 °C bath for 30 h, is cooled, and the bulk of the excess *N-t*-butyloxy-pyrrole is removed in *vacuo* using a dry ice/acetone condenser. The dark oily residue is chromatographed over 1 kg silica gel (230-400 mesh) eluting with 0-15% EtOAc/hexane. The appropriate fractions are combined and concentrated to afford 97 g (57%) of 7-*tert-*butyl 2-methyl 3-bromo-7-azabicyclo[2.2.1]hepta-2,5-diene-2,7-dicarboxylate as a dark yellow oil. HRMS (FAB) calc'd for C₁₃H₁₆BrNO₄+H: 330.0341, found 330.0335 (M+H)⁺.

7-*tert*-Butyl 2-methyl 3-bromo-7-azabicyclo[2.2.1]hepta-2,5-diene-2,7-dicarboxylate (97 g, 0.294 mol) is added to10% Pd/C (6.8g) in 900 ml absolute EtOH in a PARR bottle. The suspension is diluted with a solution of NaHCO₃ (25 g, 0.301 mol) in 250 ml water and the mixture is hydrogenated at 50 PSI for 2.5 h. The catalyst is removed by filtration, is washed with fresh EtOH, and the filtrate is concentrated *in vacuo* to give a residue. The residue is partitioned between 1 x 200 ml saturated NaHCO₃ and CH₂Cl₂ (4 x 100 ml). The combined organic layer is dried over 1:1 K₂CO₃/MgSO₄ and concentrated *in vacuo* to afford 72.8 g (98%) of (+/-) *endo*-7-*tert*-butyl 2-methyl 7-azabicyclo[2.2.1]heptane-2,7-dicarboxylate. MS (EI) for C₁₄H₂₂O₄, *m*/*z*: 255 (M)⁺.

(+/-)*Endo*-7-*tert*-butyl 2-methyl 7-azabicyclo[2.2.1]heptane-2,7-dicarboxylate (72.8 g, 0.285 mol) is dissolved in 1000 ml dry MeOH in a dried flask under nitrogen. The solution is treated with solid NaOMe (38.5 g, 0.713 mol) neat, in a single lot and the reaction is warmed to reflux for 4h. The mixture is cooled to 0°C, is treated with 400 ml water, and the reaction is stirred 1h as it warms to RT. The mixture is concentrated *in vacuo* to about 400 ml and the pH of the aqueous residue is adjusted to 4.5 with 12N HCl. The precipitate is collected and dried. The tan, slightly tacky solid is washed with 2 x 100 ml 60% ether in hexane and is dried to provide 47 g (68%) of (+/-) *exo*-7-(tert-butoxycarbonyl)-7-azabicyclo[2.2.1]heptane-2-carboxylic acid as an off-white powder. HRMS (FAB) calc'd for C₁₂H₁₉NO₄+H: 242.1392, found 242.1390 (M+H)⁺.

(+/-)*Exo*-7-(*tert*-butoxycarbonyl)-7-azabicyclo[2.2.1]heptane-2-carboxylic acid (103.9 g, 0.430 mol) is combined with TEA (60 ml, 0.430 mol) in 1200 ml dry toluene in a dry flask under nitrogen. The solution is treated drop-wise with diphenylphosphoryl azide (92.8 ml, 0.430 mol), and is allowed to stir for 20 min at RT. The mixture is treated with benzyl alcohol (47.9 ml, 0.463 mol), and the reaction is stirred overnight at 55°C. The mixture is cooled, is extracted successively with 2 x 500 ml 5% citric acid, 2 x 500 ml water, 2 x 500 ml saturated sodium bicarbonate, and 500 ml brine. The organic layer is dried over MgSO₄ and concentrated *in vacuo* to an amber oil. The crude material is chromatographed over 900 g silica gel (230-400 mesh), eluting with 10-30% EtOAc/hexane. The appropriate fractions are combined and concentrated to give 106 g (71%) of (+/-) *exo-tert-*butyl 2-{[(benzyloxy)carbonyl]amino}-7-azabicyclo[2.2.1]heptane-7-carboxylate as a pale oil. ¹H NMR (CDCl₃) δ 1.29-1.60, 1.44, 1.62-2.01, 3.76-3.88, 4.10, 4.24, 5.10, 7.36 ppm.

(+/-) *Exo*-tert-Butyl 2-{[(benzyloxy)carbonyl]amino}-7-azabicyclo[2.2.1]heptane-7-carboxylate (1.5 g, 4.33 mmol) is combined with 10% Pd/C (150 mg) in 40 ml EtOH in a 250 ml Parr shaker bottle. The mixture is hydrogenated at 50 PSI for 1.5 h. The catalyst is removed by filtration and the filtrate is concentrated *in vacuo.* The crude material is chromatographed over 30 g silica gel (230-400 mesh), eluting with 7% MeOH/CH₂Cl₂ + 1% conc. NH₄OH. The appropriate fractions are combined and concentrated to provide 606 mg (66%) of (+/-) *exo*-tert-butyl 2-amino-7-azabicyclo[2.2.1]heptane-7-carboxylate. HRMS (FAB) calcd for C₁₁H₂₀N₂O₂+H: 213.1603, found 213.1580 (M+H)⁺. This racemic mixture will be referenced as (+/-)-7-aza-[2.2.1]-Amine.

Resolution of racemic carboxylate mixture:

The isolated (+/-) *exo-tert-*butyl 2-{[(benzyloxy)carbonyl]amino}-7-azabicyclo[2.2.1]heptane-7-carboxylate is resolved via preparative chiral HPLC (50x500 mm Chiralcel OJ column, 30 deg. C, 70 mL/min. 10/90 (v/v) isopropanol/heptane). The resolution affords 40 g of *tert*-butyl (1*S,* 2*R,* 4*R*)-(+)-2{[(benzyloxy)carbonyl]amino}-7-azabicyclo[2.2.1]heptane-7-carboxylate and 42 g of *tert*-butyl-(1*R,* 2*S,* 4*S*)(-)-2{[(benzyloxy)carbonyl]amino}-7-azabicyclo[2.2.1]heptane-7-carboxylate.

The 2*R* enantiomer is triturated with 40 ml ether followed by 40 ml hexane (to remove lingering diastereo and enantiomeric impurities) and is dried to afford 30 g (56%) of purified *tert*-butyl (1*S,* 2*R,* 4*R*)-(+)-2{[(benzyloxy)carbonyl]amino}-7-azabicyclo[2.2.1]heptane-7-carboxylate with 99% enantiomeric excess. MS (EI) for C₁₉H₂₆N₂O₄, *m*/*z*: 346 (M)⁺. [α]²⁵_{D} = 22, (*c* 0.42, chloroform).

The 2*S* enantiomer is triturated with 40 ml ether followed by 40 ml hexane to give 35 g (66%) of purified *tert*-butyl (1*R,* 2*S,* 4*S*)-(-)-2{[(benzyloxy)carbonyl]amino}-7-azabicyclo[2.2.1]heptane-7-carboxylate with 99% enantiomeric excess. MS (EI) for C₁₉H₂₆N₂O₄, *m*/*z*: 346 (M)⁺. [α]²⁵D =-23, (c 0.39, chloroform).

*tert*-Butyl (1*S*, 2*R*, 4*R*)-(+)-2{[(benzyloxy)carbonyl]amino}-7-azabicyclo[2.2.1]heptane-7-carboxylate (9.5 g, 27.4 mmol) is combined with 950 mg 10% Pd/C in 75 ml absolute EtOH in a 500 ml Parr bottle. The reaction mixture is hydrogenated at 50 PSI for 3h, the catalyst is removed by filtration, and the filter cake is washed with MeOH. The filtrate is concentrated *in vacuo* to give 6.4 g of a residue. The crude material is chromatographed over 200 g silica gel (230-400 mesh) eluting with 7% CH₃OH/CHCl₃ containing 1% conc. NH₄OH. The appropriate fractions are combined and concentrated to give 5.61 g (96%) of *tert*-butyl-(1*S*, 2*R*, 4*R*)-(+)-2-amino-7-azabicyclo[2.2.1]heptane-7-carboxylate as a pale oil. MS (EI) for C₁₁H₂₀N₂O₂, *m*/*z*: 212 (M)⁺. [α]²⁵D = 9, (*c* 0.67, CHCl₃). This will be referenced as (2*R*)-7-aza-[2.2.1]-Amine.

The following examples are provided as examples and are not intended to limit the scope of this invention to only those provided examples and named compounds. Also, the salts made in the examples are only exemplary and are not intended to limit the invention. Any pharmaceutically acceptable salt can be made by one of ordinary skill in the art. The invention includes the following examples in pure stereoisomeric form or as racemic mixtures.

### Example 1: N-[(1S, 2R, 4R)-7-azabicyclo[2.2.1]hept-2-yl]-3-methylfuro[2,3-c]pyridine-5-carboxamide dihydrochloride:

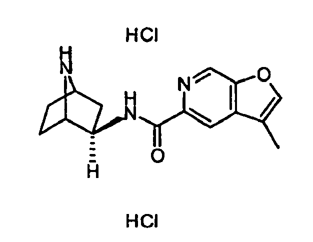

2-Chloro-6-(hydroxymethyl)-4-iodo-3-pyridinol (7.14 g, 25.0 mmol) is dissolved in DMF (50 mL) in a dried flask under nitrogen, is treated with sodium hydride (60% dispersion in mineral oil) (1.0 g, 25.0 mmol), and is stirred for 1 h at rt. Allyl bromide (2.38 ml, 27.5 mmol) is added to the reaction mixture and the resulting mixture is stirred 48 h at rt. The mixture is diluted with EtOAc (50 mL) and washed with a 50% saturated solution of 1:1 NaCl/ NaHCO₃ (4 X 25 mL). The organic layer is dried over MgSO₄ and is concentrated *in vacuo* to a white solid. The solid is washed with hexane and dried to afford 5.51 g (68%) of 3-(allyloxy)-2-chloro-6-(hydroxymethyl)-4-iodopyridine as a white solid. MS for C₉H₉ClINO₂, (EI) *m*/*z*: 325 (M)⁺.

3-(Allyloxy)-2-chloro-6-(hydroxymethyl)-4-iodopyridine (5.51 g, 16.9 mmol) is suspended in benzene (30 mL) in a dry flask under nitrogen. Azo(bis)isobutyryl nitrile (289 mg, 1.8 mmol) is added, the mixture rapidly heated to reflux, and tributyltin hydride (4.91 mL, 18.2 mmol) in benzene (10 mL) is added. The solution is refluxed for 1.5 h, cooled to rt and concentrated *in vacuo* to a residue. The resulting residue is chromatographed over 125 g slurry-packed silica gel, eluting with a gradient of EtOAc/hexane (20% - 60%). The appropriate fractions are combined and concentrated to a colorless oil that solidified upon standing to afford 3.0 g (89%) of (7-chloro-3-methyl-2,3-dihydrofuro[2,3-c]pyridin-5-yl)methanol as a white solid. MS for C₉H₁₀ClNO₂, (ESI): 200.1(MH)⁺.

(7-Chloro-3-methyl-2,3-dihydrofuro[2,3-c]pyridin-5-yl)methanol (3.00 g, 15.0 mmol) is combined with 20% Pd(OH)₂/C (800 mg) and 2N NaOH (9.2 mL, 18.2 mmol) in a PARR shaker bottle. The mixture is hydrogenated at 20 psi for 3 h, is filtered through celite and concentrated *in vacuo.* The resulting residue is partitioned between water (50 mL) and CH₂Cl₂ (4 X 30 mL). The combined organic layer is dried over MgSO₄ and concentrated to a colorless oil which solidified upon standing to afford 2.5 g of (3-methyl-2,3-dihydrofuro[2,3-c]pyridin-5-yl)methanol as a white crystalline solid. MS for C₉H₁₁NO₂, (EI) *m*/*z*: 165 (M)⁺.

(3-Methyl-2,3-dihydrofuro[2,3-c]pyridin-5-yl)methanol (2.48 g, 15.03 mmol) is dissolved in pyridine (15 mL), treated with Ac₂O (4.18 mL, 45.09 mmol) and is stirred for 16 h at rt under nitrogen. The mixture is concentrated *in vacuo,* the residue diluted with EtOAc (75 mL), washed with 50% saturated NaHCO₃ (4 X 30 mL) and dried over MgSO₄. The organic layer is concentrated *in vacuo* to afford 2.85 g (92%) of (3-methyl-2,3-dihydrofuro[2,3-c]pyridin-5-yl)methyl acetate as a colorless oil. MS for C₁₁H₁₃NO₃ (EI) *m*/*z:* 207 (M)⁺.

(3-Methyl-2,3-dihydrofuro[2,3-c]pyridin-5-yl)methyl acetate (2.85 g, 13.8 mmol) is dissolved in dioxane (100 mL), is treated with 2,3,5,6-tertachlorobenzoquinone (3.72 g, 15.1 mmol) and is heated to reflux for 17 h. The mixture is concentrated *in vacuo,* the resulting brown solid washed with 1:1 EtOAc/Et₂O (50 mL), and the insoluble material is filtered off. The filtrate is concentrated to a brown solid, dissolved in MeOH (50 mL), treated with 2 N NaOH (16 mL, 32 mmol), and stirred at rt for 1 h. The mixture is concentrated to dryness, dissolved in 1 N NaOH (75 mL), extracted with CH₂Cl₂ (4 X 50 mL), dried over K₂CO₃ and concentrated to a white solid (2.0 g). The crude material is adsorbed onto silica gel (4 g) and chromatographed over a standard 40 g Biotage column, eluting with 90% EtOAc/ hexane. The appropriate fractions are collected and concentrated to afford 1.88 g (84%) of (3-methylfuro[2,3-c]pyridin-5-yl)methanol as a white solid. MS C₉H₉NO₂ (El) *m*/*z*: 163 (M)⁺.

Dimethylsulfoxide (18.8 mL, 26.5 mmol) is slowly added to a solution of oxalyl chloride (1.16 mL, 13.2 mmol) in CH₂Cl₂ (30 mL) in a dried flask, under nitrogen, in a dry ice/acetone bath. The solution is stirred for 20 min, then treated with (3-methylfuro[2,3-c]pyridin-5-yl)methanol (1.88 g, 11.5 mmol). The mixture is stirred for 1 h in a dry ice /acetone bath, then 30 min in an ice bath. The material is washed with saturated NaHCO₃ (75 mL), dried over K₂CO₃ and concentrated *in vacuo* to a yellow solid (3.23 g). The crude material is adsorbed onto silica gel (6 g) and chromatographed over a standard 40 g Biotage column, eluting with 25% EtOAc/hexane. The appropriate fractions are concentrated to afford 1.33 g (72%) of 3-methylfuro[2,3-c]pyridine-5-carbaldehyde as a white solid. MS for C₉H₇NO₂, (EI) *m*/*z*: 161 (M)⁺.

3-Methylfuro[2,3-c]pyridine-5-carbaldehyde (1.33 g, 8.28 mmol) is dissolved in THF (50 mL), *tert*-butylalcohol (25 mL) and water (25 mL), under nitrogen, and single portions of sodium chlorite (2.81 g, 24.84 mmol) and potassium dihydrogen phosphate (2.25 g, 16.56 mmol) are added. The reaction mixture is stirred overnight at rt, concentrated to dryness, dissolved in 50% saturated brine (60 mL) and extracted with ether (3 X). TLC of extracts indicated acid as well as residual aldehyde, so organics and aqueous layer combined and basified to pH 10 with conc. NH₄OH. The layers are separated and the residual aldehyde extracted with additional ether. The aqueous layer is acidified to pH 3 with 12M HCl, then extracted with CH₂Cl₂ (4 X). Large amounts of acid remained in the aqueous layer, so the aqueous layer is concentrated to dryness. Solid extractions with chloroform (4 X), followed by 10% MeOH/CH₂Cl₂ (4X) sequestered much of the acid in the organic layer. The organic layer is dried over Na₂SO₄ and concentrated to a tan solid (1.69 g). The solid is diluted with CHCl₃ and refluxed for 3 h. The flask is removed from heat, allowed to cool slightly, then filtered. The filtrate is concentrated to a tan solid (1.02 g). The solid is triturated with ether, filtered and dried to afford 747 mg (51%) of 3-methylfuro[2,3-c]pyridine-5-carboxylic acid as a light tan solid. MS for C₉H₇NO₃, (CI) *m*/*z*: 178 (M)⁺.

3-Methyl-furo[2,3c]pyridine-5-carboxylic acid (213 mg, 1.2 mmol) is added to 10 ml CH₂Cl₂ in a dried flask under nitrogen. The solution is treated with TEA (153 µL, 1.1 mmol) followed by bis (2-oxo-3-oxazolidinyl) phosphinic chloride (280 mg, 1.1 mmol), and the reaction is stirred 1 h at rt. The mixture is treated with (2R)-7-aza-[2.2.1]-Amine (212 mg, 1.0 mmol) in 2 ml CH₂Cl₂ and the reaction is stirred 4 h at rt. The mixture is washed with 1 x 10 ml saturated NaHCO₃, the organic layer is dried over K₂CO₃, and is concentrated *in vacuo* to a residue. The crude material is chromatographed over 25 g silica gel (230-400 mesh) eluting with 35% EtOAc/hexane. The appropriate fractions are combined and concentrated to afford the intermediate *exo*-*tert*-butyl (1S, 2R, 4R)-2-{[3-methylfuro[2,3-c]pyridine-5-carbonyl]amino}-7-azabicyclo[2.2.1]heptane-7-carboxylate as a residue. The residue is combined with 5 ml 2N methanolic HCl acid in 5 ml MeOH under nitrogen. The reaction is warmed in a 60°C bath for 2 h, is cooled, and is concentrated *in vacuo* to a residue. The residue is dissolved in 1 ml IPA, is diluted with 2 ml diethyl ether, and is allowed to crystallize. The white solid is washed with ether and is dried to give 136 mg (62%) of Example 1 as a white solid. HRMS (FAB) calcd for C₁₅H₁₇N₃O₂+H: 272.1399, found 272.1400 (M+H)⁺.

### Example 2: N-[(1S, 2R, 4R)-7-azabicyclo[2.2.1]hept-2-yl]thieno[2,3-c]pyridine-5-carboxamide dihydrochloride:

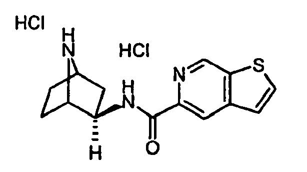

Glyoxylic acid monohydrate (20.3 g, 221 mmol) is combined with benzyl carbamate (30.6 g, 202 mmol) in ether (200 ml). The solution is allowed to stir for 24 h at rt. The resulting thick precipitate is filtered, and the residue is washed with ether, affording 21.5 g (47%) of {[(benzyloxy)carbonyl]amino}(hydroxy)acetic acid as a white solid. MS for C₁₀H₁₁NO₅, (CI) m/z: 226 (M)⁺.

{[(Benzyloxy)carbonyl]amino}(hydroxy)acetic acid (11.6 g, 51.5 mmol) is dissolved in absolute MeOH (120 ml) and chilled to 0-5°C in an ice bath. Concentrated sulfuric acid (2.0 ml) is carefully added drop-wise. The ice bath is allowed to expire as the solution stirred for 2 days. The reaction is quenched by pouring the mixture onto 500 g ice with saturated NaHCO₃ solution (400 ml). The solution is extracted with EtOAc (3 x 300 ml), and the combined organic layer is dried over MgSO₄, filtered, and concentrated to a pale oil that crystallized upon standing, yielding 12.3 g (94%) of methyl{[(benzyloxy)carbonyl]amino}(methoxy)acetate as a white solid. Anal. Calcd for C₁₂H₁₅NO₅: C, 56.91; H, 5.97; N, 5.53; Found: C, 56.99; H, 6.02; N, 5.60.

Methyl {[(benzyloxy)carbonyl]amino}(methoxy)acetate (11.76 g, 46.4 mmol) is dissolved in toluene (50 ml) under N₂ and placed in a 70°C bath. Phosphorous trichloride (23.2 ml, 46.4 mmol) is added drop-wise via syringe, and the solution is stirred for 18 h maintaining temperature. Trimethylphosphite (5.47 ml, 46.4 mmol) is then added dropwise, and stirring continued for an additional 2 h at elevated temperature. The mixture is concentrated *in vacuo* to an oil, and the crude material is dissolved in EtOAc (100 ml) and rinsed with saturated sodium bicarbonate (3 x 50 ml). The organic layer is dried over Na₂SO₄, filtered, and concentrated to a volume of 30 ml. The residue is stirred vigorously while hexane is added until a precipitate forms. The slurry is filtered, affording 12.88 g (84%) of methyl {[(benzyloxy)carbonyl]amino}(dimethoxyphosphoryl)acetate as a white solid. MS for C₁₃H₁₈NO₇P, (El) m/z: 331 (M)⁺.

Methyl {[(benzyloxy)carbonyl]amino}(dimethoxyphosphoryl)acetate (12.65 g, 38.2 mmol) is combined with Ac₂O (9.02 ml, 95.5 mmol) in MeOH (100 ml) in a PARR flask. The solution is hydrogenated with 10% Pd/C catalyst (0.640 g) at 45 psi for 3h. The catalyst is filtered off, and the filtrate is concentrated *in vacuo* to a residue. The residue is placed under hi vacuum and began to solidify. The white residue is dissolved in a small amount of EtOAc and stirred vigorously while pentane is added until a precipitate began to form. The precipitate is collected to afford 7.9 g (87%) of methyl (acetylamino)(dimethoxyphosphoryl)acetate as a white powder. MS for C₇H₁₄NO₆P, (CI) m/z: 240 (M)⁺.

2,3-Thiophene dicarboxaldehyde (1.40 g, 9.99 mmol) is dissolved in CH₂Cl₂ (100 ml) and chilled to in an ice bath. Methyl (acetylamino)(dimethoxyphosphoryl)acetate (2.63 g, 11.0 mmol) is dissolved in CH₂Cl₂ (50 ml) and combined with DBU (1.65 ml, 11.0 mmol). This solution is added drop-wise to the chilled thiophene solution. The reaction mixture is stirred cold for 1 h and then over night at rt. The mixture is concentrated *in vacuo* and the crude material is chromatographed over 300 g slurry-packed silica eluting with 50% EtOAc/hexane to provide two separate pools of product. Methyl thieno[2,3-c]pyridine-5-carboxylate elutes first; the appropriate fractions are combined and concentrated to yield 780 mg (41%) of a white solid. Methyl thieno[3,2-c]pyridine-6-carboxylate elutes second; the appropriate fractions are combined and concentrated to afford 740 mg (38%) of a yellow solid. MS results for methyl thieno[2,3-c]pyridine-5-carboxylate: MS for C₉H₇NO₂S, (EI) m/z: 193 (M)⁺. MS results for of methyl thieno[3,2-c]pyridine-6-carboxylate: MS for C₉H₇NO₂S, (EI) m/z: 193 (M)⁺.

Methyl thieno[2,3-c]pyridine-5-carboxylate (736 mg, 3.8 mmol) is dissolved in MeOH (16 ml) with water (2 ml). 2M NaOH (2.0 ml, 4.0 mmol) is added drop-wise, and the solution is stirred at rt. After 2 days (complete disappearance of ester by TLC), the mixture is concentrated *in vacuo.* The residue is dissolved in water (12 ml) and the pH is adjusted to 3.5 with 10% HCl. The slurry is filtered, and the cake is rinsed with ether, yielding 394 mg (58%) of thieno[2,3-c]pyridine-5-carboxylic acid as a white solid. HRMS (FAB) calcd for C₈H₅NO₂S+H: 180.0119, found 180.0123 (M+H)⁺.

Thieno[2,3c]pyridine-5-carboxylic acid (158 mg, 0.88 mmol) is coupled with (2R)-7-aza-[2.2.1]-Amine (187 mg, 0.88 mmol) and deprotected as described in Example 1 with non-critical variations to provide 140 mg (44%) of Example 2 as a white solid. HRMS (FAB) calcd for C₁₄H₁₅N₃OS+H: 274.1014, found 274.1011 (M+H)⁺.

### Example 3: N-[(1S, 2R, 4R)-7-azabicyclo[2.2.1]hept-2-yl]thieno[3,2-c]pyridine-6-carboxamide dihydrochloride:

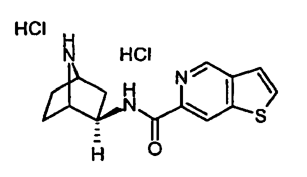

Methyl thieno[3,2-c]pyridine-6-carboxylate (Example 2) (678 mg, 3.5 mmol) is dissolved in MeOH (16 ml) with water (2 ml). 2M NaOH (1.8 ml, 3.6 mmol) is added drop-wise, and the solution is stirred at rt. After 2 days (complete disappearance of ester by TLC), the mixture is concentrated *in vacuo.* The residue is dissolved in water (12 ml), and the pH is adjusted to 3.5 with 10% HCl. The slurry is filtered, and the cake is rinsed with ether, yielding 268 mg (43%) of thieno[3,2-c]pyridine-6-carboxylic acid as a white solid. HRMS (FAB) calcd for C₈H₅NO₂S+H: 180.0119, found 180.0123 (M+H)⁺.

Thieno[3,2c]pyridine-5-carboxylic acid (77 mg, 0.43 mmol) is coupled with (2R)-7-aza-[2.2.1]-Amine (94 mg, 0.43 mmol) and deprotected as described in Example 1 with non-critical variations to provide give 55 mg (40%) of Example 3 as a white solid. HRMS (FAB) calcd for C₁₄H₁₅N₃OS+H: 274.1014, found 274.1017 (M+H)⁺.

### Example 4: N-[(2R)-7-azabicyclo[2.2.1]hept-2-yl]furo[2,3-c]pyridine-5-carboxamide dihydrochloride:

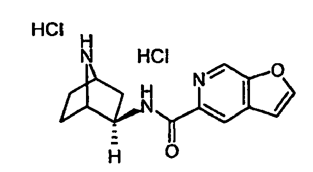

2-Chloro-3-pyridinol (20.0 g, 0.154 mole and NaHCO₃ (19.5g, 0.232 mole, 1.5 equ) are dissolved in 150 ml of water. The reaction mixture is placed in an oil bath at 90°C and after 5 min is treated with 37% aqueous formaldehyde (40.5 ml, 0.541 mole, 3.5 equ) which is added in six unequal doses; 12 ml initially, 3 x 8 ml followed by 1 x 2.2 ml all at 90 min intervals with the final 2.3 ml added after maintaining at 90°C overnight (15 h). After stirring in the 90°C bath for an additional 4 h, the flask is placed in ice bath, and the contents are treated with 100 ml of crushed ice, acidified with 39 ml of 6 N HCl to pH 1, and the precipitated material is stirred for 1.5 h in an ice bath. The undesired solid is removed by filtration, and the filtrate is extracted seven times with EtOAc. The combined organic extracts are concentrated at reduced pressure, treated with toluene, reconcentrated on rotary evaporator to azeotrope most of the water, suspended in CH₂Cl₂ and reconcentrated again at reduced pressure to obtain 19.9 g (81%) of 2-chloro-6-(hydroxymethyl)-3-pyridinol as a pale yellow solid sufficiently pure for subsequent reaction. MS for C₆H₆ClNO₂: *m*/*z:* 159 (M)⁺.

2-Chloro-6-(hydroxymethyl)-3-pyridinol (11.6 g, 72.7 mmol) and NaHCO₃ (18.3 g, 218 mmol) are dissolved in 200 ml water in a flask. The mixture is stirred until homogeneous, is cooled in an ice bath, is treated with iodine (19.4 g, 76.3 mmol), and is stirred over 60 h at rt as the cooling bath expired. The pH of the mixture is adjusted to 3 with 2N NaHSO₄, and the mixture is extracted with 4 x 50 ml EtOAc. The combined organic layer is dried over MgSO₄ and is concentrated in *vacuo* to a yellow solid. The crude solid is washed with EtOAc to provide 12.9 g (62%) of 2-chloro-6-(hydroxymethyl)-4-iodo-3-pyridinol as an off-white solid. The filtrate is concentrated to a small volume and is chromatographed over 250 g silica gel (230-400 mesh) eluting with EtOAc/CH₂Cl₂/hexane/acetic acid 2.5:4.5:4:0.1. The appropriate fractions are combined and concentrated to afford an additional 2.4 g (12%) of pure 2-chloro-6-(hydroxymethyl)-4-iodo-3-pyridinol. MS for C₆H₅ClINO₂, *m*/*z:* 285 (M)⁺.

To 2-chloro-6-(hydroxymethyl)-4-iodo-3-pyridinol (13.9 g, 48.6 mmol) in 80 ml CHCl₃/ 40 ml THF is added trimethylsilylacetylene (9.6 ml, 68 mmol), bis(triphenylphosphine) palladium dichloride (1.02 g, 1.46 mmol) and cuprous iodide (139 mg, 0.73 mmol) in a flask under nitrogen. The mixture is treated with Et₃N (21 ml, 151 mmol), is stirred 3 h at rt, and is diluted with 200 ml CHCl₃. The mixture is washed with 2 x 150 ml 5% HCl and the combined aqueous layers are extracted with 2 x 50 ml CHCl₃. The combined organic layer is washed with 1 x 100 ml 50% brine, is dried over MgSO₄, and is concentrated *in vacuo* to an amber oil. The crude material is chromatographed over 350 g silica gel (230-400 mesh), eluting with 35% EtOAc/hexane. The appropriate fractions are combined and concentrated to afford 11.4 g (92%) of 2-chloro-6-(hydroxymethyl)-4-[(trimethylsilyl)ethynyl]-3-pyridinol as a golden solid. MS for C₁₁H₁₄ClNO₂Si, *m*/*z:* 255 (M)⁺.

2-Chloro-6-(hydroxymethyl)-4-[(trimethylsilyl)ethynyl]-3-pyridinol (7.9 g, 31.2 mmol) is combined with cuprous iodide (297 mg, 1.6 mmol) in 60 ml EtOH/60 ml Et₃N in a flask. The reaction is warmed in a 70°C oil bath for 3.5 h, is cooled, and concentrated *in vacuo.* The residue is partitioned between 1 x 100 ml 5% HCl and 4 x 50 ml CH₂Cl₂. The combined organic layer is dried over MgSO₄ and is concentrated *in vacuo* to give 6.5 g of a crude amber solid. The crude material is chromatographed over 300 g silica gel (230-400 mesh) eluting with 30-40 % EtOAc/hexane to give two pools of fractions. The early-eluting fractions are combined and concentrated to afford 3.7 g (46%) of[7-chloro-2-(trimethylsilyl)furo[2,3-c]pyridin-5-yl]methanol as a white solid. The later-eluting fractions are combined and concentrated to provide 1.56 g (27%) of (7-chlorofuro[2,3-c]pyridin-5-yl)methanol as a white solid. For [7-chloro-2-(trimethylsilyl)furo[2,3-c]pyridin-5-yl]methanol: MS, Calculated for C ₁₁H₁₄ClNO₂Si: 255.0482. Found: 255.0481. For (7-chlorofuro[2,3-c]pyridin-5-yl)methanol: MS for C₈H₆ClNO₂, *m*/*z*: 183 (M)⁺.

[7-Chloro-2-(trimethylsilyl)furo[2,3-c]pyridin-5-yl]methanol (1.05 g, 4.1 mmol) is combined with 10% Pd/C (1.05 g) in 20 ml absolute EtOH in a flask. The suspension is treated with cyclohexene (4 ml, 40.1 mmol), and the mixture is refluxed for 2.5 h, and is filtered through celite. The solids are washed with 1:1 EtOH/CH₂Cl₂, and the filtrate is concentrated to a pale yellow solid. The residue is partitioned between 1 x 40 ml saturated sodium bicarbonate and CH₂Cl₂ (4 x 20 ml), and the combined organic layer is dried over MgSO₄. The organic layer is concentrated *in vacuo* to a residue (1.04 g) which is chromatographed over 50 g silica gel (230-400 mesh) eluting with 50-70% EtOAc/hexane. The appropriate fractions are combined and concentrated to afford 820 mg (90%) of [2-(trimethylsilyl)furo[2,3-c]pyridin-5-yl]methanol as a white solid. MS for C₁₁H₁₅NO₂Si, *mlz:* 221 (M)⁺.

5-Hydroxymethyl-2-trimethylsilyl-furo[2,3c]pyridine (770 mg, 3.48 mmol) is dissolved in 10 ml MeOH. 2N NaOH (3 ml, 6 mmol) is added, the reaction is stirred for 1.5 h at rt, and the mixture is concentrated *in vacuo.* The residue is partitioned between 1 x 20 ml water and CH₂Cl₂ (4 x 10 ml), and the combined organic layer is dried over K₂CO₃. The dried organic layer is concentrated *in vacuo* to afford 469 mg (90%) of furo[2,3-c]pyridin-5-yl methanol as a white solid. Analysis: Calculated for C₈H₇NO₂: C, 64.42; H, 4.73; N, 9.39. Found: C, 64.60; H, 4.56; N, 9.44.

Oxalyl chloride (685 µL, 7.8 mmol) is dissolved in 30 ml CH₂Cl₂ in a dried flask under nitrogen. The solution is cooled in a dry ice/acetone bath, is treated drop-wise with DMSO (1.11 ml, 15.6 mmol) in 1 x 5 ml CH₂Cl₂, and the mixture is stirred for 20 min. The mixture is treated with furo[2,3-c]pyridin-5-yl methanol (1.0 g, 6.7 mmol) in 1 x 10 ml CH₂Cl₂, is stirred 30 min at in a dry ice/acetone bath, and is treated with Et₃N (4.7 ml, 33.5 mmol). The reaction is allowed to warm to rt, is stirred 1 h, and is washed with 1 x 25 ml saturated NaHCO₃. The organic layer is dried over K₂CO₃ and is concentrated *in vacuo* to an orange solid. The crude material is chromatographed over 50 g silica gel (230-400 mesh) eluting with 33% EtOAc/hexane. The appropriate fractions are combined and concentrated to provide 850 mg (86%) offuro[2,3-c]pyridine-5-carbaldehyde as a white solid. MS for C₈H₅NO₂, (EI) *m*/*z*: 147 (M)⁺.

Furo[2,3-c]pyridine-5-carbaldehyde (850 mg, 5.8 mmol) is dissolved in 10 ml DMSO. To this solution is added potassium dihydrogen phosphate (221 mg, 1.6 mmol) in 3 ml water followed by sodium chlorite (920 mg, 8.2 mmol) in 7 ml water. The resulting reaction mixture is stirred for 3h at rt. The reaction is diluted with 25 ml water, the pH is adjusted to 10 with 2N NaOH, and the mixture is extracted with 3 x 20 ml ether. The pH of the aqueous layer is adjusted to 3.5 with 10% aqueous HCl and is extracted with 13 x 10 ml 10% MeOH/CH₂Cl₂. The organic layer is dried over Na₂SO₄ and is concentrated *in vacuo* to a pale oil. The residual DMSO is removed under a stream of nitrogen to provide a white paste. The paste is dissolved in MeOH and is concentrated to dryness. The white solid is washed with ether and is dried to give 890 mg (94%) of crude furo[2,3-c]pyridine-5-carboxylic acid. MS for C₈H₅NO₃, (ESI): 162.8 (M-H)⁻.

Furo[2,3c]pyridine-5-carboxylic acid (186 mg, 1.12 mmol) is coupled with (2R)-7-aza-[2.2.1]-Amine (212 mg, 1.0 mmol) and deprotected as described in Example 1 with non-critical variations to provide 165mg (50%) of Example 4 as a white solid. HRMS (FAB) calcd for C₁₄H₁₅N₃O₂+H: 258.1242, found 258.1244 (M+H)⁺.

### Example 5: N-[(1S, 2R, 4R)-7-azabicyclo[2.2.1]hept-2-yl]-3-ethylfuro[2,3-c]pyridine-5-carboxamide dihydrochloride:

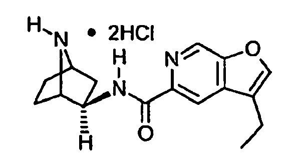

The synthesis of 3-ethylfuro[2,3-c]pyridine-5-carboxylic acid is carried out as outlined for the corresponding 3-methylfuro[2,3-c]pyridine-5-carboxylic acid described in Example 1 with non-critical changes by starting with 1-chloro-2-butene and 2-chloro-6-(hydroxymethyl)-4-iodo-3-pyridinol. HRMS (FAB) calcd for C₁₀H₉NO₃+H: 192.0661, found 192.0659 (M+H)⁺.

3-Ethyl furo[2,3c]pyridine-5-carboxylic acid (213 mg, 1.1 mmol) is coupled with (2R)-7-aza-[2.2.1]-Amine (212 mg, 1.0 mmol) and deprotected as described in Example 1 with non-critical variations to provide 208 mg (58%) of Example 5 as a white solid. HRMS (FAB) calcd for C₁₆H₁₉N₃O₂+H: 286.1555, found 286.1549 (M+H)⁺.

### Example 6: N-[(1S, 2R, 4R)-7-azabicyclo[2.2.1]hept-2-yl]-3-isopropyl-furo[2,3-c]pyridine-5-carboxamide dihydrochloride:

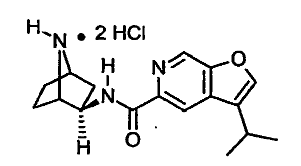

The synthesis of 3-isopropylfuro[2,3-c]pyridine-5-carboxylic acid is carried out as outlined for the corresponding 3-methylfuro[2,3-c]pyridine-5-carboxylic acid described in Example 1 with non-critical changes by starting with 1-chloro-3-methyl-2-butene and 2-chloro-6-(hydroxymethyl)-4-iodo-3-pyridinol. MS for C₁₁H₁₁NO₃, (EI) *m*/*z*: 205 (M)⁺.

3-Isopropyl furo[2,3c]pyridine-5-carboxylic acid (226 mg, 1.1 mmol) is coupled with (2R)-7-aza-[2.2.1]-Amine (212 mg, 1.0 mmol) and deprotected as described in Example 1 with non-critical variations to provide 258 mg (69%) of Example 6 as a white solid. MS for C₁₇H₂₁N₃O₂, (EI) *m*/*z*: 299 (M)⁺.

### Example 7: N-[(1S, 2R, 4R)-7-azabicyclo[2.2.1]hept-2-yl]-1H-pyrrolo[2,3-c]pyndme-5-carboxanude dihydrochloride:

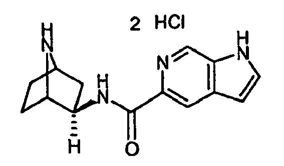

2,4-Lutidine (51.4 ml, 0.445 mole) is added drop-wise to 250 ml fuming sulfuric acid under nitrogen in a flask in an ice bath. The solution is treated portion-wise with potassium nitrate (89.9 g, 0.889 mole) over a 15 min period. The reaction is stirred 1h in an ice bath, 2 h at rt, is gradually warmed in a 100°C oil bath for 5 h, and then in a 130°C oil bath for 4 h. The mixture is cooled, is poured into 1000 ml ice, and the mixture is neutralized with NaHCO₃ (1,100g, 13.1 mole). The precipitated Na₂SO₄ is removed by filtration, the solid is washed with 500 ml water, and the filtrate is extracted with 4 x 500 ml ether. The combined organic layer is dried over MgSO₄ and is concentrated *in vacuo* to a yellow oil (50 g). The crude oil is distilled under vacuum to provide three fractions: 16 g recovered 2,4-lutidihe (85°C), 16 g 2,4-dimethyl-3-nitro-pyridine contaminated with 25% 2,4-dimethyl-5-nitro-pyridine (135-145°C), and 16 g 2,4-dimethyl-5-nitro-pyridine contaminated with 2,4-dimethyl-3-nitropyridine (145-153°C). 2,4-Dimethyl-3-nitropyridine: ¹H NMR (CDCl₃) δ 2.33, 2.54, 7.10, 8.43 ppm. 2,4-Dimethyl-5-nitropyridine: ¹H NMR (CDCl₃) δ 2.61, 2.62, 7.16, 9.05 ppm.

2,4-Dimethyl-5-nitropyridine/2,4-dimethyl-3-nitropyridine (75:25) (5.64 g, 37 mmol) is combined with benzeneselenic anhydride (8.2 g, 22.8 mmol) in 300 ml dioxane in a flask under nitrogen. The reaction is warmed to reflux for 10 h, is cooled, and is concentrated to a dark yellow oil. The oil is chromatographed over 250 g silica gel (230-400 mesh) eluting with 15% EtOAc/hexane. The appropriate fractions are concentrated to give 2.5 g (66%) of 2-formyl-4-methyl-5-nitropyridine. HRMS (EI) calcd for C₇H₆N₂O₃: 166.0378, found 166.0383 (M+H)⁺.

2-Formyl-4-methyl-5-nitropyridine (1.15 g, 6.9 mmol) is combined with p-toluene sulfonic acid (41 mg, 0.22 mmol) and ethylene glycol (1.41 ml, 25 mmol) in 25 ml toluene in a flask equipped with a Dean-Starke trap. The reaction is warmed to reflux for 2 h, is cooled to rt, and is concentrated *in vacuo* to an oily residue. The crude oil is chromatographed over 40 g silica gel (Biotage), eluting with 20% EtOAc/hexane. The appropriate fractions are combined and concentrated to give 1.31 g (90%) of 2-(1,3-dioxolan-2-yl)-4-methyl-5-nitropyridine. MS for C₉H₁₀N₂O₄, (EI) *m*/*z*: 210 (M)⁺.

2-(1,3-Dioxolan-2-yl)-4-methyl-5-nitropyridine (1.3 g, 6.2 mmol) is combined with DMF dimethyl acetal (1.12 ml, 8.4 mmol) in 15 ml DMF in a flask under nitrogen. The reaction is warmed to 90°C for 3 h, is cooled, and the volatiles are removed *in vacuo* (hi vacuum). The residue is combined with 1.25 g 5% Pd/BaSO₄ in 20 ml EtOH in a 250 ml PARR shaker bottle, and the mixture is hydrogenated at ambient pressure until uptake ceased. The catalyst is removed by filtration, and the filtrate is combined with 500 mg 10% Pd/C in a 250 ml PARR shaker bottle. The mixture is hydrogenated at ambient pressure for 1 h. No additional hydrogen uptake is observed. The catalyst is removed by filtration, and the filtrate is concentrated in *vacuo* to a tan solid. The crude material is chromatographed over 50 g silica gel (230-400 mesh), eluting with 7% MeOH/CH₂Cl₂. The appropriate fractions are combined and concentrated to give 819 mg (69%) of 5-(1,3-dioxolan-2-yl)-1H-pyrrolo[2,3-c]pyridine. MS for C₁₀H₁₀N₂O₂, (EI) *m*/*z*: 190 (M)⁺.

5-(1,3-Dioxolan-2-yl)-1H-pyrrolo[2,3-c]pyridine (800 mg, 4.21 mmol) is dissolved in 44 ml 10% aqueous acetonitrile in a flask. The solution is treated with p-toluene sulfonic acid (630 mg, 3.3 mmol), and the mixture is heated to reflux for 5 h. The mixture is cooled to rt, is concentrated *in vacuo,* and the resultant residue is diluted with 15 ml saturated NaHCO₃. The pale yellow solid is collected, washed with water, and is dried to give 500 mg (81%) of 1H-pyrrolo[2,3-c]pyridine-5-carbaldehyde. HRMS (FAB) calcd for C₈H₆N₂O+H: 147.0558, found 147.0564 (M+H)⁺.

1H-Pyrrolo[2,3-c]pyridine-5-carbaldehyde (500 mg, 3.42 mmol) is dissolved in 1.5 ml formic acid. The solution is cooled in an ice bath, is treated drop-wise with 30% aqueous hydrogen peroxide (722 µL, 6.8 mmol), is stirred 1 h in an ice bath, and allow to stand overnight at 5°C. The mixture is diluted with water, the solid is collected, washed with water and is dried to give 522 mg of an off-white solid. The formate salt is combined with 7 ml water, is diluted with 3 ml 2N NaOH, and the pH is adjusted to 3 with 5% aqueous HCl. The precipitate is collected and is dried to give 370 mg (67%) of 1H-pyrrolo[2,3-c]pyridine-5-carboxylic acid. HRMS (FAB) calcd for C₈H₆N₂O₂+H: 163.0508, found 163.0507 (M+H)⁺.

1H-Pyrrolo[2,3-c]pyridine-5-carboxylic acid(186 mg, 1.12 mmol) is coupled with (2R)-7-aza-[2.2.1]-Amine (212 mg, 1.0 mmol) and deprotected as described in Example 1 with non-critical variations to provide 133 mg (40%) of Example 7 as an off-white solid. HRMS(FAB) calc'd for C₁₄H₁₆N₄O+H: 257.1402. Found: 257.1391 (M+H)⁺.

### Example 8: N-[(1S, 2R, 4R)-7-azabicyclo[2.2.1]hept-2-yl]-1,3-benzodioxole-5-carboxamide hydrochloride:

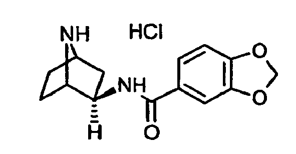

Piperonylic acid (91 mg, 0.55 mmol) is combined with TEA (0.076 ml, 0.55 mmol) and bis(2-oxo-3-oxazolidinyl)-phosphinic chloride (140 mg, 0.55 mmol) in CH₂Cl₂ (3 ml) and stirred at rt for 30 min. (2R)-7-Aza-[2.2.1]-Amine (106 mg, 0.5 mmol) is dissolved in CH₂Cl₂ (2 ml) and added drop-wise to the previous solution, stirring for 3 h. The reaction is washed with saturated NaHCO₃ solution (1x10ml), and the organic is dried over K₂CO₃, filtered, and concentrated to an oil. The crude material is chromatographed over 20 g slurry-packed silica, eluting with 35% EtOAc/hexane. The appropriate fractions are collected and concentrated to a glass. This material is dissolved in 1M HCl in MeOH (10 ml) and stirred overnight. Volatiles are removed *in vacuo,* and the residue is treated with IPA (2 ml) and ether (1 ml). The resulting precipitate is isolated via filtration, affording 50 mg (35%) of Example 8 as a white solid. MS for C₁₄H₁₆N₂O₃, MS (ESI): *m*/*z* 261 (M+H)⁺.

### Example 9: N-[(1S,2R,4R)-7-azabicyclo[2.2.1]hept-2-yl]isoquinoline-3-carboxamide dihydrochloride:

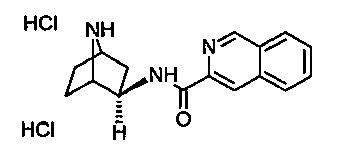

Isoquinoline-3-carboxylic acid hydrate is coupled with (2R)-7-aza-[2.2.1]-Amine (212 mg, 1.0 mmol) and deprotected as described in Example 1 with non-critical variations to provide 136 mg (47%) of Example 9 as a white solid. HRMS (FAB) calcd for C₁₆H₁₇N₃O +H: 268.1450, found 268.1452 (M+H)⁺.

### Example 10: N-[(1S, 2R, 4R)-7-azabicyclo[2.2.1]hept-2-yl]-1H-pyrrolo[2,3-c]pyridine-2-carboxamide dihydrochloride:

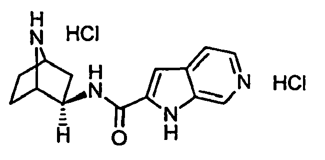

Potassium methoxide (2.8 g, 39.8 mmol) is combined with methyl oxalate (4.7 g, 39.8 mmol) in 80 ml Et₂O in a dry flask under nitrogen. The suspension is diluted with 12 ml CH₃OH, is treated dropwise with 4-methyl-3-nitropyridine (5 g, 36.2 mmol) in 25 ml Et₂O, and the black suspension is stirred 24 h at RT. The black solid is collected, washed with fresh Et₂O, and is dried to give 8.15 g (86%) of potassium-3-methoxy-1-(3-nitropyridin-4-yl)-3-oxoprop-1-en-2-olate. HRMS (FAB) calcd for C₉H₈N₂O₅ +H: 225.0511, found 225.0515 (M+H)⁺.

Potassium-3-methoxy-1-(3-nitropyridin-4-yl)-3-oxoprop-1-en-2-olate (4.19 g, 16 mmol) is combined with 10% Pd/C (400 mg) and 25 ml glacial acetic acid in a 250 ml PARR shaker flask. The mixture is hydrogenated at 40 PSI for 2 h. The catalyst is removed by filtration and the bulk of the volatiles are removed *in vacuo.* The residue is suspended in H₂O and the pH is adjusted to 7 with solid NaHCO₃. The solid is collected, washed with H₂O, and is dried to 1.98 g of a tan solid. The solid is triturated with hot EtOAc, is cooled, and is filtered to give 1.8 g (64%) of methyl 1H-pyrrolo[2,3-c]pyridine-2-carboxylate as a tan solid. HRMS (FAB) calcd for C₉H₈N₂O₂ +H: 177.0664, found 177.0671 (M+H)⁺.

Methyl 1H-pyrrolo[2,3-c]pyridine-2-carboxylate (1.0 g, 5.68 mmol) is suspended in 18 ml CH₃OH. The mixture is treated with 2N NaOH (6.24 ml, 12.5 mmol) and the mixture is stirred overnight at RT. The volatiles are removed *in vacuo,* the residue is dissolved in 10 ml H₂O, and the pH is adjusted to 4.3 with 5% aqueous HCl. The tan precipitate is collected, washed with H₂O, and is dried to 516 mg (56%) of 1H-pyrrolo[2,3-c]pyridine-2-carboxylic acid as a tan solid. HRMS (FAB) calcd for C₈H₆N₂O₂ +H: 163.0508, found 163.0498 (M+H)⁺.

1H-Pyrrolo[2,3-c]pyridine-2-carboxylic acid (122 mg, 0.75 mmol) is dissolved in DMF (5 ml) with DIEA (0.39 ml, 2.25 mmol) and (2R)-7-aza-[2.2.1]-Amine (175 mg, 0.83 mmol) and cooled to 0°C. HATU (285 mg, 0.75 mmol) is added portionwise, and the reaction is stirred overnight at RT, allowing the ice bath to expire. Volatiles are removed *in vacuo,* and the crude material is chromatographed over 25 g sluny-packed silica, eluting with 5% CH₃OH/CHCl₃. The appropriate fractions are collected and concentrated to a yellow oil. The oil is dissolved in 1M HCl in CH₃OH (10 ml) and stirred 2 days. Volatiles are again removed *in vacuo,* and the residue is treated with Et₂O (2 ml). The resulting precipitate is isolated via filtration, rinsed with Et₂O, and dried to afford 111 mg (45%) of Example 10 as a tan solid. HRMS (FAB) calcd for C₁₄H₁₆N₄O+H: 257.1402, found 257.1409 (M+H)⁺.

### Example 11: N-[(1S, 2R, 4R)-7-methyl-7-azabicyclo[2.2.1]hept-2-yl]furo[2,3-c]pyridine-5-carboxamide:

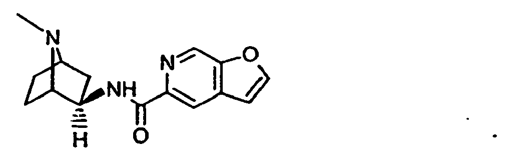

N-[(1S, 2R, 4R)-7-azabicyclo[2.2.1]hept-2-yl]furo[2,3-c]pyridine-5-carboxamide dihydrochloride (495 mg, 1.5 mmol) is combined with NaHCO₃ (252 mg, 3.0 mmol) in 12 ml water. The solution is treated with glacial acetic acid (2.2 ml), 37% aqueous formaldehyde (3 ml), and NaCNBH₃ (94 mg, 1.5 mmol), and the reaction is stirred 4 h at RT. The reaction is added dropwise to a mixture of 6 g NaHCO₃ in 50 ml water, the mixture is diluted with 10 ml conc. NH₄OH, and is extracted with 4 x 20 ml CHCl₃. The combined organic layer is dried over K₂CO₃ and is concentrated *in vacuo* to a pale paste. The crude material is chromatographed over 30 g silica gel (230-400 mesh), eluting with 3.5% CH₃OH/CHCl₃ + 1% NH₄OH. The appropriate fractions are combined and concentrated to give 270 mg of a pale foam. The foam is crystallized from Et₂O to provide 125 mg (31%) of Example 11 as a fine white solid. MS (EI) *m*/*z:* 271 (M)⁺.

### Example 12: N-[(1S, 2R, 4R)-7-methyl-7-azabicyclo[2.2.1]hept-2-yl]-l-benzofuran-5-carboxamide:

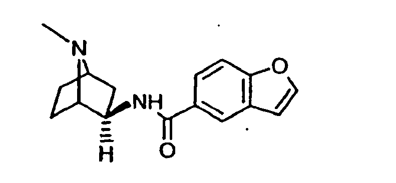

Example 12 is obtained as described in Example 11 with non-critical variations by starting with N-[(1S, 2R, 4R)-7-azabicyclo[2.2.1]hept-2-yl]-1-benzofuran-5-carboxamide (384 mg, 1.5 mmol) to give 280 mg (69%) of Example 12 as a fine white solid. MS (EI) *m*/*z*: 270 (M)⁺.

### Example 13: N-[(1S, 2R, 4R)-7-Azabicyclo[2.2.1]hept-2-yl]-1-benzofuran-6-carboxamide hydrochloride:

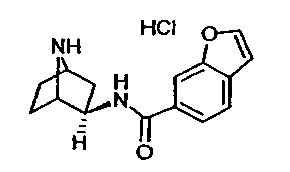

3-Hydroaybenzoic acid (13.8 g, 100 mmol) is dissolved in concentrated NH₄OH (200 mL) using an overhead stirrer and is treated slowly dropwise with a solution of iodine (23.4 g, 92 mmol) and KI (18.26 g, 110 mmol) in water (100 mL). The solution is stirred for 1 h at RT and then treated rapidly dropwise with concentrated HCl (180 mL). The white solid is collected via filtration, rinsed with water and dried overnight by pulling air through the solid to afford 13.05 g (54%) of 3-hydroxy-4-iodobenzoic acid as a tan solid.

3-Hydroxy-4-iodobenzoic acid (12.55 g, 47.5 mmol) is dissolved in CH₃OH (200 mL), treated slowly dropwise with thionyl chloride (32.3 mL, 442.9 mmol) at RT, then heated to reflux for 20 h. The mixture is concentrated to dryness and partitioned between CH₂Cl₂ (100 mL) and saturated NaHCO₃ (50 mL). Not all of the residue is solubilized, so the mixture is filtered and the solid is washed with a small amount of CH₂Cl₂ and CH₃OH. The original filtrate and the organic washes are combined, concentrated to dryness, dissolved in 10% CH₃OH / CH₂Cl₂ (200 mL), diluted with water (50 mL) and the layers separated. The organics are washed with saturated NaHCO₃ (2 x 50 mL), then water (50 mL), dried over Na₂SO₄ and concentrated to a tan solid. This solid is triturated with CH₂Cl₂ (50 mL) and filtered. The two solids are combined to afford 9.4 g (70%) of methyl 3-hydroxy-4-iodobenzoate as a beige solid. HRMS (FAB) calcd for C₈H₇IO₃ +H: 278.9520, found 278.9521 (M+H)⁺.

Methyl 3-hydroxy-4-iodobenzoate (5.22 g, 18.8 mmol) is combined with trimethylsilylacetylene (3.71 mL, 26.3 mmol), bis(triphenylphosphine)palladium dichloride (386 mg, 0.55 mmol) and cuprous iodide (54 mg, 0.28 mmol) in THF (20 mL) / CHCl₃ (40 mL) in a dry flask under nitrogen. TEA (8.14 mL< 58.4 mmol) is added and the mixture is heated to 50°C for 4 h. The mixture is diluted with CHCl₃ (60 mL), washed with 5% HCl (2 x 40 mL), dried over MgSO₄ and concentrated to a brown oily-solid (8.31 g). The crude material is chromatographed over a standard 90 g Biotage column, eluting with 10% EtOAc/hexane (1 L) followed by 15 % EtOAc/hexane (1 L). The appropriate fractions are combined and concentrated to afford 4.22 g (91%) of methyl 3-hydroxy-4-[(trimethylsilyl)ethynyl]benzoate as a yellow solid. HRMS (FAB) calcd for C₁₃H₁₆O₃Si +H: 249.0947, found 249.0947 (M+H)⁺.

Methyl 3-hydroxy-4-[(trimethylsilyl)ethynyl]benzoate (3.0 g, 12.1 mmol) is dissolved in 30 ml 1:1 EtOH/Et₃N, is treated with cuprous iodide (114 mg, 0.6 mmol), and the reaction is warmed to 75°C for 3 h. The mixture is treated with DARCO and 15 ml MeOH and is heated to reflux for 1 h. The reaction is filtered through a fine fritted glass funnel, the filtrate is treated with 3N NaOH (24.2 ml, 72.5 mmol), and the mixture is stirred overnight at RT. The mixture is concentrated to dryness, the residue is dissolved in 20 ml water, and the pH of the mixture is adjusted to 2 with 12N HCl. The resulting yellow precipitate is collected, washed with water, and is dried to give 1.83 g (93%) of benzofiuan-6-carboxylic acid as a tan solid. HRMS (FAB) calcd for C₉H₆O₃ +H: 163.0395, found 163.0389 (M+H)⁺.

1-Benzofiuan-6-carboxylic acid (162 mg, 1.0 mmol) is coupled with (2R)-7-aza-[2.2.1]-Amine (212 mg, 1.0 mmol) and deprotected as described in Example 10 with non-critical variations (carbamate eluted with 40% EtOAc/hexane) to provide 233 mg (76%) of Example 13 as a white solid. HRMS (FAB) calcd for C₁₅H₁₆N₂O₂ +H: 257.1290, found 257.1299 (M+H)⁺.

### Example 14: N-[(1S, 2R, 4R)-7-Azabicyclo[2.2.1]hept-2-yl]-1-benzofuran-5-carboxamide hydrochloride:

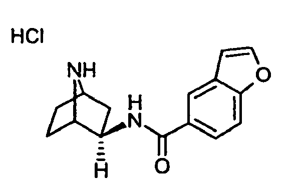

4-Hydroxy-3-iodobenzoic acid, see Edgar, K., Falling, S.N., *J. Org. Chem.,* **55** (18), 5287-5291, 1990, (59.6 g, 226 mmol) is combined with 3 N methanolic HCl (276 mL, 678 mmol) and heated to 65°C for 24 h, then concentrated to dryness. The residue is diluted with water, neutralized to pH 7 with 3 N NaOH and the resulting solid collected via filtration. The crude material is adsorbed onto silica gel and chromatographed over 1 kg of silica gel. Solubility problems necessitated flushing the column with 50% EtOAc/hexane. All fractions containing product are combined and concentrated to a solid (47.2 g). The material is recrystallized with EtOAc to afford methyl 4-hydroxy-3-iodobenzoate (16.6 g). A second recrystallization of the filtrate from EtOAc resulted in a second solid of comparable purity (6.2 g). The remaining solid (24.5 g) is carried on without further purification. Recrystallized total: 22.8 g (36%) as a white solid. HRMS (FAB) calcd for C₈H₇IO₃ +H: 278.9520, found 278.9534 (M+H)⁺.

Methyl 4-hydroxy-3-iodobenzoate (5.56 g, 20 mmol) is combined with trimethylsilylacetylene (3.96 mL, 28 mmol), bis(triphenylphosphine)palladium dichloride (414 mg, 0.6 mmol) and cuprous iodide (57 mg, 0.3 mmol) in THF (20 mL)/CHCl₃ (40 mL) in a dry flask under nitrogen. TEA (8.7 mL, 62.3 mmol) is added and the mixture heated to 50°C for 4 h. The mixture is diluted with CHCl₃ (60 mL), washed with 5% HCl (2 x 40 mL), dried over MgSO₄ and concentrated to a brown solid. The crude material is adsorbed onto silica gel and chromatographed over 200 g silica gel, eluting with 15% EtOAc/hexane (2 L) followed by 20% EtOAc/hexane (1 L). The appropriate fractions are combined and concentrated to afford 2.50 g (50%) of methyl 4-hydroxy-3-[(trimethylsilyl)ethynyl]benzoate as a yellow solid. Given poor recovery, the column is flushed with 25-30% EtOAc/hexane and fractions with the desired compound are combined to afford 2.73 g (55%) of the benzoate as an orange solid. HRMS (FAB) calcd for C₁₃H₁₆O₃SI +H: 249.0947, found 249.0955 (M+H)⁺.

Methyl 4-hydroxy-3-[(trimethylsilyl)ethynyl]benzoate (11 g, 44.5 mmol) is combined with cuprous iodide (423 mg, 2.2 mmol)and diisopropylamine (7.1 ml, 50 mmol) in 110 ml CH₃OH in a flask under nitrogen. The reaction is warmed to 60°C for 6 h, the volatiles are removed *in vacuo,* and the brown-green residue is chromatographed over 500 g silica gel (230-400 mesh) eluting with 20% EtOAc/hexane. Two separate groups of fractions are combined to provide 3.43 g (31%) of the early eluting methyl 2-trimethylsilylbenzofuran-5-carboxylate and 2.63 g (33%) of the later eluting methyl benzofuran-5-carboxylate. The pools are combined in 130 ml CH₃OH in a 500 ml. The solution is treated with 2N NaOH (46.8 ml, 93.6 mmol), is warmed to 50°C, and is stirred 2 h. The mixture is cooled, the volatiles are removed *in vacuo,* and the residue is dissolved in 50 ml H₂O. The pH of the mixture is adjusted to 2 with 12 N HCl, is diluted with 40 ml H₂O, and the mixture is cooled to 0°C. The off-white solid is collected, washed with water, and is dried to give 6.0 g. The solid is dried *in vacuo* over P₂O₅ for 18 h to give 4.6 g (99%) of benzofuran-5-carboxylic acid as an off-white solid. ¹H NMR (300 MHz, DMSO-*d*₆) δ) 7.08, 7.69, 7.91, 8.11, 8.30, 12.91 ppm.

Example 14 is obtained in 41 % yield by coupling 1-benzofuran-5-carboxylic acid with (2R)-7-aza-[2.2.1]-Amine (elution of the carbamate with 40% EtOAc/hexane), deprotecting, and making the salt according to the procedures provided for Example 10, making non-critical changes. Example 14 is a hygroscopic, amorphous yellow solid. HRMS (FAB) calcd for C₁₅H₁₆N₂O₂ +H: 257.1290, found 257.1304 (M+H)⁺.

### Example 15: N-[(1S, 2R, 4R)-7-Azabicyclo[2.2.1]hept-2-yl]-3-bromofuro[2,3-c]pyridine-5-carboxamide dihydrochloride:

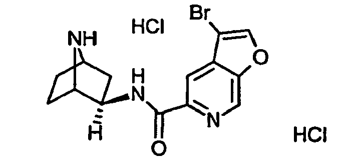

Furo[2,3-c]pyridin-5-yhnethyl acetate (5.17 g, 27.05 mmol) is dissolved in CH₂Cl₂ (130 mL), layered with saturated NaHCO₃ (220 mL), treated with Br₂ (8.36 mL, 162.3 mmol) and stirred very slowly for 4.5 h at RT. The mixture is stirred vigorously for 30 min, is diluted with CH₂Cl₂ (100 mL) and the layers are separated. The aqueous layer is extracted with CH₂Cl₂ (2 x 100 mL) and the combined organics are concentrated to a small volume under a stream of nitrogen. The solution is diluted with EtOH (200 mL), treated with K₂CO₃ (22.13 g, 160.1 mmol) and stirred for 2.5 days at RT. The mixture is concentrated to dryness, partitioned between 50% brine (200 mL) and CH₂Cl₂ (5 x 200 mL), dried over Na₂SO₄ and concentrated *in vacuo* to a yellow solid (6.07 g). The crude material is adsorbed onto silica gel (12 g) and chromatographed over 250 g slurry-packed silica gel, eluting with a gradient of 50% EtOAc/hexane to 100% EtOAc. The appropriate fractions are combined and concentrated *in vacuo* to afford 5.02 g (81%) of (3-bromofuro[2,3-c]pyridin-5-yl)methanol as a white solid. MS (EI) *m*/*z:* 227 (M⁺).

Oxalyl chloride (1.77 mL, 20.1 mmol) is combined with CH₂Cl₂ (60 mL) in a dry flask under nitrogen, cooled to -78°C, treated dropwise with DMSO (2.86 mL, 40.25 mmol) and stirred for 20 min. The cooled solution is treated dropwise with a solution of (3-bromofuro[2,3-c]pyridin-5-yl)methanol (4.0 mg, 17.5 mmol) in THF (50 mL), stirred for 1 h, then treated dropwise with Et₃N (12.2 mL, 87.5 mmol). The mixture is stirred for 30 min at -78°C, then 30 min at 0°C. The mixture is washed with saturated NaHCO₃ (120 mL) and the organics dried over K₂CO₃ and concentrated *in vacuo* to a dark yellow solid (3.91 g). The crude material is chromatographed over 150 g slurry-packed silica gel, eluting with 30% EtOAc/hexane. The appropriate fractions are combined and concentrated *in vacuo* to afford 3.93 g (99%) of 3-bromofuro[2,3-c]pyridine-5-carbaldehyde as a white solid. MS (EI) *m*/*z*: 225 (M⁺).

3-Bromofuro[2,3-c]pyridine-5-carbaldehyde (3.26 g, 14.42 mmol) is dissolved in THF (100 mL)/t-BuOH (50 mL)/H₂O (50 mL), treated with a single portion of NaOCl₂ (4.89 g, 43.3 mmol) and KH₂PO₄ (3.92 g, 28.8 mmol) and stirred at RT for 18 h. The white solid is collected via filtration (lot A) and the filtrate is concentrated *in vacuo* to dryness. The residue is suspended in water (25 mL), acidified to pH 2 with concentrated HCl and the resulting solid collected via filtration (lot B). Both lots are dried in a vacuum oven at 50°C for 18 h and combined to afford 3.52g (99%) of 3-bromofuro[2,3-c]pyridine-5-carboxylic acid as a white solid. MS (EI) *m*/*z:* 241 (M)⁺.

3-Bromofuro[2,3-c]pyridine-5-carboxylic acid (242 mg, 1.0 mmol) is coupled with (2R)-7-aza-[2.2.1]-Amine (233 mg, 1.1 mmol) (elution of the carbamate with 40% EtOAc/hexane) and deprotected as described in Example 10 with non-critical variations (elution of the carbamate with 30% EtOAc/hexane) to provide 167 mg (41%) of Example 15 as a white solid. HRMS (FAB) calcd for C₁₄H₁₄BrN₃O₂ +H: 336.0348, found 336.0346 (M+H)⁺.

### Example 16: N-[(1S, 2R, 4R)-7-Azabicyclo[2.2.1]hept-2-yl]-1-benzofuran-2-carboxamide hydrochloride:

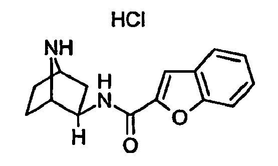

1-Benzofwan-2-carboxylic acid (162 mg, 1.0 mmol) is coupled with (2R)-7-aza-[2.2.1]-Amine (212 mg, 1.0 mmol) and deprotected as described in Example 10 with non-critical variations (elution of the carbamate with 30% EtOAc/hexane) to provide 150 mg (51%) of Example 16 as a white solid. HRMS (FAB) calcd for C₁₅H₁₆N₂O₂ +H: 257.1290, found 257.1279 (M+H)⁺.

### Example 17: N-[(1S, 2R, 4R)-7-Azabicyclo[2.2.1]hept-2-yl]furo[3,2-c]pyridine-6-carboxamide dihydrochloride:

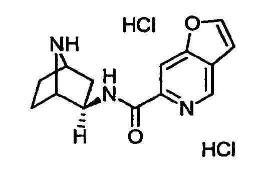

3-Bromofuran (8.99 mL, 100.0 mmol) is dissolved in DMF (8.5 mL), cooled to 0°C, treated dropwise with POCl₃ (9.79 mL, 105.0 mmol), stirred for 1 h at RT and then heated to 80°C for 2 h. The mixture is cooled to RT, poured over ice (1 kg) and neutralized to pH 9 with solid K₂CO₃. The mixture is stirred for 1 h, extracted with Et₂O (3 X 500 mL), dried over K₂CO₃ and concentrated to a dark brown oil. The crude material is chromatographed over 600 g slurry-packed silica gel, eluting with 6% EtOAc/hexane (4L), 8% EtOAc/hexane (2L), 10% EtOAc/hexane (1L), and finally 20% EtOAc/hexane. The appropriate fractions are combined and concentrated *in vacuo* to afford 14.22 g (81%) of 3-bromo-2-furaldehyde as a yellow oil. MS (EI) *m*/*z:* 174 (M⁺).

3-Bromo-2-furaldehyde (14.22 g, 81.3 mmol) is combined with ethylene glycol (6.55 mL, 117.4 mmol) and para-toluene sulfonic acid monohydrate (772 mg, 4.06 mmol) in benzene (200 mL) and heated to reflux with a Dean-Stark trap for 5 h. Additional ethylene glycol (1.64 mL, 29.41 mmol) and benzene (150 mL) are added and the solution is heated for an additional 2 h. The mixture is cooled to RT, treated with saturated NaHCO₃ and stirred for 0.5 h. The layers are separated and the organics are dried over Na₂SO₄ and concentrated to a brown oil (18.8 g). The crude material is chromatographed over 700 g slurry-packed silica gel, eluting with 15% EtOAc/hexane. The appropriate fractions are combined and concentrated *in vacuo* to afford 16.45 g (92%) of 2-(3-bromo-2-furyl)-1,3-dioxolane as a yellow-orange oil. MS (EI) *m*/*z*: 218 (M⁺).

2-(3-Bromo-2-furyl)-1,3-dioxolane (438 mg, 2.0 mmol) is dissolved in Et₂O (5 mL) in a dry flask under nitrogen, cooled to -78°C, treated dropwise with *tert-*butyllithium (2.59 mL, 4.4 mmol) and stirred for 1 h. DMF (178 µL, 2.3 mmol) in Et₂O (2 mL) is added dropwise, the mixture stirred for 4 h at -78°C, then treated with oxalic acid dihydrate (504 mg, 4.0 mmol) followed by water (2 mL). The cooling bath is removed and the mixture allowed to warm to RT over 1 h. The mixture is diluted with water (20 mL) and EtOAc (20 mL), the layers are separated and the aqueous layer extracted with EtOAc (1 X 20 mL). The organics are dried over Na₂SO₄ and concentrated to a yellow oil. The crude material is chromatographed over 12 g slurry-packed silica gel, eluting with 15% EtOAc/hexane. The appropriate fractions are combined and concentrated *in vacuo* to afford 228 mg (68%) of 2-(1,3-dioxolan-2-yl)-3-furaldehyde as a pale yellow oil. MS (EI) *m*/*z*: 168 (M⁺).

2-(1,3-Dioxolan-2-yl)-3-furaldehyde (2.91 g, 17.31 mmol) is combined with formic acid (17 mL, 451 mmol) and water (4.25 mL) and stirred at RT for 18 h. The mixture is slowly transferred into a solution of NaHCO₃ (45 g, 541 mmol) in water (600 mL), then strirred for 0.5 h. EtOAc (200 mL) is added, the layers separated and the aqueous layer extracted with EtOAc (2 X 200 mL). The combined organics are dried over Na₂SO₄ and concentrated to a yellow oil (3.28 g). The crude material is chromatographed over 90 g slurry-packed silica gel, eluting with 20% EtOAc/hexane. The appropriate fractions are combined and concentrated to afford 2.45 g of furan-2,3-dicarbaldehyde slightly contaminated with ethylene glycol diformate as a yellow oil. ¹H NMR (CDCl₃): δ 7.00 (d, *J* = 2 Hz, 1 H), 7.67 (d, *J* = 2 Hz, 1 H), 10.07 (s, 1 H), 10.49 (s, 1 H) ppm.

Methyl (acetylamino)(dimethoxyphosphoryl)acetate (2.34 g, 9.8 mmol) is dissolved in CHCl₃ (40 mL), treated with DBU (1.46 mL, 9.8 mmol), stirred for 5 min then added dropwise to a 0°C solution of furan-2,3-dicarbaldehyde (1.65 g; 8.9 mmol) in CHCl₃ (80 mL). The mixture is stirred for 2.5 h as the cooling bath expires then 5.5 h at RT and finally 24 h at 50°C. The mixture is concentrated *in vacuo* to a yellow oily-solid (6.66 g). The crude material is chromatographed over a standard 100g slurry-packed silica gel, eluting with 65% EtOAc/hexane. The appropriate fractions are combined and concentrated *in vacuo* to afford 1.30 g (82%) of methyl furo[3,2-c]pyridine-6-carboxylate as a yellow solid. MS (EI) *m*/*z*: 177 (M⁺).

Methyl furo[3,2-c]pyridine-6-carboxylate (1.55 g, 8.74 mmol) is dissolved in MeOH (30 mL) and H₂O (15 mL), treated with 3 N NaOH (6.4 mL) and stirred at RT for 7 h. The mixture is concentrated to dryness, dissolved in H₂O (10 mL) and acidified to pH 2 with concentrated HCl. The solution is concentrated to dryness, suspended in a smaller amount of water (7 mL) and the resulting solid collected via filtration (lot A). The filtrate is concentrated, triturated with water (3 mL) and the resulting solid collected via filtration (lot B). The filtrate from lot B is concentrated and carried on without further purification as an acid/salt mixture (lot C). Both lots A and B are dried in a vacuum oven at 50°C for 18 h to afford 690 mg (48%) for lot A and 591 mg (42%) for lot B of fiuo[3,2-c]pyridine-6-carboxylic acid as yellow solids. MS (CI) *m*/*z*: 164 (M + H⁺).

Furo[3,2-c]pyridine-6-carboxylic acid (199 mg, 1.0 mmol) is coupled with (2R)-7-aza-[22.1]-Amine (233 mg, 1.1 mmol) and deprotected as described in Example 10 with non-critical variations (elution of the carbamate with 45% EtOAc/hexane) to provide 257 mg (78%) of Example 17 as a white solid. HRMS (FAB) calcd for C₁₄H₁₅N₃O₂ +H: 258.1242, found 258.1253 (M+H)⁺.

### Example 18: N-[(1S, 2R, 4R)-7-azabicyclo[2.2.1]hept-2-yl]-3-chlorofuro[2,3-c]pyridine-5-carboxamide dihydrochloride:

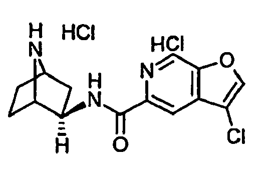

Furo[2,3-c]pyridin-5-ylmethanol (7.70 g, 51.63 mmol) is dissolved in pyridine (45 mL), treated with acetic anhydride (14.36 mL, 154.9 mmol) and stirred for 18 h at RT. The pyridine is removed under high vacuum, and the resulting residue is dissolved in EtOAc (200 mL), washed with 50% saturated sodium bicarbonate (4 x 90 mL), dried over MgSO₄ and concentrated *in vacuo* to afford 9.32 g (94%) offuro[2,3-c]pyridin-5-ylmethyl acetate as a yellow oil. MS (EI) *m*/*z*: 191 (M⁺), 277, 148, 119, 118, 86, 84, 77, 63, 51, 50..

Furo[2,3-c]pyridin-5-yhnethyl acetate (956 mg, 5 mmol) is dissolved in CH₂Cl₂ (40 mL) and cooled to 0°C. Chlorine gas is bubbled through the solution for 15 min, the cooling bath is immediately removed and the mixture stirred for 2 h. The mixture is re-cooled to 0°C, saturated with chlorine gas, the cooling bath removed and the solution warmed to RT. The solution is layered with saturated NaHCO₃ (20 mL), stirred gently for 2 h then stirred vigorously for 15 min. The mixture is diluted with saturated NaHCO₃ (50 mL), extracted with CH₂Cl₂ (1 x 40 mL then 1 x 20 mL), dried over K₂CO₃ and concentrated to a volume of 20 mL under a stream of nitrogen. The solution is diluted with EtOH (35 mL), treated with K₂CO₃ (4.09 g, 29.6 mmol) and stirred for 18 h at RT. Water (7 mL) is added and the mixture is stirred for 2 days. The mixture is concentrated to dryness, partitioned between 50% brine (50 mL) and CH₂Cl₂ (4 x 50 mL), dried over K₂CO₃ and concentrated *in vacuo* to a brown solid (833 mg). The crude material is chromatographed over a standard 40 g Biotage column, eluting with 50% EtOAc/hexane. The appropriate fractions are combined and concentrated to afford 624 mg (68%) of (3-chlorofuro[2,3-c]pyridin-5-yl)methanol as a yellow oil. ¹H NMR (DMSO-*d₆*): δ 4.69 (d, *J*= 6 Hz, 2 H), 5.56 (t, *J* = 6 Hz, 1 H), 7.69 (s, 1 H), 8.55 (s, 1 H), 8.93 (s, 1 H) ppm.

Oxalyl chloride (231 µL, 2.6 mmol) is combined with CH₂Cl₂ (10 mL), cooled to -78°C, treated dropwise with DMSO (373 µL, 5.3 mmol) and stirred for 20 min. The cooled solution is treated dropwise with a solution of (3-chlorofuro[2,3-c]pyridin-5-yl)methanol (420 mg, 2.3 mmol) in THF (5 mL)/CH₂Cl₂ (5 mL), stirred for 1 h, then treated dropwise with Et₃N (1.59 mL, 11.45 mmol). The mixture is stirred for 30 min at -78°C, then 30 min at 0°C. The mixture is washed with saturated NaHCO₃ (20 mL) and the organics dried over K₂CO₃ and concentrated *in vacuo* to a yellow solid (410 mg). The crude material is chromatographed over 20 g slurry-packed silica gel, eluting with 15% EtOAc/hexane. The appropriate fractions are combined and concentrated *in vacuo* to afford 322 mg (77%) of 3-chlorofuro[2,3-c]pyridine-5-carbaldehyde as a white solid. ¹H NMR (CDCl₃): δ 7.89 (s, 1 H), 8.33 (s, 1 H), 9.02 (s, 1 H), 10.18 (s, 1 H) ppm.

3-Chlorofuro[2,3-c]pyridine-5-carbaldehyde (317 mg, 1.74 mmol) is dissolved in THF (10 mL)/t-BuOH (5 mL)/H₂O (5 mL), treated with a single portion of sodium chlorite (592 mg, 5.24 mmol) and KH₂PO₄ (473 mg, 3.48 mmol) and stirred at RT for 18 h. The reaction mixture is concentrated *in vacuo* to dryness, suspended in water (10 mL), acidified to pH 3.5 with concentrated HCl and stirred at RT for 2 h. The resulting solid is filtered, washed with water and dried in a vacuum oven at 40°C for 18 h to afford 364 mg of 3-chlorofuro[2,3-c]pyridine-5-carboxylic acid as a white solid. MS (EI) *m*/*z*: 197 (M⁺).

3-Chlorofuro[2,3-c]pyridine-5-carboxylic acid (147 mg, 0.75 mmol) is coupled with (2R)-7-aza-[2.2.1]-Amine (175 mg, 0.83 mmol) and deprotected as described in Example 10 with non-critical variations (elution of the carbamate with 40% EtOAc/hexane) to provide 157 mg (58%) of Example 18 as a pale yellow solid. HRMS (FAB) calcd for C₁₄H₁₄ClN₃O₂+H: 292.0853, found 292.0843 (M+H)⁺.

### Example 19: N-[(1S, 2R, 4R)-7-azabicyclo[2.2.1]hept-2-yl]thieno[3,4-c]pyridine-6-carboxamide dihydrochloride:

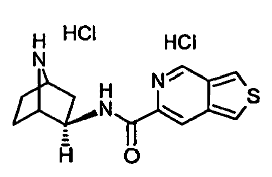

3,4-Dibromothiophene (12.5 ml, 113 mmol) is combined with CuCN (30.4 g, 339 mmol) in DMF (40 ml) in a dry flask under nitrogen utilizing an over-head stirrer. The reaction is allowed to reflux at 180°C for 5 h. The dark mixture is then poured into a solution of FeCl₃ (113.6 g, 700 mmol) in 1.7M HCl (200 ml) and heated at 65°C for 0.5 h, again using the over-head stirrer. The reaction is cooled to rt and extracted with CH₂Cl₂ (7 x 300 ml). Each extract is washed individually with 200 ml *each* 6M HCl (2X), water, saturated NaHCO₃, and water. The organics are then combined, dried over MgSO₄, filtered, and concentrated, affording 10.49 g (69%) of 3,4-dicyanothiophene as a fluffy tan solid. HRMS (EI) calcd for C₆H₂N₂S: 133.9939, found 133.9929 (M⁺).

3,4-Dicyanothiophene (5.0 g, 37.2 mmol) is suspended in benzene (150 ml) in a dry flask under nitrogen utilizing an over-head stirrer. Diisobutyl aluminum hydride (1.0M in toluene) (82.0 ml, 82.0 mmol) is added dropwise, and the reaction stirred at rt for 2 h. The reaction is then carefully quenched with MeOH (5 ml) and poured onto 30% H₂SO₄ (60 ml) with ice (200 g). The slurry is stirred until all lumps are dissolved, and the layers are allowed to separate. The aqueous layer is extracted with Et₂O (4 x 200 ml), and the combined organics are dried over MgSO₄, filtered, and adsorbed onto silica. The crude material is chromatographed over 225 g slurry-packed silica, eluting with 40% EtOAc/hexane. The appropriate fractions are combined and concentrated to afford 1.88 g (36%) of 3,4-thiophene dicarboxaldehyde as a pale yellow solid. MS (EI) m/z: 140 (M⁺).

3,4-Thiophene dicarboxaldehyde (1.0 g, 7.13 mmol) is dissolved in CH₂Cl₂. (40 ml) and chilled to 0°C. Methyl (acetylamino)(dimethoxyphosphoryl)acetate (1.88 g, 7.85 mmol) is dissolved in CH₂Cl₂ (30 ml) and combined with DBU (1.1 ml, 7.85 mmol). This solution is added dropwise to the chilled thiophene solution after stirring for 5 min. The reaction mixture is stirred at 0°C for 1 h and then overnight at rt. The volatiles are removed *in vacuo* and the crude material is chromatographed over 68 g slurry-packed silica eluting with 70% EtOAc/hexane. The appropriate fractions are combined and concentrated to yield 2.09 g of the carbinol intermediate as a white foam. The intermediate is dissolved in CHCl₃ (50 ml) and treated with DBU (1.32 ml, 8.8 mmol) and trifluoracetic anhydride (1.24 ml, 8.8 mmol) in a drop-wise fashion. The reaction is stirred overnight at rt and is then quenched with saturated NaHCO₃ solution (50ml). The layers are separated, and the aqueous layer is extracted with CHCl₃ (2 x 50 ml). The combined organics are dried over MgSO₄, filtered, and concentrated to a yellow oil. This oil is chromatographed over 50 g slurry-packed silica, eluting with 90% EtOAc/hexane. The appropriate fractions are combined and concentrated to afford 1.2 g (88%) of methyl thieno[3,4-c]pyridine-6-carboxylate as a yellow solid. MS (EI) *m*/*z*: 193 (M⁺).

Methyl thieno[3,4-c]pyridine-6-carboxylate (250 mg, 1.3 mmol) is dissolved in MeOH (7 ml) and water (1 ml). 2M NaOH (0.72 ml, 1.43 mmol) is added drop-wise. The reaction is stirred overnight at rt and is monitored by TLC. The volatiles are removed *in vacuo* and the residue is dissolved in water (2 ml). 10% HCl is used to adjust the pH to 3, and the reaction again stirred overnight at rt. The aqueous solution is extracted repeatedly with EtOAc (20 x 10 ml). The combined organics are dried over MgSO₄, filtered, and concentrated to a yellow solid. The amount of isolated product via extraction is minimal (67 mg), so the aqueous layer is concentrated and found to contain the majority of product. Extraction of the solid aqueous residue with EtOAc provided 225 mg (97%) of thieno[3,4-c]pyridine-6-carboxylic acid as a yellow solid. MS (EI) *m*/*z:* 179 (M⁺).

Thieno[3,4-c]pyridine-6-carboxylic acid (97 mg, 0.54 mmol) is coupled with (2R)-7-aza-[2.2.1]-Amine (126 mg, 0.6 mmol) and deprotected as described in Example 10 with non-critical variations (elution of the carbamate with 35% EtOAc/hexane) to provide 15 mg (15%) of Example 19 as a dark yellow solid. HRMS (FAB) calcd for C₁₄H₁₅N₃OS+H: 274.1014, found 274.1004 (M+H)⁺.

### Example 20: N-[(1S, 2R, 4R)-7-azabicyclo[2.2.1]hept-2-yl]dibenzo[b,d]thiophene-2-carboxamide hydrochloride:

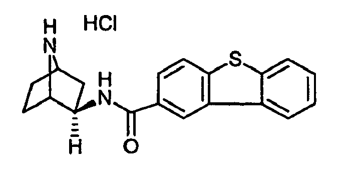

Dibenzo[b,d]thiophene-2-carboxylic acid (171 mg, 0.75 mmol) is coupled with (2R)-7-aza-[2.2.1]-Amine (175 mg, 0.83 mmol) and deprotected as described in Example 10 with non-critical variations (elution of the carbamate with 35% EtOAc/hexane) to provide 183 mg (68%) of Example 20 as a tan solid. HRMS (FAB) calcd for C₁₉H₁₈N₂OS+H: 323.1218, found 323.1217 (M+H)⁺.

### Example 21: N-[(1S, 2R, 4R)-7-azabicyclo[2.2.1]hept-2-yl]benzothieno[2,3-c]pyridine-3-carboxamide dihydrochloride:

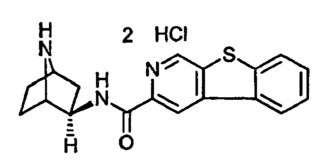

N-Butyl lithium (150.6 ml, 241 mmol) is added dropwise to Et₂O (100 ml) at -20°C under N₂. 3-Bromothianaphthene (10.5 ml, 80.3 mmol) is dissolved in ether (50 ml) and also added dropwise to the chilled solution, stirring cold for 0.5 h. DMF (16.3 ml, 210 mmol) is suspended in Et₂O (75 ml) and added in a similar manner, and the solution stirred an additional 15 h at -20°C. The reaction is quenched on ice (300 g) and 10% sulfuric acid (200 ml) and is stirred until both layers turned yellow in color. The resulting slurry is filtered and the cake is allowed to dry in the air stream, affording 8.69 g (60%) of 1-benzothiophene-2,3-dicarbaldehyde as a yellow solid. MS (EI) *m*/*z:* 190 (M⁺).

1-Benzothiophene-2,3-dicarbaldehyde (1.91 g, 10.0 mmol) is dissolved in CH₂Cl₂ (100 ml) and chilled to 0°C. Methyl (acetylamino)(dimethoxyphosphoryl) acetate (2.63 g, 11.0 mmol) is dissolved in CH₂Cl₂ (50 ml) and combined with DBU (1.65 ml, 11.0 mmol), stirring for 5 min. This solution is added dropwise to the chilled thiophene solution. The reaction mixture is stirred cold for 1 h and then over night at RT. The volatiles are removed *in vacuo* and the crude material is chromatographed over 500 g slurry-packed silica eluting with 50% EtOAc/hexane. Two sets of fractions are collected:
The appropriate earlier-eluting fractions are combined and concentrated to give 300 mg (12%) methyl benzothieno[2,3-c]pyridine-3-carboxylate: ¹H NMR (CDCl₃) δ 4.12, 7.62, 7.69, 7.99, 8.37, 8.92, 9.30 ppm.
The appropriate later-eluting fractions are combined and concentrated to yield 1.75 g (73%) of methyl benzothieno[3,2-c]pyridine-3-carboxylate as a white solid): ¹H NMR (CDCl₃) δ 4.10, 7.63, 7.96, 8.37, 8.72, 9.51 ppm. MS (EI) *m*/*z:* 243 (M⁺).

Methyl benzothieno[2,3-c]pyridine-3-carboxylate (200 mg, 0.82 mmol) is dissolved in MeOH (4 ml) with water (0.5 ml). 2M NaOH (0.45 ml, 0.9 mmol) is added dropwise and the solution stirred at RT. When the reaction is complete by TLC, the volatiles are removed *in vacuo,* and the residue is dissolved in water (10 ml). The pH is adjusted to 3.5 with concentrated HCl, and the solution is allowed to stir over night. The slurry is then filtered and the cake is dried in an air stream, yielding 162 mg (86%) of benzothieno[2,3-c]pyridine-3-carboxylic acid as a tan solid. ¹H NMR (DMSO-*d₆*) δ 7.62, 7.73, 8.21, 8.70, 9.05, 9.42 ppm.

Benzothieno[2,3-c]pyridine-3-carboxylic acid (75 mg, 0.33 mmol) is coupled with (2R)-7-aza-[2.2.1]-Amine (77 mg, 0.36 mmol) and deprotected as described in Example 10 with non-critical variations (elution of the carbamate with 45% EtOAc/hexane) to provide 115 mg (89%) of Example 21 as a white solid. HRMS (FAB) calcd for C₁₈H₁₇N₃OS+H: 324.1170, found 324.1177 (M+H)⁺.

### Example 22: N-[(1S, 2R, 4R)-7-azabicyclo[2.2.1]hept-2-yl]benzothieno[3,2-c]pyridine-3-carboxamide dihydrochloride:

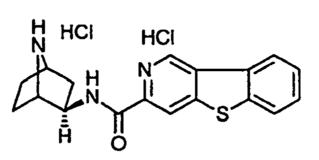

Example 22 is prepared using the method outlined in Example 21 making non-critical changes by coupling the acid derived from the saponification of methyl benzothieno[3,2-c]pyridine-3-carboxylate with (2R)-7-aza-[2.2.1]-Amine to provide Example 22 as a white fluffy solid. HRMS (FAB) calcd for C₁₈H₁₇N₃OS+H: 324.1170, found 324.1180 (M+H)⁺.

### Example 23: N-[(1S, 2R, 4R)-7-azabicyclo[2.2.1]hept-2-yl]dibenzo[b,d]furan-2-carboxamide dihydrochloride:

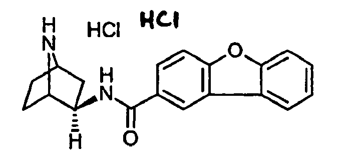

Dibenzo[b,d]furan-2-carboxylic acid (159 mg, 0.75 mmol) is coupled with (2R)-7-aza-[2.2.1]-Amine (176 mg, 0.83 mmol) and deprotected as described in Example 10 with non-critical variations (elution of the carbamate with 40% EtOAc/hexane) to provide 225 mg (89%) of Example 23 as a white solid HRMS (FAB) calcd for C₁₉H₁₈N₂O₂+H: 307.1446, found 307.1449 (M+H)⁺.

### ExamDle 24: N-[(1S, 2R, 4R)-7-azabicyclo[2.2.1]hept-2-yl]benzothiophene-5-carboxamide hydrochloride:

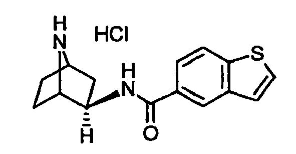

1-Benzothiophene-5-carboxylic acid (Badger, G.M.; Clark, D.J.; Davies, W.; Ferrer, K.T.; Kefford, N.P. Thionaphthencarboxylic Acids. *J. Am. Chem. Soc.* **1957**, *79,* 2624-2630. Amin, H.B.; Awad, A.A.; Archer, W.J.; Taylor, R. Electrophilic Aromatic Substitution. Part 33. Partial Rate Factors for Protiodetritiation of Benzo[*b*]thiophen; the Resonance-dependent Reactivity of the Ring Positions. *J*. *Chem. Soc., Perkin Trans. II* **1982**, 1489-1492.) (134 mg, 0.75 mmol) is coupled with (2R)-7-aza-[2.2.1]-Amine (175 mg, 0.83 mmol) and deprotected as described in Example 10 with non-critical variations (elution of the carbamate with 50% EtOAc/hexane) to provide 112 mg (48%) of Example 24 as an ivory solid. HRMS (FAB) calcd for C₁₅H₁₆N₂OS+H: 273.1061, found 273.1054.

### Example 25: N-[(1S, 2R, 4R)-7-azabicyclo[2.2.1]hept-2-yl]-2-methyl-1-henzofuran-6-carboxamide hydrochloride:

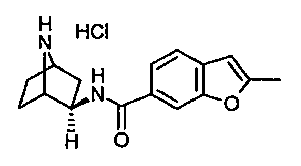

Methyl 3-hydroxy-4-iodobenzoate (2.0 g, 7.2 mmol) is dissolved in DMF (15 ml) with propargyl trimethylsilane (1.19 ml, 7.98 mmol), bis(triphenylphosphine)palladium dichloride (71 mg, 0.10 mmol), copper iodide (55 mg, 0.29 mmol), and piperidine (1.14 ml, 11.5 mmol) in a dry flask under nitrogen. The reaction is heated at 45°C for 7 h and then stirred at RT overnight. The reaction mixture is diluted with EtOAc (75 ml) and washed with 50% 1:1 NaCl/NaHCO₃ (4 x 25 ml). The organic is dried over Na₂SO₄ and concentrated to an amber oil. The crude material is chromatographed over 100 g slurry-packed silica gel, eluting with 20% EtOAc/hexane. The appropriate fractions are collected and concentrated to afford 1.5 g of a mixture of esters, neither of which could be isolated independently from one another. The mixture of esters is dissolved in MeOH (15 ml) and water (1 ml) and treated with 2N NaOH (3.15 ml, 6.3 mmol). After 2 days, the volatiles are removed *in vacuo* and the residue dissolved in water (5 ml). TLC indicates the presence of 2 spots, so the solution is washed with Et₂O (3x10 ml) to remove the undesired reaction component. The pH is then adjusted to 3 with concentrated HCl and the resulting slurry is filtered. The isolated cake is dried overnight to yield 789 mg (85%) of 2-methyl-1-benzofuran-6-carboxylic acid as a pale yellow solid. MS (EI) *m*/*z* 176 (M)⁺.

2-Methyl-1-benzofuran-6-carboxylic acid (176 mg, 1.0 mmol) is coupled with (2R)-7-aza-[2.2.1]-Amine (233 mg, 1.1 mmol) and deprotected as described in Example 10 with non-critical variations (elution of the carbamate with 40% EtOAc/hexane) to provide 102 mg (33%) of Example 25 as a white solid. HRMS (FAB) calcd for C₁₆H₁₈N₂O₂+H: 271.1446, found 271.1454 (M+H)⁺.

### Example 26: N-[(1S, 2R, 4R)-7-azabicyclo[2.2.1]hept-2-yl]-3-methyl-1-benzofuran-6-carboxamide hydrochloride:

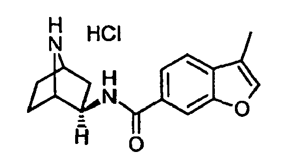

Methyl 3-hydroxy-4-iodobenzoate (2.0 g, 7.2 mmol) is dissolved in DMF (25 ml) in a dry flask under nitrogen. Allyl bromide (0.65 ml, 7.55 mmol) is added dropwise, followed by the careful addition of sodium hydride (303 mg, 7.55 mmol). The reaction is stirred over night at RT and is quenched onto water (30 ml). The mixture is extracted with EtOAc (3 x 30 ml), and the combined organics are washed with 50% saturated brine (4 x 25 ml), dried over MgSO₄, filtered, and concentrated under high-vacuum to an oil that solidified upon standing to obtain 2.28 g (100%) of methyl 3-(allyloxy)-4-iodobenzoate as a tan solid. HRMS (FAB) calcd for C₁₁H₁₁IO₃+H: 318.9833, found 318.9831 (M+H)⁺.

Methyl 3-(allyloxy)-4-iodobenzoate (2.0 g, 6.28 mmol) is dissolved in DMF (15 ml) and treated with palladium acetate (71 mg, 0.31 mmol), Na₂CO₃ (1.67 g, 15.7 mmol), sodium formate (427 mg, 6.28 mmol), and (n-Bu)₄N⁺Cl⁻ hydrate (1.92 g, 6.92 mmol). The reaction is stirred at 80°C for 2 days. The mixture is then filtered, and the liquor is diluted with EtOAc (75 ml). The solution is washed with 50% saturated brine (4 x 25 ml) followed by 5% HCl (1 x 25 ml). The organic is dried over Na₂SO₄, filtered, and concentrated to a brown oil. The crude material is chromatographed over 50 g slurry-packed silica gel, eluting with 20% EtOAc/hexane. The appropriate fractions are combined and concentrated to afford 797 mg (67%) of methyl 3-methyl-1-benzofuran-6-carboxylate as a pale oil. HRMS (FAB) calcd for C₁₁H₁₀O₃+H: 191.0708, found 191.0714 (M+H)⁺.

Methyl 3-methyl-l-benzofuran-6-carboxylate (720 mg, 3.78 mmol) is dissolved in MeOH (10 ml) and treated with 2N NaOH (2.27 ml, 4.5 mmol). Following overnight stirring, the volatiles are removed *in vacuo.* The residue is dissolved in water (5 ml) and concentrated HCl is used to adjust the pH to 3. The resulting slurry is filtered after overnight stirring to afford 545 mg (82%) of 3-methyl-1-benzofuran-6-carboxyic acid as a white solid. HRMS (FAB) calcd for C₁₀H₈O₃+H: 177.0552, found 177.0551 (M+H)⁺.

3-Methyl-1-benzofuran-6-carboxylic acid (176 mg, 1.0 mmol) is coupled with (2R)-7-aza-[2.2.1]-Amine (233 mg, 1.1 mmol) and deprotected as described in Example 10 with non-critical variations (elution of the carbamate with 40% EtOAc/hexane) to provide 225 mg (74%) of Example 26 as a white solid. HRMS (FAB) calcd for C₁₆H₁₈N₂O₂+H: 271.1446, found 271.1437 (M+H)⁺.

### Example 27: N-[(1S, 2R, 4R)-7-azabicyclo[2.2.1]hept-2-yl]benzofuro[3,2-c]pyridine-3-carboxamide dihydrochloride:

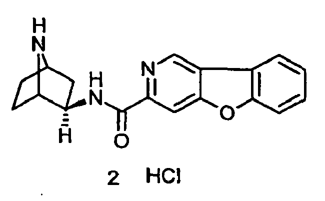

Benzofuran (11.02 ml, 100 mmol) and potassium acetate (1.96g, 200 mmol) are dissolved in CHCl₃ (50 ml). Bromine (10.3 ml, 200 mmol) is dissolved in CHCl₃ (20 ml) and added dropwise. Following addition, the reaction is heated at 50°C for 5 h. The mixture is cooled to RT and quenched onto 5% sodium bisulfite solution (100 ml). The layers are allowed to separate, and the organic is washed with 5% NaHCO₃ (1 x 100 ml), dried over Na₂SO₄, filtered, and concentrated to a green oil. The crude material is chromatographed over 1 kg slurry-packed silica eluting with 100% pentane. The appropriate fractions are combined and concentrated to give 15.86 g (57%) of 2,3-dibromobenzofuran as a pale oil. HRMS (EI) calcd for C₈H₄Br₂O: 273.8630, found 273.8624 (M)⁺.

2,3-Dibromobenzofuran (1.37 g, 5.0 mmol) is dissolved in Et₂O (20 ml) in a dry flask under nitrogen and cooled to -78°C. *tert*-Butylithium (6.47 ml, 11.0 mmol) is added dropwise, and the chilled solution is stirred 1 h. DMF (0.45 ml, 5.75 mmol) is dissolved in Et₂O (5 ml) and also added dropwise, and the mixture is stirred at -78°C for another 4 h. The reaction is warmed to RT, and then oxalic acid dihydrate (1.26 g, 10.0 mmol) and water (5 ml) are added. The reaction continues stirring at RT for 2 days and is then diluted with water (25 ml) and EtOAc (35 ml). The layers are allowed to separate, and the aqueous is extracted with EtOAc (1 x 35 ml). The organics are combined, dried over Na₂SO₄, filtered, and concentrated to an orange oil that solidified upon standing. The crude material is chromatographed over 100 g slurry-packed silica, eluting with 20% EtOAc/hexane. The appropriate fractions are combined and concentrated to afford 628 mg (56%) of 3-bromo-1-benzofuran-2-carbaldehyde as a yellow crystalline solid. HRMS (FAB) calcd for C₉H₅BrO₂+H: 224.9552, found 224.9555 (M+H)⁺.

3-Bromo-1-benzofuran-2-carbaldehyde (5.49 g, 24.4 mmol) is combined with para-toluene sulfonic acid hydrate (232 mg, 1.2 mmol) and ethylene glycol (2.44 ml, 43.9 mmol) in benzene (75 ml). The reaction is refluxed with a Dean-Stark trap for 5 h. The mixture is cooled to RT and diluted with saturated NaHCO₃ solution (20 ml) and left to stir for an additional 12 h. The layers are allowed to separate, and the organic layer is dried over Na₂SO₄, filtered, and concentrated to afford 6.6 g (100%) of 3-bromo-2-(1,3-dioxolan-2-yl)-1-benzofuran as a dark brown oil. HRMS (FAB) calcd for C₁₁H₉BrO₃+H: 268.9814, found 268.9821 (M+H)⁺.

3-Bromo-2-(1,3-dioxolan-2-yl)-1-benzofuran (6.6g 24.5 mmol) is dissolved in Et₂O (100 ml) in a dry flask under nitrogen and cooled to -78°C. *tert*-Butylithium (31.7 ml, 53.9 mmol) is added dropwise, and the chilled solution is stirred 1 h. DMF (2.18 ml, 28.2 mmol) is dissolved in Et₂O (25 ml) and also added dropwise, and the mixture is stirred at -78°C for another 7 h. The reaction is warmed to RT, and then oxalic acid dihydrate (6.18 g, 49.0 mmol) and water (25 ml) are added. The reaction continues stirring at RT overnight and is then diluted with water (125 ml) and EtOAc (175 ml). The layers are allowed to separate, and the aqueous is extracted with EtOAc (1 x 100 ml). The organics are combined, dried over Na₂SO₄, filtered, and concentrated to a brown oil. The crude material is chromatographed over 350 g slurry-packed silica, eluting with 30% EtOAc/hexane. The appropriate fractions are combined and concentrated to afford 3.84 g (72%) of 2-(1,3-dioxolan-2-yl)-1-benzofuran-3-carbaldehyde as a yellow/orange oil. MS (EI) *m*/*z:* 218 (M⁺).

2-(1,3-Dioxolan-2-yl)-1-benzofuran-3-carbaldehyde (3.63 g, 16.6 mmol) is dissolved in formic acid (16.3 ml, 433 mmol) with water (4.1 ml). After 2 h, additional formic acid (10 ml) and water (2.5 ml) are added to alleviate the slurry. The reaction is stirred 12 h and is diluted with water (30 ml). The resulting slurry is filtered, dried in an air stream, affording 2.66 g (92%) of 1-benzofuran-2,3-dicarbaldehyde as an orange solid. MS (EI) *m*/*z*: 174 (M⁺).

1-Benzofuran-2,3-dicarbaldehyde (174 mg, 1.0 mmol) is dissolved in CH₂Cl₂ (5 ml) and chilled to 0°C. Methyl (acetylamino)(dimethoxyphosphoryl) acetate (263 mg, 1.1 mmol) is dissolved in CH₂Cl₂ (5 ml) and combined with DBU (0.16 ml, 1.1 mmol), stirring for 5 min. This solution is added dropwise to the chilled benzofuran solution. The reaction mixture is stirred 0°C for 1 h, 4 days at RT, and 2 days at 45°C. The volatiles are removed *in vacuo* and the crude material is chromatographed over 50 g slurry-packed silica eluting with 40% EtOAc/hexane. The appropriate fractions are combined and concentrated to give 180 mg (79%) of methyl benzofuro[3,2-c]pyridine-3-carboxylate as a yellow solid. HRMS (FAB) calcd for C₁₃H₉NO₃+H: 228.0661, found 228.0654 (M+H)⁺.

Methyl benzofuro[3,2-c]pyridine-3-carboxylate (2.02 g, 8.89 mmol) is dissolved in MeOH (50 ml) and water (10 ml). 2M NaOH (5.3 ml, 10.67 mmol) is added dropwise, and the reaction is stirred overnight at RT. When the reaction is complete by TLC, the volatiles are removed *in vacuo.* The solid residue is suspended in water (40 ml) and the pH is adjusted to 3 with concentrated HCl. The white slurry is filtered, and the cake is dried first in a stream of air and then in a vacuum oven overnight, affording 1.84 g (97%) of benzofuro[3,2-c]pyridine-3-carboxylic acid as a pale yellow solid. ¹H NMR (DMSO-*d₆*) δ 7.56, 7.68, 7.88, 8.38, 9.51 ppm.

Benzofuro[3,2-c]pyridine-3-carboxylic acid (213 mg, 1.0 mmol) is coupled with (2R)-7-aza-[2.2.1]-Amine (233 mg, 1.1 mmol) and deprotected as described in Example 10 with non-critical variations (elution of the carbamate with 40% EtOAc/hexane) to provide 297 mg (78%) of Example 27 as a white solid. HRMS (FAB) calcd for C₁₈H₁₇N₃O₂+H: 308.1399, found 308.1401 (M+H)⁺.

### Example 28: N-[(1S, 2R, 4R)-7-azabicyclo[2.2.1]hept-2-yl]benzofuro[2,3-c]pyridine-3-carboxamide dihydrochloride:

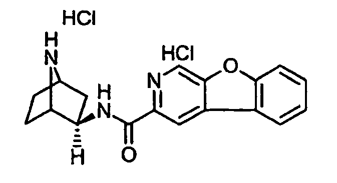

2-(1,3-Dioxolan-2-yl)-1-benzofuran-3-carbaldehyde (1.4 g, 6.4 mmol) is dissolved in CH₂Cl₂ (50 ml) and chilled to 0°C. Methyl (acetylamino)(dimethoxyphosphoryl) acetate (1.69 g, 7.06 mmol) is dissolved in CH₂Cl₂ (20 ml) and combined with DBU (1.05 ml, 7.06 mmol), stirring for 5 min. This solution is added dropwise to 1-benzofuran-2,3-dicarbaldehyde. The reaction mixture is stirred 2 days, allowing the ice bath to expire. The volatiles are removed in *vacuo* and the crude material is chromatographed over 100 g slurry-packed silica eluting with 50% EtOAc/hexane. The appropriate fractions are combined and concentrated, yielding 1.97 g (93%) of methyl (2Z)-2-(acetylamino)-3-[2-(1,3-dioxolan-2-yl)-1-benzofuran-3-yl] prop-2-enoate as a yellow oil. ¹H NMR (CDCl₃) δ 1.99, 3.91, 4.13, 4.26, 6.10, 7.29, 7.35, 7.49 ppm.

Methyl (2Z)-2-(acetylamino)-3-[2-(1,3-dioxolan-2-yl)-1-benzofuran-3-yl] prop-2-enoate (1.97 g, 5.94 mmol) is dissolved in formic acid (6 ml, 160 mmol) with water (2 ml). The reaction stirred 2 days at RT. The reaction is diluted with water (40 ml), and the resulting slurry is filtered and dried in an air stream to afford 1.02 g (75%) of methyl benzofuro[2,3-c]pyridine-3-carboxylate as tan solid. ¹H NMR (CDCl₃) δ 4.09, 7.51, 7.71, 8.12, 8.82, 9.19 ppm.

Methyl benzofuro[2,3-c]pyridine-3-carboxylate (681 mg, 3.0 mmol) is dissolved in MeOH (15 ml) and water (3.5 ml). 2M Sodium hydroxide (1.8ml, 3.6 mmol) is added dropwise, and the reaction is stirred overnight at RT. When the reaction is complete by TLC, the volatiles are removed *in vacuo.* The solid residue is suspended in water (25 ml) and the pH is adjusted to 3 with concentrated HCl. The solution is stirred overnight, and the resulting white slurry is filtered. The cake is dried first in a stream of air and then in a vacuum oven overnight, affording 583 mg (91%) of benzofuro[2,3-c]pyridine-3-carboxylic acid as an ivory solid. HRMS (FAB) calcd for C₁₂H₇NO₃+H: 214.0504, found 214.0493 (M+H)⁺.

Benzofuro[2,3-c]pyridine-3-carboxylic acid (213 mg, 1.0 mmol) is coupled with (2R)-7-aza-[2.2.1]-Amine (233 mg, 1.1 mmol) and deprotected as described in Example 10 with non-critical variations (elution of the carbamate with 50% EtOAc/hexane) to provide 293 mg (77%) of Example 28 as an ivory solid. HRMS (FAB) calcd for C₁₈H₁₇N₃O₂+H 308.1399, found 308.1389 (M+H)⁺.

### Example 29: N-[(1S, 2R, 4R)-7-azabicyclo[2.2.1]hept-2-yl]-3-isopropyl-1-benzofuran-5-carboxamide hydrochloride:

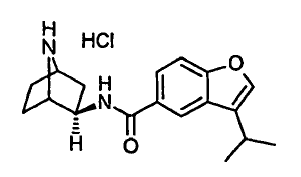

Methyl 4-hydroxy-3-iodobenzoate (6.0 g, 21.5 mmol) is dissolved in DMF (35 ml) in a dry flask under nitrogen and cooled to 0°C. 60% Sodium hydride (860 mg, 21.5 mmol) is added portionwise and the reaction is stirred 1 h, allowing the ice bath to expire. The mixture is then treated with 1-chloro-3-methyl-2-butene (2.67 ml, 23.7 mmol) and sodium iodide (323 mg, 2.15 mmol), and the reaction stirred 18 h at RT. The mixture is diluted with EtOAc (150 ml) and washed with 1:1 saturated NaCl/NaHCO₃ (1 x 100 ml). The organic layer is dried with MgSO₄ and concentrated to an oil. The crude material is chromatographed over 700 g slurry-packed silica gel, eluting with 15% EtOAc/hexane. The appropriate fractions are collected and concentrated to afford 5.13 g of a pale oil. The oil is then dissolved in DMF (40 ml) and treated successively with palladium acetate (165 mg, 0.74 mmol), sodium carbonate (3.9 g, 36.8 mmol), sodium formate (1.0 g, 14.7 mmol), and N-butyl tetraammonium chloride (4.5 g, 16.2 mmol). The mixture stirred 2 days at 80°C. The reaction is quenched onto EtOAc (200 ml) and washed with 50% saturated brine (3 x 75 ml) and 5% HCl (1 x 75 ml). The organic is dried over MgSO₄, filtered, and concentrated to a brown oil. The crude material is chromatographed over 250 g slurry-packed silica gel, eluting with 10% EtOAc/hexane. The appropriate fractions are collected and concentrated to afford 1.33 g (28% over 2 steps) of methyl 3-isopropyl-1-benzofuran-5-carboxylate as a mobile oil. HRMS (FAB) calcd for C₁₃H₁₄O₃+H: 219.1021, found 219.1021 (M+H)⁺.

Methyl 3-isopropyl-1-benzofuran-5-carboxylate 1.20 g, 5.51 mmol) is dissolved in MeOH (20 ml) and water (4 ml). 2N NaOH (3.3 ml, 6.6 mmol) is added dropwise and the reaction is stirred 2 days. Slight heating at 40°C is required for 4 h. Volatiles are removed *in vacuo,* and the residue is dissolved in water (10 ml). Concentrated HCl is used to adjust the pH to 3, and the resulting precipitate is isolated via filtration and dried overnight to afford 1.08 g (97%) of 3-isopropyl-1-benzofuran-5-carboxylic acid as a white solid. MS (ESI-) for C₁₂H₁₂O₃ *m*/*z*: 203.0 (M-H)⁻.

3-Isopropyl-1-benzofuran-5-carboxylic acid (204 mg, 1.0 mmol) is coupled with (2R)-7-aza-[2.2.1]-Amine (233 mg, 1.1 mmol) and deprotected as described in Example 10 with non-critical variations (elution of the carbamate with 40% EtOAc/hexane) to provide 245 mg (73%) of Example 29 as a white solid. HRMS (FAB) calcd for C₁₈H₂₂N₂O₂+H: 299.1759, found 299.1754 (M+H)⁺.

### Example 30: N-[(1S, 2R, 4R)-7-azabicyclo[2.2.1]hept-2-yl]-2-methyl-1-benzofuran-5-carboxamide hydrochloride:

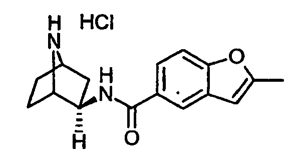

2-Methyl-1-benzofuran-5-carboxylic acid is obtained from methyl 4-hydroxy-3-iodobenzoate according to the procedures discussed in Example 25, making non-critical changes (eluting the intermediate ester with 15% EtOAc/hexane) in 80% overall yield as a gray solid. ¹H NMR (DMSO-*d₆*) δ 2.47, 6.70, 7.57, 7.85, 8.16, 12.81 ppm.

2-Methyl-1-benzofuran-5-carboxylic acid (176 mg, 1.0 mmol) is coupled with (2R)-7-aza-[2.2.1]-Amine (233 mg, 1.1 mmol) and deprotected as described in Example 10 with non-critical variations (elution of the carbamate with 40% EtOAc/hexane) to provide 244 mg (80%) of Example 30 as a light tan solid. HRMS (FAB) calcd for C₁₆H₁₈N₂O₂+H 271.1446, found 271.1446 (M+H)⁺.

### Example 31: N-[(1S, 2R, 4R)-7-azabicyclo[2.2.1]hept-2-yl]-3-bromo-1-benzofuran-5-carboxamide hydrochloride:

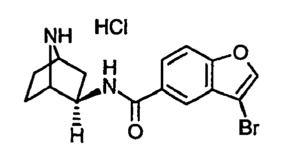

Methyl benzofuran-5-carboxylate (667 mg, 3.8 mmol) (described in Example 14) is dissolved in 20 ml CH₂Cl₂ under nitrogen. The solution is treated with bromine (1.2 ml, 22.8 mmol), is layered with 20 ml saturated NaHCO₃, and the reaction is stirred gently for 2 h at RT. The reaction is stirred vigorously for 30 min, the layers are separated, and the organic layer is concentrated *in vacuo* to an amber oil. The residue is dissolved in 30 ml EtOH, the solution is treated with anhydrous potassium carbonate (3.15 g, 22.8 mmol), and the reaction is stirred vigorously overnight. The insoluble material is removed by filtration and the filtrate is diluted with 3 ml 3N NaOH and the mixture is stirred 3 h at RT. The mixture is concentrated *in vacuo,* the residue is dissolved in 10 ml water, and the pH of the solution is adjusted to 2 with 10% aqueous HCl. The precipitate is collected, washed with water, and is dried to afford 880 mg (96%) of 3-bromobenzofuran-5-carboxylic acid as an off-white solid. HRMS (FAB) calcd for C₉H₅BrO₃ +H: 240.9501, found 240.9505 (M+H)⁺.

3-Bromobenzofuran-5-carboxylic acid (241 mg, 1.0 mmol) is coupled with (2R)-7-aza-[2.2.1]-Amine (233 mg, 1.1 mmol) and deprotected as described in Example 10 with non-critical variations (elution of the carbamate with 32% EtOAc/hexane) to provide 310 mg (83%) of Example 31 as a white solid. HRMS (FAB) calcd for C₁₅H₁₅BrN₂O₂ +H: 335.0396, found 335.0379 (M+H)⁺.

### Example 32: N-[(1S, 2R, 4R)-7-azabicyclo[2.2.1]hept-2-yl]-3-ethynyl-l-benzofuran-5-carboxamide fumarate:

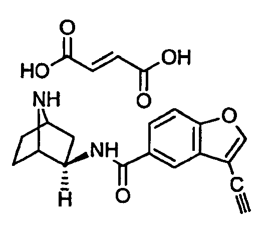

3-Bromobenzofuran-5-carboxylic acid (1.25 g, 5.2 mmol) is dissolved in DMF (10 ml) with DIEA (3.6 ml, 20.6 mmol) and (2R)-7-aza-[2.2.1]-Amine (1.1 g, 5.2 mmol). HATU (1.97 g, 5.2 mmol) is added portion-wise and the reaction is stirred overnight at RT. Volatiles are removed *in vacuo,* and the residue is dissolved in 50 ml CHCl₃, is washed with 1 x 50 ml 1:1 sat'd NaCl/conc. NH₄OH, and the aqueous layer is washed with 50 ml CHCl₃. The combined organic layer is dried over K₂CO₃ and is concentrated *in vacuo.* The residue is chromatographed over 50 g silica gel (230-400 mesh), eluting with 32% EtOAc/hexane. The appropriate fractions are collected and concentrated to give 1.64 g (73%) of *tert*-butyl (1S, 2R, 4R)-2-{[(3-bromo-1-benzofuran-5-yl)carbonyl]amino}-7-azabicyclo[2.2.1]heptane-7-carboxylate as a pale oil.

*tert*-Butyl (1S, 2R, 4R)-2-{[(3-bromo-1-benzofuran-5-yl)carbonyl]amino}-7-azabicyclo[2.2.1]heptane-7-carboxylate (436 mg, 1.0 mmol) is combined with bis(benzonitrile)palladium dichloride (57 mg, 0.15 mmol), cuprous iodide (19 mg, 0.10 mmol), tri-*t*-butylphosphine ((10% in hexane) 658 µL, 0.325 mmol), and trimethylsilyl acetylene (170 µL, 1.2 mmol) in 3 ml dry dioxane in a dry flask under nitrogen. The mixture is treated with diisopropylamine (168 µL, 1.2 mmol) is stirred overnight at RT, and is diluted with 25 ml EtOAc. The reaction is washed with 4 x 25 ml 1:1:0.1 H₂O/brine/conc NH₄OH, the organic layer is dried over K₂CO₃, and the volatiles are removed *in vacuo.* The crude material is chromatographed over 30 g silica gel (230-400 mesh) eluting with 25% EtOAc/hexane. The appropriate fractions are combined and concentrated to give 385 mg (81%) of N-[(1S, 2R, 4R)-7-azabicyclo[2.2.1]hept-2-yl]-3-[(trimethylsilyl)ethynyl]-1-benzofuran-5-carboxamide as an orange foam. HRMS (FAB) calcd for C₂₅H₃₂N₂O₄Si +H: 453.2209, found 453.2227 (M+H)⁺.

N-[(1S, 2R, 4R)-7-azabicyclo[2.2.1]hept-2-yl]-3-[(trimethylsilyl)ethynyl]-1-benzofuran-5-carboxamide is dissolved in 5 ml CH₃OH, and the solution is treated with 5 ml 3N methanolic HCl. The reaction is warmed to 50°C for 2 h, is carefully quenched with 9 ml saturated NaHCO₃, and the reaction is stirred overnight at RT and then at 50°C for an additional 2 h. The volatiles are removed *in vacuo* and the residue is partitioned between 10 ml H₂O and 4 x 10 ml CHCl₃. The combined organic layer is dried over K₂CO₃ and is concentrated *in vacuo* to a dark oil. The crude material is chromatographed over 30 g silica gel (230-400 mesh) eluting with 8% CH₃OH/CHCl₃ +1% NH₄OH. The appropriate fractions are combined and concentrated to afford 166 mg of a pale oil. The oil is combined with fumaric acid (69 mg, 0.59 mmol) in 1 ml CH₃OH and the solution is treated dropwise with 15 ml EtOAc. The mixture is stirred 1 h, the solid is collected, washed with EtOAc/Et₂O and is dried to give 208 mg (69%) of Example 32 as a white solid. HRMS (FAB) calcd for C₁₇H₁₆N₂O₂ +H: 281.1290, found 281.1279 (M+H)⁺.

### Example 33: N-[(1S, 2R, 4R)-7-azabicyclo[2.2.1]hept-2-yl]-3-prop-1-ynyl-1-benzofuran-5-carboxamide fumarate:

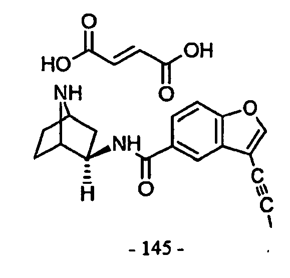

Methyl-3-bromobenzofuran-5-carboxylate (750 mg, 2.94 mmol) is combined with bis(benzonitrile)palladium dichloride (169 mg, 0.44 mmol), cuprous iodide (19 mg, 0.10 mmol), tri-*t*-butylphosphine ((10% in hexane) 1.92 ml, 0.95 mmol), and propyne (2 ml, 35 mmol) in 7 ml dry dioxane in 15 ml screw cap pressure tube under nitrogen. The mixture is treated with diisopropylamine (494 µL, 3.6 mmol), is stirred overnight at RT, and is diluted with 50 ml EtOAc. The reaction is washed with 4 x 25 ml 1:1:0.1 H₂O/brine/conc NH₄OH, the organic layer is dried over MgSO₄, and the volatiles are removed *in vacuo.* The crude material is chromatographed over 50 g silica gel (230-400 mesh) eluting with 12% EtOAc/hexane. The appropriate fractions are combined and concentrated to give 460 mg (73%) of methyl 3-prop-1-ynyl-1-benzofuran-5-carboxylate as a pale solid. MS (EI) *m*/*z*): 214 (M)⁺.

Methyl 3-prop-1-ynyl-1-benzofuran-5-carboxylate (388 mg, 1.81 mmol) is dissolved in 10 ml CH₃OH. The solution is treated with 2N NaOH (2.3 ml, 4.6 mmol) followed by 2 ml H₂O and the reaction is stirred overnight at RT. The reaction is concentrated to dryness, the residue is dissolved in 9 ml H₂O, and the pH is adjusted to 3 with 10% aqueous HCl. The precipitate is collected, washed with H₂O, and is dried to afford 356 mg (98%) of 3-prop-1-ynyl-1-benzofuran-5-carboxylic acid as an off-white solid. HRMS (EI) calcd for C₁₂H₈O₃: 200.0473, found 200.0476 (M⁺).

3-Prop-1-ynyl-1-benzofuran-5-carboxylic acid (208 mg, 1.1 mmol) is coupled with (2R)-7-aza-[2.2.1]-Amine (257 mg, 1.2 mmol) and deprotected as described in Example 10 with non-critical variations (elution of the carbamate with 30% EtOAc/hexane) and making the salt as described in Example 32 to provide 330 mg (80%) of Example 33 as a white solid. HRMS (FAB) calcd for C₁₈H₁₈N₂O₂ +H: 295.1446, found 295.1439 (M+H)⁺.

### Example 34: N-[(1S, 2R, 4R)-7-azabicyclo[2.2.1]hept-2-yl]-3-bromothieno[2,3-c]pyridine-5-carboxamide dihydrochloride:

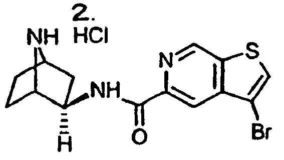

Methyl-thieno[2,3-c]pyridine-5-carboxylate (630 mg, 3.3 mmol) is dissolved in 20 ml CH₂Cl₂. The solution is treated with Br₂ (1.1 ml, 20 mmol), is layered with 20 ml saturated NaHCO₃, and the two-phase mixture is agitated gently for 2 h. The reaction is stirred vigorously for 30 min, the layers are separated, and the organic layer is dried over K₂CO₃. The organic layer is concentrated to a dark tan solid. The solid is dissolved in 20 ml 10% MeOH/CH₂Cl₂, is adsorbed onto 2 g silica gel (230-400 mesh), and the plug is chromatographed over 25 g silica gel (230-400 mesh) eluting with 65% EtOAc/hexane. The appropriate fractions are combined and concentrated to afford 635 mg (71%) of methyl-3-bromothieno[2,3-c]pyridine-5-carboxylate as a tan solid. ¹H NMR (CDCl₃) δ 4.09 (s, 3 H), 7.82 (s, 1 H), 8.59 (m, 1 H), 9.25 (s, 1 H) ppm.

Methyl-3-bromothieno[2,3-c]pyridine-5-carboxylate (635 mg, 2.33 mmol) is combined with 25 ml MeOH. The mixture is treated with 2N NaOH (3 ml, 6 mmol) and 3 ml H₂O and the reaction is stirred 4 h at RT. The volatiles are removed *in vacuo* and the residue is combined with 5 ml H₂O. The pH of the mixture is adjusted to 3.5 with 10% aqueous HCl. The tan precipitate is collected, washed with water, and is dried *in vacuo* at 50°C to afford 475 mg (79%) of 3-bromothieno[2,3-c]pyridine-5-carboxylic acid as a tan solid. MS (ESI): 257.9.

3-Bromo-thieno[2,3-c]pyridine-5-carboxylic acid (237 mg, 0.92 mmol) is coupled with (2R)-7-aza-[2.2.1]-Amine (212 mg, 1.0 mmol) and deprotected as described in Example 10 with non-critical variations (elution of the carbamate with 35% EtOAc/hexane) to provide 330 mg (84%) of Example 34 as a white solid. MS (EI) *m*/*z*): 365 (M)⁺.

### Example 35: N-[(1S, 2R, 4R)-7-azabicyclo[2.2.1]hept-2-yl]-1H-indole-2-carboxamide hydrochloride:

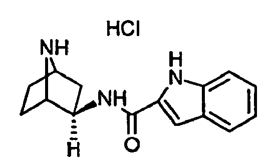

Indole-2-carboxylic acid (133 mg, 0.83 mmol) is coupled with (2R)-7-aza-[2.2.1]-Amine (160 mg, 0.75 mmol) and deprotected as described in Example 1 with non-critical variations to provide 140 mg (50%) of Example 35 as a pale yellow solid. HRMS (FAB) calcd for C₁₅H₁₇N₃O +H: 256.1450, found 256.1450 (M+H)⁺.

### Example 36: N-[(1S,2R,4R)-7-azabicyclo[2.2.1]hept-2-yl]-1-benzothiophene-2-carboxamide hydrochloride:

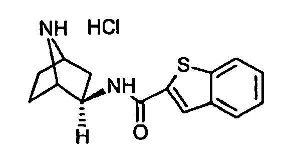

Benzo[b]thiophene-2-carboxylic acid (147 mg, 0.83 mmol) is is coupled with (2R)-7-aza-[2.2.1]-Amine (160 mg, 0.75 mmol) and deprotected as described in Example 10 with non-critical variations (elution of the carbamate with 25% EtOAc/hexane) to provide 125 mg (32%) of Example 36 as a white solid. HRMS (FAB) calcd for C₁₅H₁₆N₂OS +H: 273.1061, found 273.1058 (M+H)⁺.

### Example 37: N-[(1S, 2R, 4R)-7-Azabicyclo[2.2.1]hept-2-yl]-3-methyl-1-benzofuran-5-carboxamide hydrochloride:

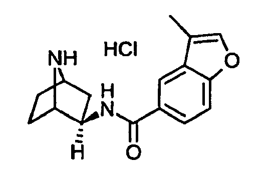

Methyl 4-hydroxy-3-iodobenzoate (1.85 g, 6.65 mmol) is dissolved in anhydrous DMF (15 mL) in a dry flask under nitrogen, treated with sodium hydride (60% dispersion in oil, 265 mg, 6.65 mmol) and stirred for 1 h at RT. Allyl bromide (633 µL, 7.32 mmol) is added and the mixture is stirred for 16 h. The mixture is concentrated and the residue is partitioned between EtOAc (25 mL) and water (25 mL). The organic layer is washed with a 50% saturated mixture of 1:1 NaCl / NaHCO₃ (2 x 10 mL), dried over magnesium sulfate and concentrated to a yellow oil which solidified to a white solid upon standing (2.12 g). The crude material is chromatographed over 100 g slurry-packed silica gel, eluting with 10% EtOAc/hexane. The appropriate fractions are combined and concentrated to afford 1.94 g (90%) of methyl 4-(allyloxy)-3-iodobenzoate. MS (EI) *m*/*z:* 318 (M)⁺.

Methyl 4-(allyloxy)-3-iodobenzoate (587 mg, 1.84 mmol) is combined with palladium acetate (5%, 20 mg, 0.1 mmol), Na₂CO₃ (487 mg, 4.6 mmol), sodium formate (125 mg, 1.8 mmol) and (nBu)₄N⁺Cl⁻ (561 mg, 2.0 mmol) in DMF (5 mL) and heated to 80°C for 2 d. The mixture is concentrated under high vacuum and partitioned between 50% brine (10 mL) and CH₂Cl₂ (4x10 mL). The combined organics are washed with 5% HCl (10 mL), dried over Na₂SO₄ and concentrated to a brown oil. The crude material is chromatographed over 15 g slurry-packed silica gel, eluting with 15% EtOAc/hexane. Tha appropriate fractions are combined and concentrated to afford 153 mg (44%) of methyl 3-methyl-l-benzofuran-5-carboxylate as a white solid. HRMS (FAB) calcd for C₁₁H₁₀O₃ +H: 191.0708, found: 191.0705 (M+H)⁺.

Methyl 3-methyl-1-benzofuran-5-carboxylate (365 mg, 1.9 mmol) is dissolved in MeOH (7 mL), diluted with water (3.5 mL) and treated with 3N NaOH (1.41 mL, 4.2 mmol). The mixture is diluted with MeOH (3.5 mL) to homogeneity, stirred at RT for 2.5 days then concentrated to dryness. The residue is dissolved in water (5 mL) and acidified to pH 2 with concentrated HCl. The resulting solid is filtered and dried in a vacuum oven at 40°C for 18 h to afford 321 mg (95%) of 3-methyl-1-benzofuran-5-carboxylic acid as a white solid. HRMS (FAB) calcd for C₁₀H₈O₃ +H: 177.0552, found: 177.0553 (M+H)⁺.

3-Methyl-1-benzofuran-5-carboxylic acid (156 mg, 0.9 mmol) is coupled with (2R)-7-aza-[2.2.1]-Amine (212 mg, 1.0 mmol) and deprotected as described in Example 10 with non-critical variations (elution of the carbamate with 45% EtOAc/hexane) to provide 213 mg (79%) of Example 37 as a white solid. HRMS (FAB) calcd for C₁₆H₁₈N₂O₂ +H: 271.1446, found: 271.1452 (M+H)⁺.

### Materials and Methods for Determining α7 nAChR Agonist Activity

### Cell-based Assay for Measuring the EC₅₀ of α7 nAChR Agonists

### Construction and expression of the α7-5HT₃ receptor:

The cDNA encoding the N-terminal 201 amino acids from the human α7 nAChR that contain the ligand binding domain of the ion channel was fused to the cDNA encoding the pore forming region of the mouse 5HT₃ receptor as described by Eisele JL, et al., Chimaeric nicotinic-serotonergic receptor combines distinct ligand binding and channel specificities, Nature (1993), Dec. 2;366(6454):479-83, and modified by Groppi, et al., WO 00/73431. The chimeric α7-5HT₃ ion channel was inserted into pGS 175 and pGS 179 which contain the resistance genes for G-418 and hygromycin B, respectively. Both plasmids were simultaneously transfected into SH-EP1 cells and cell lines were selected that were resistant to both G-418 and hyrgromycin B. Cell lines expressing the chimeric ion channel were identified by their ability to bind fluorescent α-bungarotoxin on their cell surface. The cells with the highest amount of fluorescent α-bungarotoxin binding were isolated using a Fluorescent Activated Cell Sorter (FACS). Cell lines that stably expressed the chimeric α7-5HT₃ were identified by measuring fluorescent α-bungarotoxin binding after growing the cells in minimal essential medium containing nonessential amino acids supplemented with 10% fetal bovine serum, L-glutamine, 100 units/ml penicillin/streptomycin, 250 ng/mg fungizone, 400 µg/ml hygromycin B, and 400 µg/ml G-418 at 37° C with 6% CO₂ in a standard mammalian cell incubator for at least 4 weeks in continuous culture.

### Assay of the activity of the chimeric α7-5HT₃ receptor

To assay the activity of the α7-5HT₃ ion channel, cells expressing the channel were plated into each well of either a 96 or 384 well dish (Corning #3614) and grown to confluence prior to assay. On the day of the assay, the cells were loaded with a 1:1 mixture of 2 mM Calcium Green 1, AM (Molecular Probes) dissolved in anhydrous DMSO and 20% pluronic F-127 (Molecular Probes). This solution was added directly to the growth media of each well to achieve a final concentration 2 µM. The cells were incubated with the dye for 60 min at 37° C and then washed with a modified version of Earle's balanced salt solution (MMEBSS) as described in WO 00/73431. The ion conditions of the MMEBSS was adjusted to maximize the flux of calcium ion through the chimeric α7-5HT₃ ion channel as described in WO 00/73431. The activity of compounds on the chimeric α7-5HT₃ ion channel was analyzed on FLIPR. The instrument was set up with an excitation wavelength of 488 nanometers using 500 milliwatts of power. Fluorescent emission was measured above 525 nanometers with an appropriate F-stop to maintain a maximal signal to noise ratio. Agonist activity of each compound was measured by directly adding the compound to cells expressing the chimeric α7-5HT₃ ion channel and measuring the resulting increase in intracellular calcium that is caused by the agonist-induced activation of the chimeric ion channel. The assay is quantitative such that concentration-dependent increase in intracelluar calcium is measured as concentration-dependent change in Calcium Green fluorescence. The effective concentration needed for a compound to cause a 50% maximal increase in intracellular calcium is termed the EC₅₀. The examples of the present invention have EC₅₀ values ranging from 45-18,000 nM.

### Binding Constants:

Another way for measuring α7 nAChR agonist activity is to determine binding constants of a potential agonist in a competition binding assay. For α7 nAChR agonists, there is good correlation between functional EC₅₀ values using the chimeric α7-5HT₃ ion channel as a drug target and binding affinity of compounds to the endogenous α7 nAChR.

### Membrane Preparation.

Male Sprague-Dawley rats (300-350g) are sacrificed by decapitation and the brains (whole brain minus cerebellum) are dissected quickly, weighed and homogenized in 9 volumes/g wet weight of ice-cold 0.32 M sucrose using a rotating pestle on setting 50 (10 up and down strokes). The homogenate is centrifuged at 1,000 x g for 10 minutes at 4 °C. The supernatant is collected and centrifuged at 20,000 x g for 20 minutes at 4 °C. The resulting pellet is resuspended to a protein concentration of 1 - 8 mg/mL. Aliquots of 5 mL homogenate are frozen at -80 °C until needed for the assay. On the day of the assay, aliquots are thawed at rt and diluted with Kreb's - 20 mM Hepes buffer pH 7.0 (at rt) containing 4.16 mM NaHCO₃, 0.44 mM KH₂PO₄, 127 mM NaCl, 5.36 mM KCl, 1.26 mM CaCl₂, and 0.98 mM MgCl₂, so that 25 - 150 µg protein are added per test tube. Proteins are determined by the Bradford method (Bradford, M.M., *Anal. Biochem.,* 72, 248-254, 1976) using bovine serum albumin as the standard.

### Binding Assay.

For saturation studies, 0.4 mL homogenate are added to test tubes containing buffer and various concentrations of radioligand, and are incubated in a final volume of 0.5 mL for 1 hour at 25 °C. Nonspecific binding was determined in tissues incubated in parallel in the presence of 0.05 mls MLA for a final concentration of 1 µM, added before the radioligand. In competition studies, drugs are added in increasing concentrations to the test tubes before addition of 0.05 mls [³H]-MLA for a final concentration 3.0 to 4.0 nM. The incubations are terminated by rapid vacuum filtration through Whatman GF/B glass filter paper mounted on a 48 well Brandel cell harvester. Filters are pre-soaked in 50 mM Tris HCl pH 7.0 - 0.05 % polyethylenimine. The filters are rapidly washed two times with 5 mL aliquots of cold 0.9% saline and then counted for radioactivity by liquid scintillation spectrometry.

### Data Analysis.

In competition binding studies, the inhibition constant (Ki) was calculated from the concentration dependent inhibition of [³H]-MLA binding obtained from non-linear regression fitting program according to the Cheng-Prusoff equation (Cheng, Y.C. and Prussoff, W.H., *Biochem. Pharmacol.,* 22, p. 3099-3108, 1973). Hill coefficients were obtained using non-linear regression (GraphPad Prism sigmoidal dose-response with variable slope).

## Claims

1. A compound of Formula I: 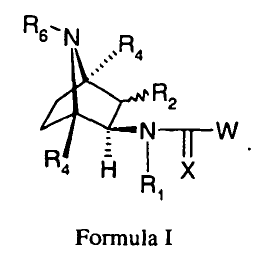
wherein the stereochemistry of the of the 7-azabicyclo[2.2.1]heptane ring is 1*S,* 4*R* and the nitrogen substituent at the C-2 carbon has the *exo* orientation and is 2*R*;
X is O;
W is 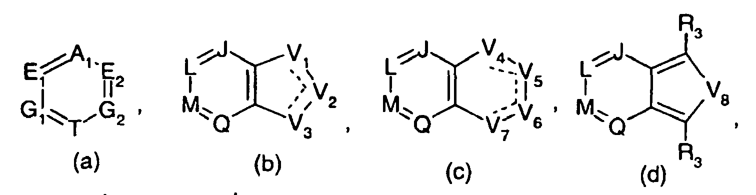 or 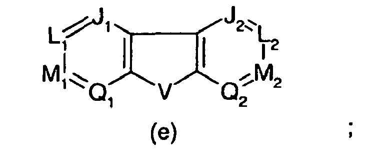
A₁ is N;
Each E₁, E₂, G₁, G₂, and T is independently selected from CR₃ and N, provided that no more than two of E₁, E₂, G₁, G₂, and T are N, and further provided that when E₁ is N, G₁ and E₂ must be CR₃, and further provided that when E₂ is N, E₁ and G₂ must be CR₃;
Each V and V₈ is independently selected from O, S, NA₅, and C(R₃)₂, provided that when V₈ is C(R₃)₂ the R₃ substitutents on the carbon atoms adjacent to V₈ are moieties other than H;
Each V₁, V₂, V₃, V₄, V₅, V₆, and V₇ is independently selected from O, S, N, C(R₃), C(R₃)₂, or NA₅, provided that V₁ and V₂, V₂ and V₃, V₄ and V₅, V₅ and V₆, and V₆ and V₇ are not simultaneously O or S, or combinations of O and S, and further provided that at least one of V₁, V₂, and V₃, at least one of V₄, V₅, and V₆, and at least one of V₅, V₆, and V₇ is C(R₃) or C(R₃)₂;
Each J, J₁, J₂, L, L₁, L₂, M, M₁, M₂, Q, Q₁, and Q₂ is independently selected from CR₃ and N, provided that no more than two of J, L, M, and Q, or no more than two of J₁, L₁, M₁, and Q₁, or no more than two of J₂, L₂, M₂, and Q₂ are N, and provided that at least one of J, L, M, and Q is N when V₁, V₂, and V₃ are independently selected from C(R₃)₂ and C(R₃), when V₄, V₅, V₆, and V₇ are independently selected from C(R₃)₂ and C(R₃), and when V₈ is C(R₃)₂, and further provided that at least one of J₁, L₁, M₁, and Q₁ or at least one of J₂, L₂, M₂, and Q₂ is N when V is C(R₃)₂;
Each A₅ is independently H, alkyl having from 1-6 carbon atoms, alkenyl having from 2-6 carbon atoms, alkynyl having from 2-6 carbon atoms, cycloalkyl having from 3-6 carbon atoms, heterocycloalkyl having 4-7 atoms with 1-2 atoms within the ring being -S-, -N(R₁₈)-, or -O-, halogenated alkyl having from 1-6 carbon atoms, halogenated alkenyl having from 2-6 carbon atoms, halogenated alkynyl having from 2-6 carbon atoms, halogenated cycloalkyl having from 3-6 carbon atoms, or halogenated heterocycloalkyl having 4-7 atoms with 1-2 atoms within the ring being -S-, -N(R₁₈)-, or -O-;
R₁ is H, alkyl having from 1-6 carbon atoms, or halogenated alkyl having from 1-6 carbon atoms;
R₂ is H, alkyl having from 1-6 carbon atoms, or halogenated alkyl having from 1-6 carbon atoms;
Each R₃ is independently H, F, Cl, Br, I, -CN, -NO₂, alkyl having from 1-6 carbon atoms, alkenyl having from 2-6 carbon atoms, alkynyl having from 2-6 carbon atoms, cycloalkyl having from 3-6 carbon atoms, heterocycloalkyl having 4-7 atoms with 1-2 atoms within the ring being -S-, -N(R₁₈)-, or -O-, halogenated alkyl having from 1-6 carbon atoms, halogenated alkenyl having from 2-6 carbon atoms, halogenated alkynyl having from 2-6 carbon atoms, halogenated cycloalkyl having from 3-6 carbon atoms, or halogenated heterocycloalkyl having 4-7 atoms with 1-2 atoms within the ring being -S-, -N(R₁₈)-, or -0-; or
R₃ forms a bond to the carbon of the amide group bound to the C-2 carbon of the 7-azabicyclo[2.2.1]heptane ring
Each R₄ is independently H, or alkyl having from 1-6 carbon atoms;
R₆ is H, alkyl having from 1-6 carbon atoms, or an alkyl group having from 1-6 carbon atoms and having 1-3 substituents selected from F, Cl, Br, I, -OH, -CN, - NH₂, -NH(alkyl having from 1-6 carbon atoms), and -N(alkyl having from 1-6 carbon atoms)₂;
R₁₈ is H, alkyl having from 1-6 carbon atoms, halogenated alkyl having from 1-6 carbon atoms, cycloalkyl having from 3-6 carbon atoms, or halogenated cycloalkyl having from 3-6 carbon atoms;
and pharmaceutically acceptable salt thereof.

2. The compound of claim 1, wherein R₁ is H or alkyl having from 1-6 carbon atoms, and wherein R₂ is H, alkyl having from 1-6 carbon atoms, or halogenated alkyl having from 1-6 carbon atoms.

3. The compound of claim 2, wherein W is (b).

4. The compound of claim 2, wherein W is (c).

5. The compound of claim 2, wherein W is (d).

6. The compound of claim 2, wherein W is (e).

7. The compound of claim1 selected from:
N-[(1S,2R,4R)-7-azabicyclo[2.2.1]hept-2-yl]-3-methylfuro[2,3-c]pyridine-5-carboxamide;
N-[(1S,2R,4R)-7-azabicyclo[2.2.1]hept-2-yl]thieno[2,3-c]pyridine-5-carboxamide;
N-[(1S,2R,4R)-7-azabicyclo[2.2.1]hept-2-yl]thieno[3,2-c]pyridine-6-carboxamide;
N-[(1S,2R,4R)-7-azabicyclo[2.2.1]hept-2-yl]-3-ethylfuro[2,3-c]pyridine-5-carboxamide;
N-[(1S,2R,4R)-7-azabicyclo[2.2.1]hept-2-yl]-3-isopropyl-furo[2,3-c]pyridine-5-carboxamide;
N-[(1S,2R,4R)-7-azabicyclo[2.2.1]hept-2-yl]-1H-pyrrolo[2,3-c]pyridine-5-carboxamide;
N-[(1S,2R,4R)-7-azabicyclo[2.2.1]hept-2-yl]-1,3-benzodioxole-5-carboxamide;
N-[(1S,2R,4R)-7-azabicyclo[2.2.1]hept-2-yl]-1H-pyrrolo[2,3-c]pyridine-2-carboxamide;
N-[(1S,2R,4R)-7-methyl-7-azabicyclo[2.2.1]hept-2-yl]furo[2,3-c]pyridine-5-carboxamide;
N-[(1S,2R,4R)-7-azabicyclo[2.2.1]hept-2-yl]-1-benzofuran-6-carboxamide;
N-[(1S,2R,4R)-7-azabicyclo[2.2.1]hept-2-yl]-1-benzofuran-5-carboxamide;
N-[(1S,2R,4R)-7-azabicyclo[2.2.1]hept-2-yl]-3-bromofuran[2,3-c]pyridine-5-carboxamide;
N-[(1S,2R,4R)-7-azabicyclo[2.2.1]hept-2-yl]-1-benzofuran-2-carboxamide;
N-[(1S,2R,4R)-7-azabicyclo[2.2.1]hept-2-yl]furo[3,2-c]pyridine-6-carboxamide;
N-[(1S,2R,4R)-7-azabicyclo[2.2.1]hept-2-yl]-3-chlorofuran[2,3-c]pyridine-5-carboxamide;
N-[(1S,2R,4R)-7-azabicyclo[2.2.1]hept-2-yl]benzothiophene-5-carboxamide;
N-[(1S,2R,4R)-7-azabicyclo[2.2.1]hept-2-yl]-2-methyl-1-benzofuran-6-carboxamide;
N-[(1S,2R,4R)-7-azabicyclo[2.2.1]hept-2-yl]-3-methyl-1-benzofuran-6-carboxamide;
N-[(1S,2R,4R)-7-azabicyclo[2.2.1]hept-2-yl]-3-isopropyl-1-benzofuran-5-carboxamide;
N-[(1S,2R,4R)-7-azabicyclo[2.2.1]hept-2-yl]-2-methyl-1-benzofuran-5-carboxamide;
N-[(1S,2R,4R)-7-azabicyclo[2.2.1]hept-2-yl]-3-bromo-1-benzofuran-5-carboxamide;
N-[(1S,2R,4R)-7-azabicyclo[2.2.1]hept-2-yl]-3-ethynyl-1-benzofuran-5-carboxamide;
N-[(1S,2R,4R)-7-azabicyclo[2.2.1]hept-2-yl]-3-prop-1-ynyl-1-benzofuran-5-carboxamide;
N-[(1S,2R,4R)-7-azabicyclo[2.2.1]hept-2-yl]-3-bromothieno[2,3-c]pyridine-5-carboxamide;
N-[(1S,2R,4R)-7-azabicyclo[2.2.1]hept-2-yl]-1H-indole-2-carboxamide;
N-[(1S,2R,4R)-7-azabicyclo[2.2.1]hept-2 yl]-1-benzothiophene-2-carboxamide; and
N-[(1S,2R,4R)-7-azabicyclo[2.2.1]hept-2-yl]-3-methyl-1-benzofuran-5-carboxamide;
and the pharmaceutically acceptable salts thereof.

8. A pharmaceutical composition comprising a compound according to any one of claims 1-7 and a pharmaceutically acceptable excipient.

9. A pharmaceutical composition according to claim 8 comprising a compound according to any one of claims 1-7, an anti-psychotic agent, and a pharmaceutically acceptable excipient.

10. A compound according to any one of claims 1-7 for use as a medicament.

11. Use of a compound according to any one of claims 1-7 for the preparation of a medicament for treating a disease or condition, selected from:
cognitive and attention deficit symptoms of Alzheimer's, neurodegeneration associated with diseases such as Alzheimer's disease, pre-senile dementia (mild cognitive impairment), or senile dementia;
schizophrenia or psychosis;
depression, anxiety, general anxiety disorders, or post traumatic stress disorder; attention deficit disorder, or attention deficit hyperactivity disorder;
mood and affective disorders, amyotrophic lateral sclerosis, borderline personality disorder, traumatic brain injury, behavioral and cognitive problems in general and associated with brain tumors, AIDS dementia complex, dementia associated with Down's syndrome, dementia associated with Lewy Bodies, Huntington's disease, Parkinson's disease, tardive dyskinesia, Pick's disease, dysregulation of food intake including bulemia and anorexia nervosa, withdrawal symptoms associated with smoking cessation and dependant drug cessation, Gilles de la Tourette's Syndrome, age-related macular degeneration, glaucoma, neurodegeneration associated with glaucoma, or symptoms associated with pain.

12. The use according to claim 11, wherein the disease or condition is cognitive and attention deficit symptoms of Alzheimer's, neurodegeneration associated with diseases such as Alzheimer's disease, pre-senile dementia (mild cognitive impairment), or senile dementia.

13. The use according to claim 11, wherein the disease or condition is schizophrenia or psychosis.

14. The use according to claim 11, wherein the disease or condition is depression, anxiety, general anxiety disorders, or post traumatic stress disorder.

15. The use according to claim 11, wherein the disease or condition is attention deficit disorder, or attention deficit hyperactivity disorder.

16. The use according to claim 11, wherein the disease or condition is mood and affective disorders, amyotrophic lateral sclerosis, borderline personality disorder, traumatic brain injury, behavioral and cognitive problems in general and associated with brain tumors, AIDS dementia complex, dementia associated with Down's syndrome, dementia associated with Lewy Bodies, Huntington's disease, Parkinson's disease, tardive dyskinesia, Pick's disease, dysregulation of food intake including bulemia and anorexia nervosa, withdrawal symptoms associated with smoking cessation and dependant drug cessation, Gilles de la Tourette's Syndrome, age-related macular degeneration, glaucoma, neurodegeneration associated with glaucoma, or symptoms associated with pain.

17. Use of a compound according to any one of claims 1-7 and an anti-psychotic agent, for the preparation of a medicament for treating schizophrenia or psychosis.

## Patentansprüche

1. Verbindung der Formel I: 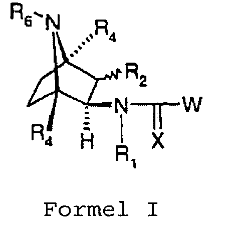
worin die Stereochemie des 7-Azabicyclo[2.2.1]heptanrings *1S, 4R* ist und der Stickstoffsubstituent am C-2-Kohlenstoff die exo-Orientierung hat und 2R ist;
X O ist;
W 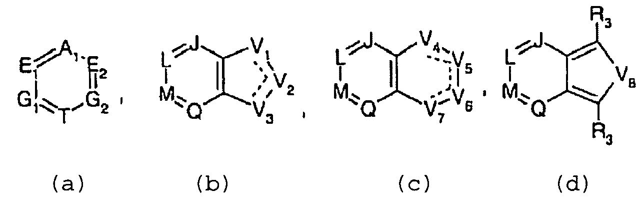 oder 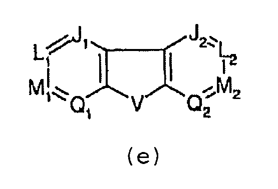 ist;
A₁ N ist;
jedes E₁, E₂, G₁, G₂ und T unabhängig ausgewählt ist aus CR₃ und N, mit der Maßgabe, dass nicht mehr als zwei von E₁, E₂, G₁, G₂ und T N sind und mit der weiteren Maßgabe, dass, wenn E₁ N ist, G₁ und E₂ CR₃ sein müssen, und mit der weiteren Maßgabe, dass, wenn E₂ N ist, E₁ und G₂ CR₃ sein müssen;
jedes V und V₈ unabhängig aus O, S, NA₅ und C(R₃)₂ ausgewählt ist, mit der Maßgabe, dass, wenn V₈ C(R₃)₂ ist, die R₃-Substituenten an den Kohlenstoffatomen, die benachbart zu V₈ sind, andere Gruppierungen als H sind;
jedes V₁, V₂, V₃, V₄, V₅, V₆ und V₇ unabhängig aus O, S, N, C(R₃), C(R₃)₂ oder NA₅ ausgewählt ist, mit der Maßgabe, dass V₁ und V₂, V₂ und V₃, V₄ und V₅, V₅ und V₆ und V₆ und V₇ nicht gleichzeitig O oder S oder Kombinationen von O und S sind, und mit der weiteren Maßgabe, dass mindestens eines von V₁, V₂ und V₃, mindestens eines von V₄, V₅ und V₆ und mindestens eines von V₅, V₆ und V₇ C(R₃) oder C(R₃)₂ ist;
jedes J, J₁, J₂, L, L₁, L₂, M, M₁, M₂, Q, Q₁ und Q₂ unabhängig aus CR₃ und N ausgewählt ist, mit der Maßgabe, dass nicht mehr als zwei von J, L, M und Q oder nicht mehr als zwei von J₁, L₁, M₁ und Q₁ oder nicht mehr als zwei von J₂, L₂, M₂ und Q₂ N sind, und mit der Maßgabe, dass mindestens eines von J, L, M und Q N ist, wenn V₁, V₂ und V₃ unabhängig aus C(R₃)₂ und C(R₃) ausgewählt sind, wenn V₄, V₅, V₆ und V₇ unabhängig aus C(R₃)₂ und C(R₃) ausgewählt sind, und wenn V₈ C(R₃)₂ ist, und mit der weiteren Maßgabe, dass mindestens eines von J₁, L₁, M₁ und Q₁ oder mindestens eines von J₂, L₂, M₂ und Q₂ N ist, wenn V C(R₃)₂ ist;
jedes A₅ unabhängig H, Alkyl mit 1 bis 6 Kohlenstoffatomen, Alkenyl mit 2 bis 6 Kohlenstoffatomen, Alkinyl mit 2 bis 6 Kohlenstoffatomen, Cycloalkyl mit 3 bis 6 Kohlenstoffatomen, Heterocycloalkyl mit 4 bis 7 Atomen, wobei 1 bis 2 Atome in dem Ring -S-, -N(R₁₈)- oder -O- sind, halogeniertes Alkyl mit 1 bis 6 Kohlenstoffatomen, halogeniertes Alkenyl mit 2 bis 6 Kohlenstoffatomen, halogeniertes Alkinyl mit 2 bis 6 Kohlenstoffatomen, halogeniertes Cycloalkyl mit 3 bis 6 Kohlenstoffatomen oder halogeniertes Heterocycloalkyl mit 4 bis 7 Atomen, wobei 1 bis 2 Atome in dem Ring -S-, -N(R₁₈)- oder -O- sind, ist;
R₁ H, Alkyl mit 1 bis 6 Kohlenstoffatomen oder halogeniertes Alkyl mit 1 bis 6 Kohlenstoffatomen ist;
R₂ H, Alkyl mit 1 bis 6 Kohlenstoffatomen oder halogeniertes Alkyl mit 1 bis 6 Kohlenstoffatomen ist;
jedes R₃ unabhängig H, F, Cl, Br, I, -CN, -NO₂, Alkyl mit 1 bis 6 Kohlenstoffatomen, Alkenyl mit 2 bis 6 Kohlenstoffatomen, Alkinyl mit 2 bis 6 Kohlenstoffatomen, Cycloalkyl mit 3 bis 6 Kohlenstoffatomen, Heterocycloalkyl mit 4 bis 7 Atomen, wobei 1 bis 2 Atome in dem Ring -S-, -N(R₁₈)- oder -O- sind, halogeniertes Alkyl mit 1 bis 6 Kohlenstoffatomen, halogeniertes Alkenyl mit 2 bis 6 Kohlenstoffatomen, halogeniertes Alkinyl mit 2 bis 6 Kohlenstoffatomen, halogeniertes Cycloalkyl mit 3 bis 6 Kohlenstoffatomen oder halogeniertes Heterocycloalkyl mit 4 bis 7 Atomen, wobei 1 bis 2 Atome in dem Ring -S-, -N(R₁₈)- oder -O- sind, ist; oder
R₃ eine Bindung an den Kohlenstoff der Amidgruppe, die an den C-2-Kohlenstoff des 7-Azabicyclo[2.2.1]heptanrings gebunden ist, bildet,
jedes R₄ unabhängig H oder Alkyl mit 1 bis 6 Kohlenstoffatomen ist;
R₆ H, Alkyl mit 1 bis 6 Kohlenstoffatomen oder eine Alkylgruppe mit 1 bis 6 Kohlenstoffatomen und mit 1 bis 3 Substituenten, ausgewählt aus F, Cl, Br, I, -OH, -CN, -NH₂, -NH(Alkyl mit 1 bis 6 Kohlenstoffatomen) und -N (Alkyl mit 1 bis 6 Kohlenstoffatomen)₂ ist;
R₁₈ H, Alkyl mit 1 bis 6 Kohlenstoffatomen, halogeniertes Alkyl mit 1 bis 6 Kohlenstoffatomen, Cycloalkyl mit 3 bis 6 Kohlenstoffatomen oder halogeniertes Cycloalkyl mit 3 bis 6 Kohlenstoffatomen ist,
und ein pharmazeutisch annehmbares Salz davon.

2. Verbindung nach Anspruch 1, wobei R₁ H oder Alkyl mit 1 bis 6 Kohlenstoffatomen ist und worin R₂ H, Alkyl mit 1 bis 6 Kohlenstoffatomen oder halogeniertes Alkyl mit 1 bis 6 Kohlenstoffatomen ist.

3. Verbindung nach Anspruch 2, worin W (b) ist.

4. Verbindung nach Anspruch 2, worin W (c) ist.

5. Verbindung nach Anspruch 2, worin W (d) ist.

6. Verbindung nach Anspruch 2, worin W (e) ist.

7. Verbindung nach Anspruch 1, ausgewählt aus:
N-[(1S,2R,4R)-7-Azabicyclo[2.2.1]hept-2-yl]-3-methylfuro[2,3-c]pyridin-5-carboxamid;
N-[(1S,2R,4R)-7-Azabicyclo[2.2.1]hept-2-yl]thieno-[2,3-c]pyridin-5-carboxamid;
N-[(1S,2R,4R)-7-Azabicyclo[2.2.1]hept-2-yl]thieno [3,2-c]pyridin-6-carboxamid;
N-[(1S,2R,4R)-7-Azabicyclo[2.2.1]hept-2-yl]-3-ethylfuro[2,3-c]pyridin-5-carboxamid;
N-[(1S,2R,4R)-7-Azabicyclo[2.2.1]hept-2-yl]-3-isopropylfuro[2,3-c]pyridin-5-carboxamid;
N-[(1S,2R,4R)-7-Aazabicyclo[2.2.1]hept-2-yl]-1H-pyrrolo[2,3-c]pyridin-5-carboxamid;
N-[(1S,2R,4R)-7-Azabicyclo[2.2.1]hept-2-yl]-1,3-benzodioxol-5-carboxamid;
N-[(1S,2R,4R)-7-Azabicyclo[2.2.1]hept-2-yl]-1H-pyrrolo[2,3-c]pyridin-2-carboxamid;
N-[(1S,2R,4R)-7-Methyl-7-azabicyclo[2.2.1]hept-2-yl] furo[2,3-c]pyridin-5-carboxamid;
N-[(1S,2R,4R)-7-Azabicyclo[2.2.1]hept-2-yl]-1-benzofuran-6-carboxamid;
N-[(1S,2R,4R)-7-Azabicyclo[2.2.1]hept-2-yl]-1-benzofuran-5-carboxamid;
N-[(1S,2R,4R)-7-Azabicyclo[2.2.1]hept-2-yl]-3-bromfuran[2,3-c]pyridin-5-carboxamid;
N-[(1S,2R,4R)-7-Azabicyclo[2.2.1]hept-2-yl]-1-benzofuran-2-carboxamid;
N-[(1S,2R,4R)-7-Azabicyclo[2.2.1]hept-2-yl]furo[3,2-c]pyridin-6-carboxamid;
N-[(1S,2R,4R)-7-Azabicyclo[2.2.1]hept-2-yl]-3-chlorfuran[2,3-c]pyridin-5-carboxamid;
N-[(1S,2R,4R)-7-Azabicyclo[2.2.1]hept-2-yl]benzothiophen-5-carboxamid;
N-[(1S,2R,4R)-7-Azabicyclo[2.2.1]hept-2-yl]-2-methyl-1-benzofuran-6-carboxamid;
N-[(1S,2R,4R)-7-Azabicyclo[2.2.1]hept-2-yl]-3-methyl-1-benzofuran-6-carboxamid;
N-[(1S,2R,4R)-7-Azabicyclo[2.2.1]hept-2-yl]-3-isopropyl-1-benzofuran-5-carboxamid;
N-[(1S,2R,4R)-7-Azabicyclo[2.2.1]hept-2-yl]-2-methyl-1-benzofuran-5-carboxamid;
N-[(1S,2R,4R)-7-Azabicyclo[2.2.1]hept-2-yl]-3-brom-1-benzofuran-5-carboxamid;
N-[(1S,2R,4R)-7-Azabicyclo[2.2.1]hept-2-yl]-3-ethinyl-1-benzofuran-5-carboxamid;
N-[(1S,2R,4R)-7-Azabicyclo[2.2.1]hept-2-yl]-3-prop-1-inyl-1-benzofuran-5-carboxamid;
N-[(1S,2R,4R)-7-Azabicyclo[2.2.1]hept-2-yl]-3-bromthieno[2,3-c]pyridin-5-carboxamid;
N-[(1S,2R,4R)-7-Azabicyclo[2.2.1]hept-2-yl]-1H-indol-2-carboxamid;
N-[(1S,2R,4R)-7-Azabicyclo[2.2.1]hept-2-yl]-1-benzothiophen-2-carboxamid; und
N-[(1S,2R,4R)-7-Azabicyclo[2.2.1]hept-2-yl]-3-methyl-1-benzofuran-5-carboxamid;
und die pharmazeutisch annehmbaren Salze davon.

8. Pharmazeutische Zusammensetzung, umfassend eine Verbindung nach einem der Ansprüche 1 bis 7 und ein pharmazeutisch annehmbares Excipiens.

9. Pharmazeutische Zusammensetzung nach Anspruch 8, umfassend eine Verbindung nach einem der Ansprüche 1 bis 7, ein anti-psychotisches Mittel und ein pharmazeutisch annehmbares Excipiens.

10. Verbindung nach einem der Ansprüche 1 bis 7 zur Verwendung als Medikament

11. Verwendung einer Verbindung nach einem der Ansprüche 1 bis 7 zur Herstellung eines Medikaments zur Behandlung einer Krankheit oder eines Zustandes, ausgewählt aus:
Wahrnehmungs- und Aufmerksamkeits-Defizitsymptomen bei Alzheimer, Neurodegeneration, assoziiert mit Erkrankungen, wie Alzheimer-Erkrankung, präsenile Demenz (milde Wahrnehmungsbeeinträchtigung) oder senile Demenz;
Schizophrenie oder Psychose;
Depression, Angstgefühlen, allgemeinen Angststörungen oder posttraumatischer Belastungsstörung; Aufmerksamkeitsdefizitstörung oder Aufmerksamkeits- und Hyperaktivitätsstörung; Gemütskrankheit und affektive Störung, amyotropher Lateralsklerose, Borderline-Persönlichkeitsstörung, traumatischer Hirnverletzung, Verhaltens- und Wahrnehmungsproblemen im Allgemeinen und im Zusammenhang mit Gehirntumoren, AIDS-Demenz-Komplex, Demenz, assoziiert mit dem Down-Syndrom, Demenz, assoziiert mit Lewy-Bodies, Huntington-Erkrankung, Parkinson-Krankheit, tardiver Dyskinesie; Picks-Krankheit, Dysregulierung der Nahrungsmittelaufnahme einschließlich Bulimie und Anorexia nervosa, Entzugssymptomen, die im Zusammenhang mit der Beendigung der Rauchens und der Drogen- bzw. Arzneimittelabhängigkeit stehen, Gilles-de-la-Tourette-Syndrom, Makulardegeneration mit Altersbezug, Glaukom, Neurodegeneration, assoziiert mit Glaukom oder Symptomen, die im Zusammenhang mit Schmerzen stehen.

12. Verwendung nach Anspruch 11, wobei die Erkrankung oder der Zustand Wahrnehmungs- und Aufmerksamkeits-Defizitsymptome bei Alzheimer, Neurodegeneration, assoziiert mit Erkrankungen, wie Alzheimer-Erkrankung, präsenile Demenz (milde Wahrnehmungsbeeinträchtigung) oder senile Demenz ist.

13. Verwendung nach Anspruch 11, wobei die Erkrankung oder der Zustand Schizophrenie oder Psychose ist.

14. Verwendung nach Anspruch 11, wobei die Erkrankung oder der Zustand Depression, Angstgefühl, allgemeine Angststörungen oder posttraumatische Belastungsstörung ist bzw. sind.

15. Verwendung nach Anspruch 11, wobei die Erkrankung oder der Zustand Aufmerksamkeitsdefizitstörung oder Aufmerksamkeits- und Hyperaktivitätsstörung ist.

16. Verwendung nach Anspruch 11, wobei die Erkrankung oder der Zustand Gemütskrankheit und affektive Störung, amyotrophe Lateralsklerose, Borderline-Persönlichkeitsstörung, traumatische Hirnverletzung, Verhaltens- und Wahrnehmungsprobleme im Allgemeinen und im Zusammenhang mit Gehirntumoren, AIDS-Demenz-Komplex, Demenz, assoziiert mit dem Down-Syndrom, Demenz, assoziiert mit Lewy-Bodies, Huntington-Erkrankung, Parkinson-Krankheit, tardive Dyskinesie, Picks-Krankheit, Dysregulierung der Nahrungsmittelaufnahme einschließlich Bulimie und Anorexia nervosa, Entzugssymptome, die im Zusammenhang mit der Beendigung des Rauchens und der Drogen- bzw. Arzneimittelabhängigkeit stehen, Gilles-de-la-Tourette-Syndrom, Makulardegeneration mit Altersbezug, Glaukom, Neurodegeneration, assoziiert mit Glaukom, oder Symptome, die im Zusammenhang mit Schmerzen stehen, ist.

17. Verwendung einer Verbindung nach einem der Ansprüche 1 bis 7 und eines anti-psychotischen Mittels zur Herstellung eines Medikaments zur Behandlung von Schizophrenie oder Psychose.

## Revendications

1. Composé de Formule I : 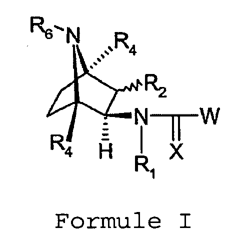
dans laquelle la stéréochimie du noyau 7-azabicyclo[2.2.1]heptane est 1*S*, 4*R* et le substituant azote sur l'atome de carbone en C-2 a l'orientation *exo* et est de type 2*R* ;
X représente O ;
W représente 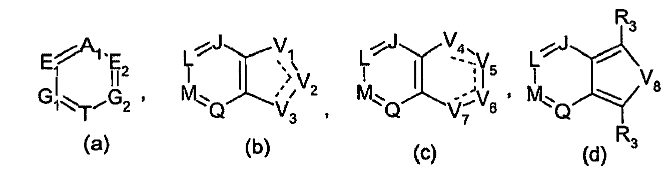 ou 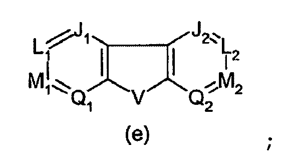
A₁ représente N ;
chacun de E₁, E₂, G₁, G₂ et T est indépendamment sélectionné parmi CR₃ et N, à condition que pas plus de deux groupes parmi E₁, E₂, G₁, G₂ et T représentent N, et à condition, en outre, que lorsque E₁ représente N, alors G₁ et E₂ doivent représenter CR₃, et à condition que lorsque E₂ représente N, alors E₁ et G₂ doivent représenter CR₃ ;
chacun de V et V₈ est indépendamment sélectionné parmi O, S, NA₅ et C(R₃)₂, à condition que, lorsque V₈ représente C(R₃)₂, les substituants de R₃ sur les atomes de carbone adjacents à V₈ sont des groupes fonctionnels autres que H ;
chacun de V₁, V₂, V₃, V₄, V₅, V₆ et V₇ est indépendamment sélectionné parmi O, S, N, C(R₃), C(R₃)₂ ou NA₅, à condition que V₁ et V₂, V₂ et V₃, V₄ et V₅, V₅ et V₆, et V₆ et V₇ ne représentent pas simultanément O ou S, ou des combinaisons de O et S, et à condition, en outre, que l'un au moins de V₁, V₂ et V₃, l'un au moins de V₄, V₅ et V₆, et l'un au moins de V₅, V₆ et V₇ représente C(R₃) ou C(R₃)₂.
chacun de J, J₁, J₂, L, L₁, L₂, M, M₁; M₂, Q, Q₁ et Q₂ est indépendamment sélectionné parmi CR₃ et N, à condition que pas plus de deux groupes parmi J, L, M et Q, ou pas plus de deux groupes parmi J₁, L₁, M₁ et Q₁, ou pas plus de deux groupes parmi J₂, L₂, M₂ et Q₂ représentent N, et à condition que l'un au moins de J, L, M et Q représente N lorsque V₁, V₂ et V₃ sont indépendamment sélectionné parmi C(R₃)₂ et C(R₃), lorsque V₄, V₅, V₆ et V₇ sont indépendamment sélectionnés parmi C(R₃)₂ et C(R₃), et lorsque V₈ représente C(R₃)₂, et à condition, en outre, que l'un au moins de J₁, L₁, M₁ et Q₁, ou l'un au moins de J₂, L₂, M₂ et Q₂ représente N lorsque V représente C(R₃)₂;
chaque A₅ représente indépendamment H, alkyle ayant de 1 à 6 atomes de carbone, alkényle ayant de 2 à 6 atomes de carbone, alkynyle ayant de 2 à 6 atomes de carbone, cycloalkyle ayant de 3 à 6 atomes de carbone, hétérocycloalkyle ayant de 4 à 7 atomes dont 1 ou 2 atomes au sein du noyau représente(nt) -S-, -N(R₁₈)- ou -O-, alkyle halogéné ayant de 1 à 6 atomes de carbone, alkényle halogéné ayant de 2 à 6 atomes de carbone, alkynyle halogéné ayant de 2 à 6 atomes de carbone, cycloalkyle halogéné ayant de 3 à 6 atomes de carbone, ou hétérocycloalkyle halogéné ayant de 4 à 7 atomes dont 1 ou 2 atomes au sein du noyau représente(nt) -S-, -N(R₁₈)- ou -0- ;
R₁ représente H, alkyle ayant de 1 à 6 atomes de carbone ou alkyle halogéné ayant de 1 à 6 atomes de carbone ;
R₂ représente H, alkyle ayant de 1 à 6 atomes de carbone ou alkyle halogéné ayant de 1 à 6 atomes de carbone ;
chaque R₃ représente indépendamment H, F, Cl, Br, I, -CN, -NO₂, alkyle ayant de 1 à 6 atomes de carbone, alkényle ayant de 2 à 6 atomes de carbone, alkynyle ayant de 2 à 6 atomes de carbone, cycloalkyle ayant de 3 à 6 atomes de carbone, hétérocycloalkyle ayant de 4 à 7 atomes dont 1 ou 2 atomes au sein du noyau représente(nt) -S-, -N(R₁₈)- ou -O-, alkyle halogéné ayant de 1 à 6 atomes de carbone, alkényle halogéné ayant de 2 à 6 atomes de carbone, alkynyle halogéné ayant de 2 à 6 atomes de carbone, cycloalkyle halogéné ayant de 3 à 6 atomes de carbone, ou hétérocycloalkyle halogéné ayant de 4 à 7 atomes dont 1 ou 2 atomes au sein du noyau représente(nt) -S-, -N(R₁₈)- ou -0- ; ou
R₃ forme une liaison avec l'atome de carbone du groupe amide lié à l'atome de carbone en C-2 du noyau 7-azabicyclo[2.2.1]heptane
chaque R₄ représente indépendamment H ou alkyle ayant de 1 à 6 atomes de carbone ;
R₆ représente H, alkyle ayant de 1 à 6 atomes de carbone, ou un groupe alkyle ayant de 1 à 6 atomes de carbone et ayant de 1 à 3 substituants sélectionnés parmi F, Cl, Br, I, -OH, -CN, -NH₂, -NH(alkyle ayant de 1 à 6 atomes de carbone), et -N(alkyle ayant de 1 à 6 atomes de carbone)₂ ;
R₁₈ représente H, alkyle ayant de 1 à 6 atomes de carbone, alkyle halogéné ayant de 1 à 6 atomes de carbone, cycloalkyle ayant de 3 à 6 atomes de carbone ou cycloalkyle halogéné ayant de 3 à 6 atomes de carbone ;
et sels pharmaceutiquement acceptables de celui-ci.

2. Composé selon la revendication 1, dans lequel R₁ représente H ou alkyle ayant de 1 à 6 atomes de carbone, et dans lequel R₂ représente H, alkyle ayant de 1 à 6 atomes de carbone ou alkyle halogéné ayant de 1 à 6 atomes de carbone.

3. Composé selon la revendication 2, dans lequel W représente (b).

4. Composé selon la revendication 2, dans lequel W représente (c).

5. Composé selon la revendication 2, dans lequel W représente (d).

6. Composé selon la revendication 2, dans lequel W représente (e).

7. Composé selon la revendication 1, sélectionné parmi :
• N-[(1S,2R,4R)-7-azabicyclo[2.2.1]hept-2-yl]-3-méthylfuro[2,3-c]pyridine-5-carboxamide ;
• N-[(1S,2R,4R)-7-azabicyclo[2.2.1]hept-2-yl]thiéno[2,3-c]pyridine-5-carboxamide ;
• N-[(1S,2R,4R)-7-azabicyclo[2.2.1]hept-2-yl]thiéno[3,2-c]pyridine-6-carboxamide ;
• N-[(1S,2R,4R)-7-azabicyclo[2.2.1]hept-2-yl]-3-éthylfuro[2,3-c]pyridine-5-carboxamide ;
• N-[(1S,2R,4R)-7-azabicyclo[2.2.1]hept-2-yl]-3-isopropylfuro[2,3-c]pyridine-5-carboxamide ;
• N-[(1S,2R,4R)-7-azabicyclo[2.2.1]hept-2-yl]-1H-pyrrolo[2,3-c]pyridine-5-carboxamide ;
• N-[(1S,2R,4R)-7-azabicyclo[2.2.1]hept-2-yl]-1,3-benzodioxole-5-carboxamide ;
• N-[(1S,2R,4R)-7-azabicyclo[2.2.1]hept-2-yl]-1H-pyrrolo[2,3-c]pyridine-2-carboxamide ;
• N-[(1S,2R,4R)-7-méthyl-7-azabicyclo[2.211]hept-2-yl]furo[2,3-c]pyridine-5-carboxamide ;
• N-[(1S,2R,4R)-7-azabicyclo[2.2.1]hept-2-yl]-1-benzofuran-6-carboxamide ;
• N-[(1S,2R,4R)-7-azabicyclo[2.2.1]hept-2-yl]-1-benzofuran-5-carboxamide ;
• N-[(1S,2R,4R)-7-azabicyclo[2.2.1]hept-2-yl]-3-bromofuran[2,3-c]pyridine-5-carboxamide ;
• N-[(1S,2R,4R)-7-azabicyclo[2.2.1]hept-2-yl]-1-benzofuran-2-carboxamide ;
• N-[(1S,2R,4R)-7-azabicyclo[2.2.1]hept-2-yl]furo[3,2-c]pyridine-6-carboxamide ;
• N-[(1S,2R,4R)-7-azabicyclo[2.2.1]hept-2-yl]-3-chlorofuran[2,3-c]pyridine-5-carboxamide ;
• N-[(1S,2R,4R)-7-azabicyclo[2.2.1]hept-2-yl]benzothiophène-5-carboxamide ;
• N-[(1S,2R,4R)-7-azabicyclo[2.2.1]hept-2-yl]-2-méthyl-1-benzofuran-6-carboxamide ;
• N-[(1S,2R,4R)-7-azabicyclo[2.2.1.]hept-2-yl]-3-méthyl-1-benzofuran-6-carboxamide ;
• N-[(1S,2R,4R)-7-azabicyclo[2.2.1]hept-2-yl]-3-isopropyl-1-benzofuran-5-carboxamide ;
• N-[(1S,2R,4R)-7-azabicyclo[2.2.1]hept-2-yl]-2-méthyl-1-benzofuran-5-carboxamide ;
• N-[(1S,2R,4R)-7-azabicyclo[2.2.1]hept-2-yl]-3-bromo-1-benzofuran-5-carboxamide ;
• N-[(1S,2R,4R)-7-azabicyclo[2.2.1]hept-2-yl]-3-éthynyl-1-benzofuran-5-carboxamide ;
• N-[(1S,2R,4R)-7-azabicyclo[2.2.1]hept-2-yl]-3-prop-1-ynyl-1-benzofuran-5-carboxamide ;
• N-[(1S,2R,4R)-7-azabicyclo[2.2.1]hept-2-yl]-3-bromothiéno[2,3-c]pyridine-5-carboxamide ;
• N-[(1S,2R,4R)-7-azabicyclo[2.2.1]hept-2-yl]-1H-indole-2-carboxamide ;
• N-[(1S,2R,4R)-7-azabicyclo[2.2.1]hept-2-yl]-1-benzothiophène-2-carboxamide ; et
• N-[(1S,2R,4R)-7-azabicyclo[2.2.1]hept-2-yl]-3-méthyl-1-benzofuran-5-carboxamide ;
• et les sels pharmaceutiquement acceptables de ceux-ci.

8. Composition pharmaceutique comprenant un composé selon l'une quelconque des revendications 1 à 7 et un excipient pharmaceutiquement acceptable.

9. Composition pharmaceutique selon la revendication 8, comprenant un composé selon l'une quelconque des revendications 1 à 7, un agent anti-psychotique et un excipient pharmaceutiquement acceptable.

10. Composé selon l'une quelconque des revendications 1 à 7, pour une utilisation en tant que médicament.

11. Utilisation d'un composé selon l'une quelconque des revendications 1 à 7, pour la préparation d'un médicament pour le traitement d'une maladie ou d'un état, sélectionné(e) parmi :
les symptômes du déficit cognitif et d'attention liés à la maladie d'Alzheimer, la neurodégénérescence associée aux maladies telles que la maladie d'Alzheimer, la démence pré-sénile (trouble cognitif léger) ou la démence sénile ;
la schizophrénie ou la psychose ;
la dépression, l'anxiété, les troubles généraux de l'anxiété ou le trouble du stress post-traumatique ;
le trouble du déficit d'attention ou le trouble du déficit d'attention avec hyperactivité ;
les troubles de l'humeur et affectifs, la sclérose amyotrophique latérale, le trouble de la personnalité borderline, la lésion cérébrale traumatique, les problèmes comportementaux et cognitifs en général et associés aux tumeurs cérébrales, le complexe démentiel du SIDA, la démence associée au syndrome de Down, la démence associée aux corps de Lewy, la maladie de Huntington, la maladie de Parkinson, la dyskinésie tardive, la maladie de Pick, le dérèglement dans la prise de nourriture, y compris la boulimie et l'anorexie nerveuse, les symptômes de sevrage associés à l'arrêt du tabagisme et l'arrêt de la prise de drogue, le syndrome de Gilles de la Tourette, la dégénérescence maculaire liée à l'âge, le glaucome, la neurodégénérescence associée au glaucome, ou les symptômes associés à la douleur.

12. Utilisation selon la revendication 11, dans laquelle la maladie ou l'état consiste en les symptômes du déficit cognitif et d'attention liés à la maladie d'Alzheimer, la neurodégénérescence associée aux maladies telles que la maladie d'Alzheimer, la démence pré-sénile (trouble cognitif léger) ou la démence sénile.

13. Utilisation selon la revendication 11, dans laquelle la maladie ou l'état est la schizophrénie ou la psychose.

14. Utilisation selon la revendication 11, dans laquelle la maladie ou l'état est la dépression, l'anxiété, les troubles généraux de l'anxiété ou le trouble du stress post-traumatique.

15. Utilisation selon la revendication 11, dans laquelle la maladie ou l'état est le trouble du déficit d'attention ou le trouble du déficit d'attention avec hyperactivité.

16. Utilisation selon la revendication 11, dans laquelle la maladie ou l'état consiste en les troubles de l'humeur et affectifs, la sclérose amyotrophique latérale, le trouble de la personnalité borderline, la lésion cérébrale traumatique, les problèmes comportementaux et cognitifs en général et associés aux tumeurs cérébrales, le complexe démentiel du SIDA, la démence associée au syndrome de Down, la démence associée aux corps de Lewy, la maladie de Huntington, la maladie de Parkinson, la dyskinésie tardive, la maladie de Pick, le dérèglement dans la prise de nourriture, y compris la boulimie et l'anorexie nerveuse, les symptômes de sevrage associés à l'arrêt du tabagisme et l'arrêt de la prise de drogue, le syndrome de Gilles de la Tourette, la dégénérescence maculaire liée à l'âge, le glaucome, la neurodégénérescence associée au glaucome, ou les symptômes associés à la douleur.

17. Utilisation d'un composé selon l'une quelconque des revendications 1 à 7 et d'un agent anti-psychotique, pour la préparation d'un médicament pour traiter la schizophrénie ou la psychose.
